# EUROPEAN PATENT APPLICATION

(11) **EP 2 650 006 A1**
(43) Date of publication of application: **16.10.2013**
(21) Application number: 13162395.1
(22) Date of filing: 05.09.2008
(51) Int. Cl.: A61K 38/00, A61K 38/27, C07K 17/00, C07K 14/575, A61K 51/08

(54) **Analogues of exendin-4 and exendin-3**

(30) Priority: 07.09.2007 US 967855 P
(62) Divisional of application: 08830910.9
(71) Applicant: IPSEN PHARMA S.A.S., 92100 Boulogne-Billancourt (FR)
(72) Inventor: Dong, Zheng Xin, Holliston, MA 01746 (US)
(74) Representative: Harrison Goddard Foote LLP

(57) **Abstract**

The present invention is directed to peptide analogues of Exendin-3 and Exendin-4 and pharmaceutically-acceptable salts thereof, to methods of using such analogues to treat mammals and to pharmaceutical compositions containing said analogues and/or pharmaceutically-acceptable salts thereof.

## Description

### FIELD OF THE INVENTION

The present invention relates to novel exendin and peptide exendin agonist formulations, dosages, and dosage formulations that are bioactive and are deliverable via injectable and non-injectable routes, for example, via the respiratory tract, the mouth, and the gut. These formulations and dosages and methods of administration are useful in the treatment of diabetes, including Type I and II diabetes, in the treatment of disorders which would be benefited by agents which lower plasma glucose levels and in the treatment of disorders which would be benefited by the administration of agents useful in delaying and/or slowing gastric emptying or reducing food intake.

### BACKGROUND

The following description includes information that may be useful in understanding the present invention. It is not an admission that any of the information provided herein is prior art to the presently claimed inventions, or relevant, nor that any of the publications specifically or implicitly referenced are prior art.

The exendins are peptides that are found in the salivary secretions of the Gila monster and the Mexican Beaded Lizard, reptiles that are indigenous to Arizona and Northern Mexico. Exendin-3 [SEQ ID NO:1; His Ser Asp Gly Thr Phe Thr Ser Asp Leu Ser Lys Gln Met Glu Glu Glu Ala Val Arg Leu Phe Ile Glu Trp Leu Lys Asn Gly Gly Pro Ser Ser Gly Ala Pro Pro Pro Ser-NH₂] is present in the salivary secretions of Heloderma a horridum (Mexican Beaded Lizard) and exendin-4 [SEQ ID NO:2; His Gly Glu Gly Thr Phe Thr Ser Asp Leu Ser Lys Gln Met Glu Glu Glu Ala Val Arg Leu Phe Ile Glu Trp Leu Lys Asn Gly Gly Pro Ser Ser Gly Ala Pro Pro Pro Ser-NH₂] is present in the salivary secretions of Heloderma suspectum (Gila monster) (Eng, J. et al., J. Biol. Chem., 265:20259-62, 1990; Eng, J. et al., J. Biol. Chem., 267:7402-05, 1992). Exendin-4 was first thought to be a (potentially toxic) component of the venom. It now appears that exendin-4 is devoid of toxicity and that it instead is made in salivary glands in the Gila monster.

The exendins [Exendin-3 and Exendin-4] possess sequence similarity to several members of the glucagon-like peptide family, with the highest homology, 53%, being to GLP-1 [7-36]NH₂ (Goke et al., J. Biol. Chem., 268:19650-55, 1993). GLP-1 [7-36] NH₂ is also known as proglucagon [78-107], or simply "GLP-1" as used most often herein. GLP-1 has an insulinotropic effect, i.e. stimulating insulin secretion from pancreatic beta cells. GLP-1 inhibits glucagon secretion from pancreatic α-cells (Ørsov et al., Diabetes, 42:658-61, 1993; D'Alessio et al., J. Clin. Invest., 97:133-38, 1996). The amino acid sequence of GLP-I (7-37) is His Ala Glu Gly Thr Phe Thr Ser Asp Val Ser Ser Tyr Leu Glu Gly Gln Ala Ala Lys Glu Phe Ile Ala Trp Leu Val Lys Gly Arg Gly (SEQ ID NO:3). GLP-1 inhibits gastric emptying (Willms, et al., J Clin Endocrinol Metab, 8 (1):327-32, 1996; Wettergren et al., Dig Dis Sci, 38(4):665-73, 1993) and gastric acid secretion (Schjoldager et al., Dig Dis Sci, 34(5):703-8, 1989; O'Halloran et al., J Endocrinol, 126(1):169-73, 1990; and Wettergren et al., Dig Dis Sci., 38(4):665-73, 1993)). GLP-1(7-37), which has an additional glycine residue at its carboxy terminus, also stimulates insulin secretion in humans (Ørsov et al., Diabetes, 42:658-61, 1993). A transmembrane G-protein adenylate-cyclase-coupled receptor said to be responsible at least in part for the insulinotropic effect of GLP-1 was cloned from a beta-cell line (Thorens, Proc. Natl. Acad. Sci. USA, 89:8641-45, 1992).

GLP-1 has been the focus of significant investigation in recent years due to reported actions such as the amplification of stimulated insulin production (Byrne and Goke, In: Fehmann H C, Goke B. Insulinotropic Gut Hormone Glucagon-Like Peptide 1. Basel, Switzerland: Karger, 219-33, 1997), the inhibition of gastric emptying (Wettergren et al., Dig. Dis. Sci, 38(4): 665-73, 1996), the inhibition of glucagon secretion (Creutzfeldt et al., Diabetes Care, 19(6):580-6, 1996), and a purported role in appetite control (Turton et al., Nature, 379(6560):69-72, 1996). GLP-1 has also been reported to restore islet glucose sensitivity in aging rats, restoring their glucose tolerance to that of younger rats (Egan et al., Diabetologia, 40(Suppl 1):A130, 1997). The short duration of biological action of GLP-1 in vivo is one feature of the peptide that has hampered its development as a therapeutic agent.

Pharmacological studies have demonstrated both similarities and differences between exendin-4 and GLP-1. Exendin-4 reportedly can act at GLP-1 receptors on insulin-secreting β-TC1 cells, at dispersed acinar cells from guinea pig pancreas, and at parietal cells from stomach. The peptide is also reported to stimulate somatostatin release and inhibit gastrin release in isolated stomachs (Goke et al., J. Biol. Chem., 268:19650-55, 1993; Schepp et al., Eur. J. Pharmacol., 69:183-91, 1994; Eissele et al., Life Sci., 55:629-34, 1994). Exendin-3 and Exendin-4 were reportedly found to stimulate cAMP production in, and amylase release from, pancreatic acinar cells (Malhotra et al., Regulatory Peptides, 41:149-56, 1992; Raufman et al., J. Biol. Chem. 267;21432-37, 1992; Singh et al., Regul. Pent., 53:47-59, 1994). Exendin-4 also has a significantly longer duration of action than GLP-1. For example, in one experiment, glucose lowering by Exendin-4 in diabetic mice was reported to persist for several hours, and, depending on dose, for up to 24 hours (Eng J., Diabetes, 45(Suppl 2):152A (abstract 554), 1996). Based on their insulinotropic activities, the use of Exendin-3 and Exendin-4 for the treatment of diabetes mellitus and the prevention of hyperglycemia has been proposed (Eng, U.S. Pat. No. 5,424,286).

C-terminally truncated exendin peptides such as Exendin-4[9-39], a carboxyamidated molecule, and fragments 3-39 through 9-39 have been reported to be potent and selective antagonists of GLP-1 (Goke et al., J. Biol. Chem., 268:19650-55, 1993; Raufman, J. P., et al., J. Biol. Chem. 266:2897-902, 1991; Schepp, W. et al., Eur. J. Pharm., 269:183-91, 1994; Montrose-Rafizadeh et al., Diabetes, 45(Suppl. 2):152A, 1996). Exendin-4[9-39] block endogenous GLP-1 *in vivo,* resulting in reduced insulin secretion (Wang, et al., J. Clin. Invest., 95:417-21, 1995; D'Alessio et al., J. Clin. Invest., 97:133-38, 1996). A receptor apparently responsible for the insulinotropic effect of GLP-1 in rats was cloned from rat pancreatic islet cell (Thorens, B., Proc. Natl. Acad. Sci. USA, 89:8641-8645, 1992). Exendins and Exendin-4[9-39] are said to bind to the cloned rat GLP-1 receptor (rat pancreatic β-cell GLP-1 receptor (Fehmann et al., Peptides, 15(3):453-6, 1994) and human GLP-1 receptor (Thorens et al., Diabetes, 42(11):1678-82, 1993)). In cells transfected with the cloned GLP-1 receptor, exendin-4 is reportedly an agonist, i.e., it increases cAMP, while exendin [9-39] is an antagonist, i.e., it blocks the stimulatory actions of exendin-4 and GLP-1.

Exendin-4 [9-39] is also reported to act as an antagonist of the full length exendins, inhibiting stimulation of pancreatic acinar cells by Exendin-3 and Exendin-4 (Raufman, J. Biol. Chem., 266:2897-902, 1991; Raufman et al., J. Biol. Chem., 266:21432-37, 1992). It is also reported that Exendin [9-39] inhibits the stimulation of plasma insulin levels by Exendin-4, and inhibits the somatostatin release-stimulating and gastrin release-inhibiting activities of Exendin-4 and GLP-1 (Kolligs et al., Diabetes, 44:16-19, 1995; Eissele et al., Life Sciences, 55:629-34, 1994). Exendin [9-39] has been used to investigate the physiological relevance of central GLP-1 in control of food intake (Turton et al., Nature, 379:69-72, 1996). GLP-1 administered by intracerebroventricular (ICV) injection inhibits food intake in rats. This satiety-inducing effect of GLP-1 delivered ICV is reported to be inhibited by ICV injection of exendin [9-39] (Turton, supra), however, it has been reported that GLP-1 does not inhibit food intake in mice when administered by peripheral injection (Turton, Nature, 379:69-72, 1996; Bhavsar, Soc. Neurosci. Abstr., 21:460(188.8), 1995).

The results of an investigation of whether exendins are the species homolog of mammalian GLP-1 was reported by Chen and Drucker who cloned the exendin gene from the Gila monster (J. Biol. Chem., 272(7):4108-15 (1997)). The observation that the Gila monster also has separate genes for proglucagons (from which GLP-1 is processed), that are more similar to mammalian proglucagon than exendin, indicates that exendins are not species homologs of GLP-1.

Agents that serve to delay gastric emptying have found a place in medicine as diagnostic aids in gastrointestinal radiological examinations. For example, glucagon is a polypeptide hormone that is produced by the alpha cells of the pancreatic islets of Langerhans. It is a hyperglycemic agent that mobilizes glucose by activating hepatic glycogenolysis. It can to a lesser extent stimulate the secretion of pancreatic insulin. Glucagon is used in the treatment of insulin-induced hypoglycemia, for example, when administration of glucose intravenously is not possible, however, as glucagon reduces the motility of the gastro-intestinal tract it is also used as a diagnostic aid in gastrointestinal radiological examinations. Glucagon has also been used in several studies to treat various painful gastrointestinal disorders associated with spasm *(*Daniel et al., Br. Med. J., 3:720, 1974) reported quicker symptomatic relief of acute diverticulitis in patients treated with glucagon compared with those who had been treated with analgesics or antispasmodics. A review by Glauser, et al. (J. Am. Coll. Emergency Physns, 8:228, 1979) described relief of acute esophageal food obstruction following glucagon therapy. In another study, glucagon significantly relieved pain and tenderness in 21 patients with biliary tract disease compared with 22 patients treated with placebo (M. J. Stower et al., Br. J. Surg., 69:591-2, 1982).

Methods for regulating gastrointestinal motility using amylin agonists are described in International Application No. PCT/US94/10225, published Mar. 16, 1995.

Methods for regulating gastrointestinal motility using exendin agonists are described in U.S. patent application Ser. No. 08/908,867, filed Aug. 8, 1997.

Methods for reducing food intake using exendin agonists are described in U.S. patent application Ser. No. 09/003,869, filed Jan. 7, 1998, entitled "Use of Exendin and Agonists Thereof for the Reduction of Food Intake,".

Exendins have also been reported to have inotropic and diuretic effects, as set forth in International Application No. PCT/US99/02554, filed Feb. 5, 1999, claiming the benefit of Provisional Application No. 60/075,122, filed Feb. 13, 1998.

Novel exendin agonist compounds are described PCT Application Serial No. PCT/US98/16387 filed Aug. 6, 1998, entitled "Novel Exendin Agonist Compounds".

Other novel exendin agonists are described in PCT Application Serial No. PCT/US98/24210, filed Nov. 13, 1998, entitled "Novel Exendin Agonist Compounds," which claims the benefit of U.S. Provisional Application No. 60/065,442 filed Nov. 14, 1997.

Still other novel exendin agonists are described in PCT Application Serial No. PCT/US98/24273, filed Nov. 13, 1998, entitled "Novel Exendin Agonist Compounds," which claims the benefit of U.S. Provisional Application No. 60/066,029 filed Nov. 14, 1997.

Since the appearance of the first therapeutically active peptides and proteins produced by genetic engineering, there has been an ever-increasing demand to be able to deliver these drugs by routes other than parenteral. This has been thwarted, however, by the very properties of peptides and proteins that set them apart from the small drug molecules widely in use today. These properties include molecular size, susceptibility to proteolytic breakdown, rapid plasma clearance, peculiar dose-response curves, immunogenicity, biocompatibility, and the tendency of peptides and proteins to undergo aggregation, adsorption, and denaturation.

It is generally understood that the administration of peptide drugs by routes other than subcutaneous or intravenous injection, or intravenous infusion, is often not practical because of, for example, in the case of oral administration, both enzymatic degradation and non-absorption in the gastrointestinal tract. Thus, there continues to exist a need for the development of alternative methods to the inconvenient, sometimes painful, injection for administration of peptide drugs, such as exendins and the peptide exendin agonist analogs referenced above. In addition to formulations and dosages useful in the administration of exendins and exendin agonists by injection, formulations, dosage formulations, and methods that solve these problems and that are useful in the non-injection delivery of therapeutically effective amounts of exendin and exendin agonists are described and claimed herein.

The contents of the above-identified articles, patents, and patent applications, and all other documents mentioned or cited herein, are hereby incorporated by reference in their entirety. Applicants reserve the right to physically incorporate into this application any and all materials and information from any such articles, patents, patent applications, or other documents mentioned or cited herein.

### SUMMARY OF THE INVENTION

In one aspect the present invention is directed to a compound of formula (I),

(R²R³)A¹-A²-A³-A⁴-A⁵-A⁶-A⁷-A⁸-A⁹-A¹⁰-A¹¹-A¹²-A¹³-A¹⁴-A¹⁵-A¹⁶-A¹⁷-A¹⁸-A¹⁹-A²⁰-A²¹-A²²-A²³-A²⁴-A²⁵-A²⁶-A²⁷-A²⁸-A²⁹-A³⁰-A³¹-A³²-A³³-A³⁴-A³⁵-A³⁶-A³⁷-A³⁸-A³⁹-A⁴⁰-A⁴¹-A⁴²-R¹ (I)

or a pharmaceutically acceptable salt thereof,
wherein:
A¹ is His or Ala, Amp, Arg, Dab, Dap, D-His, Tma-His, Hppa, Lys, Orn, Paa, 3-Pal, 4-Pal, Pta, Tyr, Ura or deleted;
A² is Gly or Ser or Acc, Aib, Ala, *β*-Ala, N-Me-Ala, N-Me-D-Ala, Arg, Dab, Dap, N-Me-Gly, Lys, Orn, HN-CH(CH₂OC(O)R⁴)-C(O), a D-Amino Acid or deleted;
A³ is Asp or Glu or Acc, Aib, Ala, Arg, N-Me-Asp, Dab, Dap, N-Me-Glu, Lys, Orn, HN-CH((CH₂)_{q}-C(O)-O-R⁵)-C(O), a D-Amino acid or deleted;
A⁴ is Gly or Acc, Aib, Ala, *β*-Ala, Arg, Dab, Dap, Lys, Orn, Ser, a D-Amino acid or deleted;
A⁵ is Thr or Acc, Aib, Ala, Arg, Dab, Dap, Lys, Orn, Ser, HN-CH(CH(CH₃)-OC(O)R⁴)-C(O), HN-CH(CH₂OC(O)R⁴)-C(O or deleted;
A⁶ is Phe or Acc, Aib, Aic, Arg, Cha, Dab, Dap, Lys, *β*-1-Nal, *β*-2-Nal, Orn, 3-Pal, 4-Pal, X¹ᵤ-Phe, Trp or deleted;
A⁷ is Thr or Acc, Aib, Ala, Arg, Dab, Dap, Lys, Orn, Ser, HN-CH(CH(CH₃)-OC(O)R⁴)-C(O), HN-CH(CH₂OC(O)R⁴)-C(O) or deleted;
A⁸ is Ser or Acc, Aib, Ala, Arg, Dab, Dap, Lys, Orn, Thr, HN-CH(CH₂OC(O)R⁴)-C(O), HN-CH(CH(CH₃)-OC(O)R⁴)-C(O) or deleted;
A⁹ is Asp or Aib, Ala, Arg, Dab, Dap, Glu, Lys, Orn or HN-CH((CH₂)_{q}-C(O)-O-R⁵)-C(O);
A¹⁰ is Leu or Abu, Acc, Aib, Ala, Arg, Cha, Dab, Dap, Ile, Lys, Nle, Orn, Phe, X¹ᵤ-Phe, Tle or Val;
A¹¹ is Ser or Acc, Aib, Ala, Arg, Dab, Dap, Lys, Orn, Thr, HN-CH(CH₂OC(O)R⁴)-C(O) or HN-CH(CH(CH₃)-OC(O)R⁴)-C(O);
A¹² is Lys or Acc, Aib, Ala, Amp, Arg, hArg, Dab, Dap, Orn, Ser, Thr or HN-CH((CH₂)ₙ-NR⁶R⁷)-C(O);
A¹³ is Gln or Acc, Aib, Arg, Asn, Cha, Dab, Dap, Lys, *β*-1-Nal, *β*-2-Nal, Orn, 3-Pal, 4-Pal, Phe or Tyr;
A¹⁴ is Met or Abu, Acc, Aib, Ala, Arg, Cha, Dab, Dap, Ile, Leu, Lys, Nle, Orn, Phe, X¹ᵤ-Phe, Tle or Val;
A¹⁵ is Glu or Acc, Aib, Ala, Arg, Asp, Dab, Dap, Lys, Orn or HN-CH((CH₂)_{q}-C(O)-O-R⁵)-C(O);
A¹⁶ is Glu or Acc, Aib, Ala, *β*-Ala, D-Ala, Arg, Asp, Dab, Dap, Gly, Lys, Orn or HN-CH((CH₂)_{q}-C(O)-O-R⁵)-C(O);
A¹⁷ is Glu or Acc, Aib, Ala, Arg, Asn, Asp, Dab, Dap, Gln, Lys, Orn or HN-CH((CH₂)_{q}-C(O)-O-R⁵)-C(O);
A¹⁸ is Ala or Abu, Acc, Aib, Arg, Dab, Dap, Lys, Orn or Val;
A¹⁹ is Val or Abu, Acc, Aib, Ala, Arg, hArg, Cha, Dab, Dap, Ile, Leu, Lys, Nle, Nva, Orn, Phe, X¹ᵤ-Phe, Tle or HN-CH((CH₂)ₙ-NR⁶R⁷)-C(O);
A²⁰ is Arg or Amp, hArg, Dab, Dap, Lys, Orn, 3-Pal, 4-Pal or HN-CH((CH₂)ₙ-NR⁶R⁷)-C(O);
A²¹ is Leu or Abu, Acc, Aib, Ala, Arg, Asp, Cha, Dab, Dap, Glu, Ile, Lys, Nle, Orn, Phe, X¹ᵤ-Phe, Tle or Val;
A²² is Phe or Acc, Aib, Aic, Ala, Arg, Cha, Dab, Dap, Lys, *β*-1-Nal, *β*-2-Nal, Orn, 3-Pal, 4-Pal, X¹ᵤ-Phe or Trp;
A²³is Ile or Abu, Acc, Aib, Ala, Arg, Cha, Dab, Dap, Leu, Lys, Nle, Orn, Phe, X¹ᵤ-Phe, Tle or Val;
A²⁴ is Glu or Abu, Acc, Aib, Ala, Arg, Asp, Dab, Dap, Orn, Val or deleted,
A²⁵ is Trp or Acc, Aib, Ala, Amp, Arg, Cha, Dab, Dap, Lys, β-1-Nal, β-2-Nal, Orn, 3-Pal, 4-Pal, Phe, X¹ᵤ-Phe, HN-CH((CH₂)_{q}-C(O)-O-R⁵)-C(O) or deleted;
A²⁶ is Leu or Abu, Acc, Aib, Ala, Arg, Cha, Dab, Dap, Ile, Lys, Nle, Orn, Phe, X¹ᵤ-Phe, Tle, Val or deleted;
A²⁷ is Lys or Abu, Acc, Aib, Ala, Amp, Arg, hArg, Cha, Dap, Dab, Ile, Leu, Nle, Nva, Orn, Phe, X¹ᵤ-Phe, Tle, Val, HN-CH((CH₂)ₙ-NR⁶R⁷)-C(O) or deleted;
A²⁸ is Asn or Acc, Aib, Ala, Amp, Arg, hArg, Dab, Dap, Gln, Lys, Orn, HN-CH((CH₂)ₙ-NR⁶R⁷)-C(O), a D-amino acid or deleted;
A²⁹ is Gly or Acc, Aib, Ala, *β*-Ala, Arg, Dab, Dap, Lys, Orn, HN-(CH₂)ₘ-C(O), a D-Amino acid or deleted;
A³⁰ is Gly or Acc, Aib, Ala, *β*-Ala, Amp, Arg, hArg, Dap, Dab, Lys, Orn, Val, HN-(CH₂)ₘ-C(O), HN-CH((CH₂)ₙ-NR⁶R⁷)-C(O), a D-Amino acid or deleted;
A³¹ is Pro or Acc, Aib, Ala, Arg, Dab, Dap, Lys, Orn, D-Pro, hPro, R⁹ or deleted,
A³² is Ser or Acc, Aib, Ala, Arg, Dab, Dap, Lys, Orn, D-Ser, Thr, HN-CH(CH₂OC(O)R⁴)-C(O), HN-CH(CH(CH₃)-OC(O)R⁴)-C(O), R⁹, a D-amino acid or deleted;
A³³ is Ser or Acc, Aib, Ala, Arg, Dab, Dap, Lys, Orn, D-Ser, Thr, HN-CH(CH₂OC(O)R⁴)-C(O), HN-CH(CH(CH₃)-OC(O)R⁴)-C(O), R⁹, a D-amino acid or deleted;
A³⁴ is Gly or Acc, Aib, Ala, *β*-Ala, Arg, Dab, Dap, Lys, Orn, HN-(CH₂)ₘ-C(O), R⁹, a D-Amino acid or deleted;
A³⁵ is Ala or Abu, Acc, Aib, *β*-Ala, D-Ala, Arg, Dab, Dap, Lys, Orn, Val, HN-(CH₂)ₘ-C(O), R⁹, a D-amino acid or deleted;
A³⁶ is Pro or Acc, Aib, Ala, Arg, Dab, Dap, Lys, Orn, D-Pro, hPro, R⁹, a D-amino acid or deleted;
A³⁷ is Pro or Acc, Aib, Ala, Arg, Dab, Dap, Lys, Orn, D-Pro, hPro, R⁹, a D-amino acid or deleted;
A³⁸ is Pro or Acc, Aib, Ala, Arg, Dab, Dap, Lys, Orn, D-Pro, hPro, R⁹, a D-amino acid or deleted;
A³⁹ is Ser or Acc, Aib, Ala, Arg, Dab, Dap, Lys, Orn, Thr, HN-CH(CH₂OC(O)R⁴)-C(O), HN-CH(CH(CH₃)-OC(O)R⁴)-C(O), R⁹, a D-Amino Acid or deleted;
A⁴⁰ is Asp, D-Asp, Glu, D-Glu, HN-(CH₂)ₘ-C(O), HN-CH((CH₂)_{q}-C(O)-O-R⁵)-C(O), R⁹ or deleted;
A⁴¹ is HN-(CH₂)ₘ-C(O), R⁹ or deleted;
A⁴² is HN-(CH₂)ₘ-C(O), R⁹ or deleted;
R¹ is OH, NH₂, or (C₁-C₁₂)alkoxy;
R² and R³ each is independently selected from the group consisting of H, (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl, hydroxynaphthyl(C₁-C₃₀)alkyl and C(O)X³;
R⁴ and R⁵ each is independently (C₁-C₃₀)alkyl;
R⁶ and R⁷ each is, independently for each occurrence, H, (C₁-C₃₀)alkyl, (C₁-C₃₀)acyl, (C₁-C₃₀)alkylsulfonyl or -C(NH)NH₂;
R⁹ is 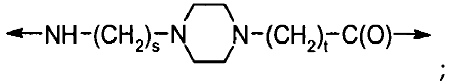
X¹ is, independently for each occurrence, (C₁-C₆)alkyl, OH or halogen;
X³ is (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl or hydroxynaphthyl(C₁-C₃₀)alkyl;
m is 1 to 20;
n is 1 to 5;
q is 1 or 2;
s is 1 to 5;
t is 1 to 5; and
u is 1 to 5;
provided that when R⁶ is (C₁-C₃₀)acyl, (C₁-C₃₀)alkylsulfonyl or -C(NH)NH₂, then R⁷ is H or (C₁-C₃₀)alkyl; and further provided that said compound is not a compound selected from the group consisting of Exendin 4(v-w)-OH, Exendin 4(v-w)-NH₂, Exendin 3(v-w)-OH and Exendin 3(v-w)-NH₂, wherein v is 1 - 8 and w is 24-39.

In one embodiment of the foregoing aspect the invention features a compound, or pharmaceutically acceptable salt thereof, wherein:
A¹ is His, Hppa, Paa, Pta, Ura or deleted;
A² is Aib, D-Ala, Gly or deleted;
A¹⁰ is A6c or Leu;
A¹¹ is Aib or Ser;
A¹² is Arg, Lys, Lys(N^{ε}-octanoyl) or Lys(N^{ε}-Acp-octanoyl);
A¹⁴ is A6c, Leu, Met or Nle;
A¹⁷ is Glu or Ser(O-decanoyl);
A¹⁸ is Aib or Ala;
A²⁵ is Phe, Trp or deleted;
A²⁶ is A6c, Leu or deleted;
A²⁷ is Arg, Lys, Lys(N^{ε}-octanoyl), Lys(N^{ε}-Acp-octanoyl), Lys(N^{ε}-Acp-decanoyl) or deleted;
A²⁸ is Aib, Asn or deleted;
A²⁹ is Aib, β-Ala, Gly or deleted;
A³⁰ is Arg, D-Arg, Gly or deleted;
A³¹ is Acp, Asp, D-Asp, Ava, Pro, Ser(O-decanoyl) or deleted;
A³² is Aun, Ava, Ser, Ser(O-decanoyl) or deleted;
A³³ is Aun, Ser, Ser(O-decanoyl) or deleted;
A³⁹ is Ser, Ser(O-decanoyl) or deleted;
A⁴⁰ is Acp, Asp, D-Asp, Ava or deleted;
A⁴¹ is Aun, Ava or deleted; and
A⁴² is Aun or deleted.

In another embodiment of the foregoing aspect, the invention features a compound, or pharmaceutically acceptable salt thereof, wherein:
A² is Aib;
A¹² is Arg or Lys;
A¹⁸ is Aib or Ala;
A²⁶ is A6c or Leu;
A²⁷ is Arg, Lys or Lys²⁷(N^{ε}-Acp-decanoyl);
A²⁸ is Aib or Asn;
A²⁹ is Aib, *β*-Ala or Gly;
A³⁰ is Arg or Gly; and
A⁴⁰ is Acp or deleted.

A preferred embodiment of the foregoing aspect of the invention features a compound according to the formula:
(Aib²) Exendin-4(1-39)NH₂; (SEQ ID NO:4)
(Aib², Nle¹⁴) Exendin-4(1-39)NH₂; (SEQ ID NO:5)
(Aib², Leu¹⁴) Exendin-4(1-39)NH₂; (SEQ ID NO:5)
(Aib², A6c¹⁴) Exendin-4(1-39)NH₂; (SEQ ID NO:5)
(Aib², Arg^{12,27}, Nle¹⁴) Exendin-4(1-39)NH₂; (SEQ ID NO:6)
(Aib², Arg¹²,²⁷, Leu¹⁴) Exendin-4(1-39)NH₂; (SEQ ID NO:6)
(Aib^{2,28}, Nle¹⁴) Exendin-4(1-39)NH₂; (SEQ ID NO:7)
(Aib^{2,28}, Leu¹⁴) Exendin-4(1-39)NH₂; (SEQ ID NO:7)
(Aib^{2,29}, Nle¹⁴) Exendin-4(1-39)NH₂; (SEQ ID NO:8)
(Aib^{2,29}, Leu¹⁴) Exendin-4(1-39)NH₂; (SEQ ID NO:8)
(Aib², Nle¹⁴, *β*-Ala²⁹) Exendin-4(1-39)NH₂; (SEQ ID NO:9)
(Aib², Leu¹⁴, *β*-Ala²⁹) Exendin-4(1-39)NH₂; (SEQ ID NO:9)
(Aib², *β*-Ala²⁹) Exendin-4(1-39)NH₂; (SEQ ID NO:9)
(Aib^{2,29}) Exendin-4(1-39)NH₂; (SEQ ID NO:8)
(Aib², Nle¹⁴, A6c²⁶) Exendin-4(1-39)NH₂; (SEQ ID NO:10)
(Aib², Leu¹⁴, A6c²⁶) Exendin-4(1-39)NH₂; (SEQ ID NO:10)
(Aib², A6c²⁶) Exendin-4(1-39)NH₂; (SEQ ID NO:10)
(Aib², A6c¹⁴,) Exendin-4(1-39)NH₂; (SEQ ID NO:5)
(Aib², A6c¹⁰, Leu¹⁴) Exendin-4(1-39)NH₂; (SEQ ID NO:11)
(Aib², A6c¹⁰, Nle¹⁴) Exendin-4(1-39)NH₂; (SEQ ID NO:11)
(Aib², A6c^{10,14}) Exendin-4(1-39)NH₂; (SEQ ID NO:11)
(Aib^{2,18}, Nle¹⁴) Exendin-4(1-39)NH₂; (SEQ ID NO:12)
(Aib^{2,18}, Leu¹⁴) Exendin-4(1-39)NH₂; (SEQ ID NO:12)
(Aib^{2,18}) Exendin-4(1-39)NH₂; (SEQ ID NO:12)
(Aib^{2,11}, Nle¹⁴) Exendin-4(1-39)NH₂; (SEQ ID NO:13)
(Aib^{2,11}, Leu¹⁴) Exendin-4(1-39)NH₂; (SEQ ID NO:13)
(Aib^{2,11}) Exendin-4(1-39)NH₂; (SEQ ID NO:13)
(D-Ala², Nle¹⁴) Exendin-4(1-39)NH₂; (SEQ ID NO:14)
(D-Ala², Leu¹⁴) Exendin-4(1-39)NH₂; (SEQ ID NO:14)
(D-Ala²) Exendin-4(1-39)NH₂; (SEQ ID NO:14)
(Aib², A6c^{14,26}) Exendin-4(1-39)NH₂; (SEQ ID NO:10)
(Aib^{2,18}, Nle¹⁴) Exendin-4(1-39)NH₂; (SEQ ID NO:12)
(Hppa¹, Nle¹⁴) Exendin-4(1-39)NH₂; (SEQ ID NO:15)
(Hppa¹, Leu¹⁴) Exendin-4(1-39)NH₂; (SEQ ID NO:15)
(Hppa¹) Exendin-4(1-39)NH₂; (SEQ ID NO:15)
(Ura¹, Nle¹⁴) Exendin-4(1-39)NH₂; (SEQ ID NO:16)
(Paa¹, Nle¹⁴) Exendin-4(1-39)NH₂; (SEQ ID NO:16)
(Pta¹, Nle¹⁴) Exendin-4(1-39)NH₂; (SEQ ID NO:16)
(Aib², Nle¹⁴, Ser³²(O-decanoyl)) Exendin-4(1-39)NH₂; (SEQ ID NO:17)
(Aib², Leu¹⁴, Ser³²(O-decanoyl)) Exendin-4(1-39)NH₂; (SEQ ID NO:17)
(Aib², Nle¹⁴, Ser³³(O-decanoyl)) Exendin-4(1-39)NH₂; (SEQ ID NO:18)
(Aib², Leu¹⁴, Ser³³(O-decanoyl)) Exendin-4(1-39)NH₂; (SEQ ID NO:18)
(Aib², Nle¹⁴, Ser³⁹(O-decanoyl)) Exendin-4(1-39)NH₂; (SEQ ID NO:19)
(Aib², Leu¹⁴, Ser³⁹(O-decanoyl)) Exendin-4(1-39)NH₂; (SEQ ID NO:19) (Aib², Nle¹⁴, Lys²⁷(N^{ε}-octanoyl)) Exendin-4(1-39)NH₂; (SEQ ID NO:20) (Aib², Leu¹⁴, Lys²⁷(N^{ε}-octanoyl)) Exendin-4(1-39)NH₂; (SEQ ID NO:20) (Aib², Arg¹², Nle¹⁴, Lys²⁷(N^{ε}-octanoyl)) Exendin-4(1-39)NH₂; (SEQ ID NO:21) (Aib², Arg¹², Leu¹⁴, Lys²⁷(N^{ε}-octanoyl)) Exendin-4(1-39)NH₂; (SEQ ID NO:21) (Aib², Lys¹²(N^{ε}-octanoyl), Nle¹⁴, Arg²⁷) Exendin-4(1-39)NH₂; (SEQ ID NO:22) (Aib², Lys¹²(N^{ε}-octanoyl), Leu¹⁴, Arg²⁷) Exendin-4(1-39)NH₂; (SEQ ID NO:22) (Aib², Arg^{12,27}, Nle¹⁴, Ava⁴⁰, Aun⁴¹) Exendin-4(1-41)NH₂; (SEQ ID NO:94) (Aib², Arg^{12,27}, Nle¹⁴, Asp⁴⁰, Ava⁴¹, Aun⁴²) Exendin-4(1-42)NH₂; (SEQ ID NO:23)
(Aib², Arg^{12,27}, Nle¹⁴, D-Asp⁴⁰, Ava⁴¹, Aun⁴²) Exendin-4(1-42)NH₂; (SEQ ID NO:23)
(Aib²) Exendin-3(1-39)NH₂; (SEQ ID NO:24)
(Aib², Nle¹⁴) Exendin-3(1-39)NH₂; (SEQ ID NO:25)
(Aib², Leu¹⁴) Exendin-3(1-39)NH₂; (SEQ ID NO:25)
(Aib², A6c¹⁴) Exendin-3(1-39)NH₂; (SEQ ID NO:25)
(Aib², Arg^{12,27}, Nle¹⁴) Exendin-3(1-39)NH₂; (SEQ ID NO:26)
(Aib², Arg^{12,27}, Leu¹⁴) Exendin-3(1-39)NH₂; (SEQ ID NO:26)
(Aib^{2,28}, Nle¹⁴) Exendin-3(1-39)NH₂; (SEQ ID NO:27)
(Aib^{2,28}, Leu¹⁴) Exendin-3(1-39)NH₂; (SEQ ID NO:27)
(Aib^{2,29}, Nle¹⁴) Exendin-3(1-39)NH₂; (SEQ ID NO:28)
(Aib^{2,29}, Leu¹⁴) Exendin-3(1-39)NH₂; (SEQ ID NO:28)
(Aib², Nle¹⁴, *β*-Ala²⁹) Exendin-3(1-39)NH₂; (SEQ ID NO:29)
(Aib², Leu¹⁴, β-Ala²⁹) Exendin-3(1-39)NH₂; (SEQ ID NO:29)
(Aib², *β*-Ala²⁹) Exendin-3(1-39)NH₂; (SEQ ID NO:29)
(Aib^{2,29}) Exendin-3(1-39)NH₂; (SEQ ID NO:28)
(Aib², Nle¹⁴, A6c²⁶) Exendin-3(1-39)NH₂; (SEQ ID NO:30)
(Aib², Leu¹⁴, A6c²⁶) Exendin-3(1-39)NH₂; (SEQ ID NO:30)
(Aib², A6c²⁶) Exendin-3(1-39)NH₂; (SEQ ID NO:30)
(Aib², A6c¹⁴) Exendin-3(1-39)NH₂; (SEQ ID NO:25)
(Aib², A6c¹⁰, Leu¹⁴) Exendin-3(1-39)NH₂; (SEQ ID NO:31)
(Aib², A6c¹⁰, Nle¹⁴) Exendin-3(1-39)NH₂; (SEQ ID NO:31)
(Aib², A6c^{10,14}) Exendin-3(1-39)NH₂; (SEQ ID NO:31)
(Aib^{2,18}, Nle¹⁴) Exendin-3(1-39)NH₂; (SEQ ID NO:32)
(Aib^{2,18}, Leu¹⁴) Exendin-3(1-39)NH₂; (SEQ ID NO:32)
(Aib^{2,11}, Nle¹⁴) Exendin-3(1-39)NH₂; (SEQ ID NO:33)
(Aib^{2,11}, Leu¹⁴) Exendin-3(1-39)NH₂; (SEQ ID NO:33)
(Aib^{2,11}) Exendin-3(1-39)NH₂; (SEQ ID NO:33)
(D-Ala², Nle¹⁴) Exendin-3(1-39)NH₂; (SEQ ID NO:34)
(D-Ala², Leu¹⁴) Exendin-3(1-39)NH₂; (SEQ ID NO:34)
(D-Ala²) Exendin-3(1-39)NH₂; (SEQ ID NO:34)
(Aib², A6c^{14,26}) Exendin-3(1-39)NH₂; (SEQ ID NO:30)
(Aib^{2,18}) Exendin-3(1-39)NH₂; (SEQ ID NO:32)
(Hppa¹, Nle¹⁴) Exendin-3(1-39)NH₂; (SEQ ID NO:35)
(Hppa¹, Leu¹⁴) Exendin-3(1-39)NH₂; (SEQ ID NO:35)
(Hppa¹) Exendin-3(1-39)NH₂; (SEQ ID NO:35)
(Ura¹, Nle¹⁴) Exendin-3(1-39)NH₂; (SEQ ID NO:36)
(Paa¹, Nle¹⁴) Exendin-3(1-39)NH₂; (SEQ ID NO:36)
(Pta¹, Nle¹⁴) Exendin-3(1-39)NH₂; (SEQ ID NO:36)
(Aib², Nle¹⁴, Ser³²(O-decanoyl)) Exendin-3(1-39)NH₂; (SEQ ID NO:37)
(Aib², Leu¹⁴, Ser³²(O-decanoyl)) Exendin-3(1-39)NH₂; (SEQ ID NO:37)
(Aib², Nle¹⁴, Ser³³(O-decanoyl)) Exendin-3(1-39)NH₂; (SEQ ID NO:38)
(Aib², Leu¹⁴, Ser³³(O-decanoyl)) Exendin-3(1-39)NH₂; (SEQ ID NO:38)
(Aib², Nle¹⁴, Ser³⁹(O-decanoyl)) Exendin-3(1-39)NH₂; (SEQ ID NO:39)
(Aib², Leu¹⁴, Ser³⁹(O-decanoyl)) Exendin-3(1-39)NH₂; (SEQ ID NO:39)
(Aib², Nle¹⁴, Ser¹⁷(O-decanoyl)) Exendin-3(1-39)NH₂; (SEQ ID NO:40)
(Aib², Leu¹⁴, Ser¹⁷(O-decanoyl)) Exendin-3(1-39)NH₂; (SEQ ID NO:40)
(Aib², Nle¹⁴, Lys²⁷(N^{ε}-octanoyl)) Exendin-3(1-39)NH₂;(SEQ ID NO:41)
(Aib², Leu¹⁴, Lys²⁷(N^{ε}-octanoyl)) Exendin-3(1-39)NH₂; (SEQ ID NO:41)
(Aib², Arg¹², Nle¹⁴, Lys²⁷(N^{ε}-octanoyl)) Exendin-3(1-39)NH₂; (SEQ ID NO:42)
(Aib², Arg¹², Leu¹⁴, Lys²⁷(N^{ε}-octanoyl)) Exendin-3(1-39)NH₂; (SEQ ID NO:42)
(Aib², Lys¹²(N^{ε}-octanoyl), Nle¹⁴, Arg²⁷) Exendin-3(1-39)NH₂; (SEQ ID NO:43)
(Aib², Lys¹²(N^{ε}-octanoyl), Leu¹⁴, Arg²⁷) Exendin-3(1-39)NH₂; (SEQ ID NO:43)
(Aib², Arg^{12,27}, Nle¹⁴, Ava⁴⁰, Aun⁴¹) Exendin-3(1-41)NH₂; (SEQ ID NO:95)
(Aib², Arg^{12,27}, Nle¹⁴, Asp⁴⁰, Ava⁴¹, Aun⁴²) Exendin-3(1-42)NH₂; (SEQ ID NO:44)
(Aib², Arg^{12,27}, Nle¹⁴, D-Asp⁴⁰, Ava⁴¹, Aun⁴²) Exendin-3(1-42)NH₂; (SEQ ID NO:44)
(Aib², Nle¹⁴, *β*-Ala²⁹, Arg³⁰) Exendin-4(1-30)NH₂; (SEQ ID NO:45)
(Aib², Leu¹⁴, *β*-Ala²⁹, Arg³⁰) Exendin-4(1-30)NH₂; (SEQ ID NO:45)
(Aib², A6c¹⁴, *β*-Ala²⁹, Arg³⁰) Exendin-4(1-30)NH₂; (SEQ ID NO:45)
(Aib^{2,29}, Nle¹⁴, Arg³⁰) Exendin-4(1-30)NH₂; (SEQ ID NO:46)
(Aib^{2,29}, Leu¹⁴, Arg³⁰) Exendin-4(1-30)NH₂; (SEQ ID NO:46)
(Aib^{2,29}, A6c¹⁴, Arg³⁰) Exendin-4(1-30)NH₂; (SEQ ID NO:46)
(Aib², Nle¹⁴, *β*-Ala²⁹, D-Arg³⁰) Exendin-4(1-30)NH₂; (SEQ ID NO:47)
(Aib², Leu¹⁴, *β*-Ala²⁹, D-Arg³⁰) Exendin-4(1-30)NH₂; (SEQ ID NO:47)
(Aib^{2,29}, Nle¹⁴, D-Arg³⁰) Exendin-4(1-30)NH₂; (SEQ ID NO:48)
(Aib^{2,29}, Leu¹⁴, D-Arg³⁰) Exendin-4(1-30)NH₂; (SEQ ID NO:48)
(Aib², Arg^{12,27,30}, Nle¹⁴, *β*-Ala²⁹) Exendin-4(1-30)NH₂; (SEQ ID NO:49)
(Aib², Arg^{12,27,30}, Leu¹⁴, *β*-Ala²⁹) Exendin-4(1-30)NH₂; (SEQ ID NO:49)
(Aib^{2,29}, Arg^{12,27,30}, Nle¹⁴) Exendin-4(1-30)NH₂; (SEQ ID NO:50)
(Aib^{2,29}, Arg^{12,27,30}, Leu¹⁴) Exendin-4(1-30)NH₂; (SEQ ID NO:50)
(Nle¹⁴, *β*-Ala²⁹, Arg³⁰) Exendin-4(1-30)NH₂; (SEQ ID NO:51)
(Leu¹⁴, *β*-Ala²⁹, Arg³⁰) Exendin-4(1-30)NH₂; (SEQ ID NO:51)
(Nle¹⁴, Aib²⁹, Arg³⁰) Exendin-4(1-30)NH₂; (SEQ ID NO:52)
(Leu¹⁴, Aib²⁹, Arg³⁰) Exendin-4(1-30)NH₂; (SEQ ID NO:52)
(Aib², *β*-Ala²⁹, Arg³⁰) Exendin-4(1-30)NH₂; (SEQ ID NO:45)
(Aib^{2,29}, Arg³⁰) Exendin-4(1-30)NH₂; (SEQ ID NO:46)
(*β*-Ala²⁹, Arg³⁰) Exendin-4(1-30)NH₂; (SEQ ID NO:51)
(Aib²⁹, Arg³⁰) Exendin-4(1-30)NH₂; (SEQ ID NO:52)
(Aib², Nle¹⁴, *β*-Ala²⁹) Exendin-4(1-30)NH₂; (SEQ ID NO:53)
(Aib², Leu¹⁴, *β*-Ala²⁹) Exendin-4(1-30)NH₂; (SEQ ID NO:53)
(Aib^{2,29}, Nle¹⁴) Exendin-4(1-30)NH₂; (SEQ ID NO:54)
(Aib^{2,29}, Leu¹⁴) Exendin-4(1-30)NH₂; (SEQ ID NO:54)
(Nle¹⁴, *β*-Ala²⁹) Exendin-4(1-30)NH₂; (SEQ ID NO:55)
(Leu¹⁴, *β*-Ala²⁹) Exendin-4(1-30)NH₂; (SEQ ID NO:55)
(Nle¹⁴, Aib²⁹) Exendin-4(1-30)NH₂; (SEQ ID NO:56)
(Leu¹⁴, Aib²⁹) Exendin-4(1-30)NH₂; (SEQ ID NO:56)
(Aib^{2,29}) Exendin-4(1-30)NH₂; (SEQ ID NO:54)
(*β*-Ala²⁹) Exendin-4(1-30)NH₂; (SEQ ID NO:55)
(Aib²⁹) Exendin-4(1-30)NH₂; (SEQ ID NO:56)
(Aib², Nle¹⁴, Ser³¹(O-decanoyl)) Exendin-4(1-31)NH₂; (SEQ ID NO:57)
(Aib², Leu¹⁴, Ser³¹(O-decanoyl)) Exendin-4(1-31)NH₂; (SEQ ID NO:57)
(Aib², Nle¹⁴ Ser¹⁷(O-decanoyl)) Exendin-4(1-30)NH₂; (SEQ ID NO:58)
(Aib², Leu¹⁴, Ser¹⁷(O-decanoyl)) Exendin-4(1-30)NH₂; (SEQ ID NO:58)
(Aib², Nle¹⁴, Lys²⁷(N^{ε}-octanoyl)) Exendin-4(1-30)NH₂; (SEQ ID NO:59)
(Aib², Leu¹⁴, Lys²⁷(N^{ε}-octanoyl)) Exendin-4(1-30)NH₂; (SEQ ID NO:59)
(Aib², Arg¹², Nle¹⁴, Lys²⁷(N^{ε}-octanoyl)) Exendin-4(1-30)NH₂; (SEQ ID NO:60)
(Aib², Arg¹², Leu¹⁴, Lys²⁷(N^{ε}-octanoyl)) Exendin-4(1-30)NH₂; (SEQ ID NO:60)
(Aib², Lys¹²(N^{ε}-octanoyl), Nle¹⁴, Arg²⁷) Exendin-4(1-30)NH₂; (SEQ ID NO:61)
(Aib², Lys¹²(N^{ε}-octanoyl), Leu¹⁴, Arg²⁷) Exendin-4(1-30)NH₂; (SEQ ID NO:61)
(Aib², Arg^{12,27,30}, Nle¹⁴, Ava³¹, Aun³²) Exendin-4(1-32)NH₂; (SEQ ID NO:96)
(Aib², Arg^{12, 27, 30}, Nle¹⁴, Asp³¹, Ava³², Aun³³) Exendin-4(1-32)NH₂; (SEQ ID NO:62)
(Aib², Arg^{12, 27, 30}, Nle¹⁴, D-Asp³¹, Ava³², Aun³³) Exendin-4(1-32)NH₂; (SEQ ID NO:62)
(Aib², Nle¹⁴, *β*-Ala²⁹, Arg³⁰) Exendin-3(1-30)NH₂; (SEQ ID NO:63)
(Aib², Leu¹⁴, *β*-Ala²⁹, Arg³⁰) Exendin-3(1-30)NH₂; (SEQ ID NO:63)
(Aib², A6c¹⁴, *β*-Ala²⁹, Arg³⁰) Exendin-3(1-30)NH₂; (SEQ ID NO:63)
(Aib^{2,29}, Nle¹⁴, Arg³⁰) Exendin-3(1-30)NH₂; (SEQ ID NO:64)
(Aib^{2,29}, Leu¹⁴, Arg³⁰) Exendin-3(1-30)NH₂; (SEQ ID NO:64)
(Aib^{2,29}, A6c¹⁴, Arg³⁰) Exendin-3(1-30)NH₂; (SEQ ID NO:64)
(Aib², Nle¹⁴, *β*-Ala²⁹, D-Arg³⁰) Exendin-3(1-30)NH₂; (SEQ ID NO:65)
(Aib², Leu¹⁴, *β*-Ala²⁹, D-Arg³⁰) Exendin-3(1-30)NH₂; (SEQ ID NO:65)
(Aib^{2,29}, Nle¹⁴, D-Arg³⁰) Exendin-3(1-30)NH₂; (SEQ ID NO:66)
(Aib^{2,29}, Leu¹⁴, D-Arg³⁰) Exendin-3(1-30)NH₂; (SEQ ID NO:66)
(Aib², Arg^{12,27,30}, Nle¹⁴ *β*-Ala²⁹) Exendin-3(1-30)NH₂; (SEQ ID NO:67)
(Aib², Arg^{12,27,30}, Leu¹⁴, *β*-Ala²⁹) Exendin-3(1-30)NH₂; (SEQ ID NO:67)
(Aib^{2,29}, Arg^{12,27,30}, Nle¹⁴) Exendin-3(1-30)NH₂; (SEQ ID NO:68)
(Aib^{2,29}, Arg^{12,27,30}, Leu¹⁴) Exendin-3(1-30)NH₂; (SEQ ID NO:68)
(Nle¹⁴, *β*-Ala²⁹, Arg³⁰) Exendin-3(1-30)NH₂; (SEQ ID NO:69)
(Leu¹⁴, *β*-Ala²⁹, Arg³⁰) Exendin-3(1-30)NH₂; (SEQ ID NO:69)
(Nle¹⁴, Aib29, Arg³⁰) Exendin-3(1-30)NH₂; (SEQ ID NO:70)
(Leu¹⁴, Aib²⁹, Arg³⁰) Exendin-3(1-30)NH₂; (SEQ ID NO:70)
(Aib², *β*-Ala²⁹, Arg³⁰) Exendin-3(1-30)NH₂; (SEQ ID NO:63)
(Aib^{2,29}, Arg³⁰) Exendin-3(1-30)NH₂; (SEQ ID NO:64)
(*β*-Ala²⁹, Arg³⁰) Exendin-3(1-30)NH₂; (SEQ ID NO:69)
(Aib²⁹, Arg³⁰) Exendin-3(1-30)NH₂; (SEQ ID NO:70)
(Aib², Nle¹⁴, *β*-Ala²⁹) Exendin-3(1-30)NH₂; (SEQ ID NO:71)
(Aib², Leu¹⁴, *β*-Ala²⁹) Exendin-3(1-30)NH₂; (SEQ ID NO:71)
(Aib^{2,29}, Nle¹⁴) Exendin-3(1-30)NH₂; (SEQ ID NO:72)
(Aib^{2,29}, Leu¹⁴) Exendin-3(1-30)NH₂; (SEQ ID NO:72)
(Nle¹⁴, *β*-Ala²⁹) Exendin-3(1-30)NH₂; (SEQ ID NO:73)
(Leu¹⁴, *β*-Ala²⁹) Exendin-3(1-30)NH₂; (SEQ ID NO:73)
(Nle¹⁴, Aib²⁹) Exendin-3(1-30)NH₂; (SEQ ID NO:74)
(Leu¹⁴, Aib²⁹) Exendin-3(1-30)NH₂; (SEQ ID NO:74)
(Aib^{2,29}) Exendin-3(1-30)NH₂; (SEQ ID NO:72)
(*β*-Ala²⁹) Exendin-3(1-30)NH₂; (SEQ ID NO:73)
(Aib²⁹) Exendin-3(1-30)NH₂; (SEQ ID NO:74)
(Aib², Nle¹⁴, Ser³¹(O-decanoyl)) Exendin-3(1-31)NH₂; (SEQ ID NO:75)
(Aib², Leu¹⁴, Ser³¹(O-decanoyl)) Exendin-3(1-31)NH₂; (SEQ ID NO:75)
(Aib², Nle¹⁴, Ser¹⁷(O-decanoyl)) Exendin-3(1-30)NH₂; (SEQ ID NO:76)
(Aib², Leu¹⁴, Ser¹⁷(O-decanoyl)) Exendin-3(1-30)NH₂; (SEQ ID NO:76)
(Aib², Nle¹⁴, Lys²⁷(N^{ε}-octanoyl)) Exendin-3(1-30)NH₂; (SEQ ID NO:77)
(Aib², Leu¹⁴, Lys²⁷(N^{ε}-octanoyl)) Exendin-3(1-30)NH₂; (SEQ ID NO:77)
(Aib², Arg¹², Nle¹⁴, Lys²⁷(N^{ε}-octanoyl)) Exendin-3(1-30)NH₂; (SEQ ID NO:78)
(Aib², Arg¹², Leu¹⁴, Lys²⁷(N^{ε}-octanoyl)) Exendin-3(1-30)NH₂; (SEQ ID NO:78)
(Aib², Lys¹² (N^{ε}-octanoyl), Nle¹⁴, Arg²⁷) Exendin-3(1-30)NH₂; (SEQ ID NO:79)
(Aib², Lys¹² (N^{ε}-octanoyl), Leu¹⁴, Arg²⁷) Exendin-3(1-30)NH₂; (SEQ ID NO:79)
(Aib², Arg^{12,27,30}, Nle¹⁴, Ava³¹, Aun³²) Exendin-3(1-32)NH₂; (SEQ ID NO:97)
(Aib², Arg^{12,27,30}, Nle¹⁴, Asp³¹, Ava³², Aun³³) Exendin-3(1-33)NH₂; (SEQ ID NO:80)
(Aib², Arg^{12,27,30}, Nle¹⁴, D-Asp³¹, Ava³², Aun³³) Exendin-3(1-33)NH₂; (SEQ ID NO:80)
(Aib², Arg^{12,27}, Nle¹⁴, *β*-Ala²⁹) Exendin-4(1-39)NH₂; (SEQ ID NO:81)
(Aib², Arg^{12,27}, Leu¹⁴, *β*-Ala²⁹) Exendin-4(1-39)NH₂; (SEQ ID NO:81)
(Aib², Arg^{12,27}, A6c¹⁴, *β*-Ala²⁹) Exendin-4(1-39)NH₂; (SEQ ID NO:81)
(Aib², Arg^{12,27}, *β*-Ala²⁹) Exendin-4(1-39)NH₂; (SEQ ID NO:81)
(Nle¹⁴, *β*-Ala²⁹) Exendin-4(1-39)NH₂; (SEQ ID NO:82)
(Leu¹⁴, Aib²⁹) Exendin-4(1-39)NH₂; (SEQ ID NO:83)
(*β*-Ala²⁹) Exendin-4(1-39)NH₂; (SEQ ID NO:82)
(Aib²⁹) Exendin-4(1-39)NH₂; (SEQ ID NO:83)
(Nle¹⁴, Aib²⁹) Exendin-4(1-39)NH₂; (SEQ ID NO:83)
(Leu¹⁴, Aib²⁹) Exendin-4(1-39)NH₂; (SEQ ID NO:83)
(Aib², Arg^{12,27}, Nle¹⁴, Phe²⁵, *β*-Ala²⁹) Exendin-4(1-39)NH₂; (SEQ ID NO:84)
(Aib², Arg^{12,27}, Leu¹⁴, Phe²⁵, *β*-Ala²⁹) Exendin-4(1-39)NH₂; (SEQ ID NO:84)
(Aib², Arg^{12,27}, A6c¹⁴, Phe²⁵, *β*-Ala²⁹) Exendin-4(1-39)NH₂; (SEQ ID NO:84)
(Aib², Arg^{12,27}, Phe²⁵, *β*-Ala²⁹) Exendin-4(1-39)NH₂; (SEQ ID NO:84)
(Aib², Nle¹⁴, Phe²⁵, *β*-Ala²⁹) Exendin-4(1-39)NH₂; (SEQ ID NO:85)
(Aib², Leu¹⁴, Phe²⁵, *β*-Ala²⁹) Exendin-4(1-39)NH₂; (SEQ ID NO:85)
(Aib², A6c¹⁴, Phe²⁵, *β*-Ala²⁹) Exendin-4(1-39)NH₂; (SEQ ID NO:85)
(Aib², Phe²⁵, *β*-Ala²⁹) Exendin-4(1-39)NH₂; (SEQ ID NO:85)
(Aib², Arg^{12,27,30}, Nle¹⁴, *β*-Ala²⁹, Acp³¹) Exendin-4(1-31)NH₂; (SEQ ID NO:86)
(Aib², Nle¹⁴, *β*-Ala²⁹, Arg³⁰, Acp³¹) Exendin-4(1-31)NH₂; (SEQ ID NO:87)
(Aib², Arg^{12,27}, Nle¹⁴ *β*-Ala²⁹, Acp⁴⁰) Exendin-4(1-40)NH₂; (SEQ ID NO:88)
(Aib², Nle¹⁴, *β*-Ala²⁹, Acp⁴⁰) Exendin-4(1-40)NH₂; (SEQ ID NO:89)
(Aib², Lys¹²(N^{ε}-Acp-octanoyl), Nle¹⁴, *β*-Ala²⁹) Exendin-4(1-39)NH₂; (SEQ ID NO:90)
(Aib², Lys¹²(N^{ε}-Acp-octanoyl), Nle¹⁴, Arg²⁷, *β*-Ala²⁹) Exendin-4(1-39)NH₂; (SEQ ID NO:90)
(Aib², Nle¹⁴, Lys²⁷(N^{ε}-Acp-octanoyl), *β*-Ala²⁹) Exendin-4(1-39)NH₂; (SEQ ID NO:91)
(Aib², Arg¹² Nle¹⁴, Lys²⁷(N^{ε}-Acp-octanoyl), *β*-Ala²⁹) Exendin-4(1-39)NH₂; (SEQ ID NO:91)
(Aib², Arg^{12,27}, Nle¹⁴, *β*-Ala²⁹, Acp⁴⁰) Exendin-4(1-40)NH₂; (SEQ ID NO:88) or
(Aib², Nle¹⁴, Lys²⁷(N^{ε}-Acp-decanoyl), *β*-Ala²⁹) Exendin-4(1-39)NH₂; (SEQ ID NO:92)
or a pharmaceutically acceptable salt thereof.

A more preferred embodiment of the foregoing aspect of the invention features a compound according to the formula:
(Aib²) Exendin-4(1-39)NH₂; (SEQ ID NO:4)
(Aib², Nle¹⁴) Exendin-4(1-39)NH₂; (SEQ ID NO:5)
(Aib², Leu") Exendin-4(1-39)NH₂; (SEQ ID NO:5)
(Aib², A6c¹⁴) Exendin-4(1-39)NH₂; (SEQ ID NO:5)
(Aib², Arg^{12,27}, Nle¹⁴) Exendin-4(1-39)NH₂; (SEQ ID NO:6)
(Aib², Arg^{12,27}, Leu¹⁴) Exendin-4(1-39)NH₂; (SEQ ID NO:6)
(Aib^{2,28}, Nle¹⁴) Exendin-4(1-39)NH₂; (SEQ ID NO:7)
(Aib^{2,28}, Leu¹⁴) Exendin-4(1-39)NH₂; (SEQ ID NO:7)
(Aib^{2,29}, Nle¹⁴) Exendin-4(1-39)NH₂; (SEQ ID NO:8)
(Aib^{2,29}, Leu¹⁴) Exendin-4(1-39)NH₂; (SEQ ID NO:8)
(Aib², Nle¹⁴ *β*-Ala²⁹) Exendin-4(1-39)NH₂; (SEQ ID NO:9)
(Aib², Leu¹⁴, *β*-Ala²⁹) Exendin-4(1-39)NH₂; (SEQ ID NO:9)
(Aib², *β*-Ala²⁹) Exendin-4(1-39)NH₂; (SEQ ID NO:9)
(Aib^{2,29}) Exendin-4(1-39)NH₂; (SEQ ID NO:8)
(Aib², Nle¹⁴, A6c²⁶) Exendin-4(1-39)NH₂; (SEQ ID NO:10)
(Aib², Leu¹⁴, A6c²⁶) Exendin-4(1-39)NH₂; (SEQ ID NO:10)
(Aib², A6c¹⁴) Exendin-4(1-39)NH₂; (SEQ ID NO:5)
(Aib², Nle¹⁴, Ser³⁹(O-decanoyl)) Exendin-4(1-39)NH₂; (SEQ ID NO:19)
(Aib², Leu¹⁴, Ser³⁹(O-decanoyl)) Exendin-4(1-39)NH₂; (SEQ ID NO:19)
(Aib², Nle¹⁴, Lys²⁷(N^{ε}-octanoyl)) Exendin-4(1-39)NH₂; (SEQ ID NO:20)
(Aib², Leu¹⁴, Lys²⁷(N^{ε}-octanoyl)) Exendin-4(1-39)NH₂; (SEQ ID NO:20)
(Aib², Arg¹², Nle¹⁴, Lys²⁷(N^{ε}-octanoyl)) Exendin-4(1-39)NH₂; (SEQ ID NO:21)
(Aib², Arg¹², Leu¹⁴, Lys²⁷(N^{ε}-octanoyl)) Exendin-4(1-39)NH₂; (SEQ ID NO:21)
(Aib², Lys¹² (N^{ε}-octanoyl), Nle¹⁴, Arg²⁷) Exendin-4(1-39)NH₂; (SEQ ID NO:43)
(Aib², Arg^{12,27}, Nle¹⁴) Exendin-3(1-39)NH₂; (SEQ ID NO:26)
(Aib², Arg^{12,27}, Leu¹⁴) Exendin-3(1-39)NH₂; (SEQ ID NO:26)
(Aib², Arg¹², Nle¹⁴, Lys²⁷(N^{ε}-octanoyl)) Exendin-3(1-39)NH₂; (SEQ ID NO:42)
(Aib², Nle¹⁴, *β*-Ala²⁹, Arg³⁰) Exendin-4(1-30)NH₂; (SEQ ID NO:45)
(Aib², Leu¹⁴, *β*-Ala²⁹, Arg³⁰) Exendin-4(1-30)NH₂; (SEQ ID NO:45)
(Aib^{2,29}, Nle¹⁴, Arg³⁰) Exendin-4(1-30)NH₂; (SEQ ID NO:46)
(Aib^{2,29}, Leu¹⁴, Arg³⁰) Exendin-4(1-30)NH₂; (SEQ ID NO:46)
(Aib², Arg^{12,27,30}, Nle¹⁴, *β*-Ala²⁹) Exendin-4(1-30)NH₂; (SEQ ID NO:49)
(Aib², Arg^{12,27,30}, Leu¹⁴, *β*-Ala²⁹) Exendin-4(1-30)NH₂; (SEQ ID NO:49)
(Aib²,²⁹, Arg¹²,²⁷,³⁰, Nle¹⁴) Exendin-4(1-30)NH₂; (SEQ ID NO:50)
(Aib^{2,29}, Arg^{12,27,30}, Leu¹⁴) Exendin-4(1-30)NH₂; (SEQ ID NO:50)
(Nle¹⁴, *β*-Ala²⁹, Arg³⁰) Exendin-4(1-30)NH₂; (SEQ ID NO:51)
(Leu¹⁴, *β*-Ala²⁹, Arg³⁰) Exendin-4(1-30)NH₂; (SEQ ID NO:51)
(Nle¹⁴, Aib²⁹, Arg³⁰) Exendin-4(1-30)NH₂; (SEQ ID NO:52)
(Leu¹⁴, Aib29, Arg³⁰) Exendin-4(1-30)NH₂; (SEQ ID NO:52)
(Aib², *β*-Ala²⁹, Arg³⁰) Exendin-4(1-30)NH₂; (SEQ ID NO:45)
(Aib^{2,29}, Arg³⁰) Exendin-4(1-30)NH₂; (SEQ ID NO:46)
(Aib², Nle¹⁴ *β*-Ala²⁹) Exendin-4(1-30)NH₂; (SEQ ID NO:53)
(Aib², Leu¹⁴, *β*-Ala²⁹) Exendin-4(1-30)NH₂; (SEQ ID NO:53)
(Aib^{2,29}, Nle¹⁴) Exendin-4(1-30)NH₂; (SEQ ID NO:54)
(Aib², Nle¹⁴, Ser³¹(O-decanoyl)) Exendin-4(1-31)NH₂; (SEQ ID NO:57)
(Aib², Arg¹², Nle¹⁴, Lys²⁷(N^{ε}-octanoyl)) Exendin-4(1-30)NH₂; (SEQ ID NO:60)
(Aib², Arg¹², Leu¹⁴, Lys²⁷(N^{ε}-octanoyl)) Exendin-4(1-30)NH₂; (SEQ ID NO:60)
(Aib², Nle¹⁴, *β*-Ala²⁹, Arg³⁰) Exendin-3(1-30)NH₂; (SEQ ID NO:63)
(Aib², Leu¹⁴, *β*-Ala²⁹, Arg³⁰) Exendin-3(1-30)NH₂; (SEQ ID NO:63)
(Aib^{2,29}, Nle¹⁴, Arg³⁰) Exendin-3(1-30)NH₂; (SEQ ID NO:64)
(Aib², Arg^{12,27}, Nle¹⁴ *β*-Ala²⁹) Exendin-4(1-39)NH₂;(SEQ ID NO:81)
(Aib², Arg^{12,27}, Leu¹⁴, *β*-Ala²⁹) Exendin-4(1-39)NH₂;(SEQ ID NO:81)
(Aib², Arg^{12,27}, Nle¹⁴, Phe²⁵, *β*-Ala²⁹) Exendin-4(1-39)NH₂; (SEQ ID NO:84)
(Aib², Arg^{12,27}, Leu¹⁴, Phe²⁵, *β*-Ala²⁹) Exendin-4(1-39)NH₂; (SEQ ID NO:84)
(Aib², Nle¹⁴, Phe²⁵, *β*-Ala²⁹) Exendin-4(1-39)NH₂; (SEQ ID NO:85)
(Aib², Leu¹⁴, Phe²⁵, *β*-Ala²⁹) Exendin-4(1-39)NH₂; (SEQ ID NO:85)
(Aib², Arg^{12,27,30}, Nle¹⁴, *β*-Ala²⁹, Acp³¹) Exendin-4(1-31)NH₂; (SEQ ID NO:86)
(Aib², Nle¹⁴, *β*-Ala²⁹, Arg³⁰, Acp³¹) Exendin-4(1-31)NH₂; (SEQ ID NO:87)
(Aib², Arg^{12,27}, Nle¹⁴, *β*-Ala²⁹, Acp⁴⁰) Exendin-4(1-40)NH₂; (SEQ ID NO:88)
(Aib², Nle¹⁴, *β*-Ala²⁹, Acp⁴⁰) Exendin-4(1-40)NH₂; (SEQ ID NO:89)
(Aib², Nle¹⁴, Lys²⁷(N^{ε}-Acp-octanoyl), *β*-Ala²⁹) Exendin-4(1-39)NH₂; (SEQ ID NO:91)
(Aib², Arg¹², Nle¹⁴ Lys²⁷(N^{ε}-Acp-octanoyl), *β*-Ala²⁹) Exendin-4(1-39)NH₂; (SEQ ID NO:91)
(Aib², Arg^{12,27}, Nle¹⁴, *β*-Ala²⁹, Acp⁴⁰) Exendin-4(1-40)NH₂; (SEQ ID NO:88) or
(Aib², Nle¹⁴, Lys²⁷(N^{ε}-Acp-decanoyl), *β*-Ala²⁹) Exendin-4(1-39)NH₂; (SEQ ID NO:92)
or a pharmaceutically acceptable salt thereof.

A still more preferred embodiment of the foregoing aspect of the invention features a compound according to the formula:
(Aib²) Exendin-4(1-39)NH₂; (SEQ ID NO:4)
(Aib², Nle¹⁴) Exendin-4(1-39)NH₂; (SEQ ID NO:5)
(Aib², Leu¹⁴) Exendin-4(1-39)NH₂; (SEQ ID NO:5)
(Aib², A6c¹⁴) Exendin-4(1-39)NH₂; (SEQ ID NO:5)
(Aib², Arg^{12,27}, Nle¹⁴) Exendin-4(1-39)NH₂; (SEQ ID NO:6)
(Aib², Arg^{12,27}, Leu¹⁴) Exendin-4(1-39)NH₂; (SEQ ID NO:6)
(Aib^{2,28}, Nle¹⁴) Exendin-4(1-39)NH₂; (SEQ ID NO:7)
(Aib^{2,29}, Nle¹⁴) Exendin-4(1-39)NH₂; (SEQ ID NO:8)
(Aib², Nle¹⁴ *β*-Ala²⁹) Exendin-4(1-39)NH₂; (SEQ ID NO:9)
(Aib², Leu¹⁴, *β*-Ala²⁹) Exendin-4(1-39)NH₂; (SEQ ID NO:9)
(Aib², Nle¹⁴ A6c²⁶) Exendin-4(1-39)NH₂; (SEQ ID NO:10)
(Aib², Nle¹⁴, Ser³⁹(O-decanoyl)) Exendin-4(1-39)NH₂; (SEQ ID NO:19)
(Aib², Nle¹⁴, Lys²⁷(N^{ε}-octanoyl)) Exendin-4(1-39)NH₂; (SEQ ID NO:20)
(Aib², Arg¹², Nle¹⁴, Lys²⁷(N^{ε}-octanoyl)) Exendin-4(1-39)NH₂; (SEQ ID NO:21)
(Aib², Nle¹⁴, *β*-Ala²⁹, Arg³⁰) Exendin-4(1-30)NH₂; (SEQ ID NO:45)
(Aib², Leu¹⁴, *β*-Ala²⁹, Arg³⁰) Exendin-4(1-30)NH₂; (SEQ ID NO:45)
(Aib^{2,29}, Nle¹⁴ Arg³⁰) Exendin-4(1-30)NH₂; (SEQ ID NO:46)
(Aib², Arg^{12,27,30}, Nle¹⁴, *β*-Ala²⁹) Exendin-4(1-30)NH₂; (SEQ ID NO:49)
(Aib², Arg^{12,27,30}, Leu¹⁴, *β*-Ala²⁹) Exendin-4(1-30)NH₂; (SEQ ID NO:49)
(Aib², Nle¹⁴, Ser³¹(O-decanoyl)) Exendin-4(1-31)NH₂; (SEQ ID NO:57)
(Aib², Arg¹², Nle¹⁴, Lys²⁷(N^{ε}-octanoyl)) Exendin-4(1-30)NH₂; (SEQ ID NO:60)
(Aib², Arg^{12,27}, Nle¹⁴, *β*-Ala²⁹) Exendin-4(1-39)NH₂; (SEQ ID NO:81)
(Aib², Arg^{12,27}, Leu¹⁴, *β*-Ala²⁹) Exendin-4(1-39)NH₂; (SEQ ID NO:81)
(Aib², Arg^{12,27}, Nle¹⁴, Phe²⁵, *β*-Ala²⁹) Exendin-4(1-39)NH₂; (SEQ ID NO:84)
(Aib², Arg^{12,27}, Leu¹⁴, Phe²⁵, *β*-Ala²⁹) Exendin-4(1-39)NH₂; (SEQ ID NO:84)
(Aib², Arg^{12,27,30}, Nle¹⁴, *β*-Ala²⁹, Acp³¹) Exendin-4(1-31)NH₂; (SEQ ID NO:86)
(Aib², Arg^{12,27}, Nle¹⁴, *β*-Ala²⁹, Acp⁴⁰) Exendin-4(1-40)NH₂; (SEQ ID NO:88)
(Aib², Arg¹², Nle¹⁴, Lys²⁷(N°-Acp-octanoyl), *β*-Ala²⁹) Exendin-4(1-39)NH₂; (SEQ ID NO:92)
(Aib², Arg^{12,27}, Nle¹⁴, *β*-Ala²⁹, Acp⁴⁰) Exendin-4(1-40)NH₂; (SEQ ID NO:88) or
(Aib², Nle¹⁴, Lys²⁷(N^{ε}-Acp-decanoyl), *β*-Ala²⁹) Exendin-4(1-39)NH₂; (SEQ ID NO:92)
or a pharmaceutically acceptable salt thereof.

A still more preferred embodiment of the foregoing aspect of the invention features a compound according to the formula:
(Aib²) Exendin-4(1-39)NH₂; (SEQ ID NO:4)
(Aib², Nle¹⁴) Exendin-4(1-39)NH₂; (SEQ ID NO:5)
(Aib², Leu¹⁴) Exendin-4(1-39)NH₂; (SEQ ID NO:5)
(Aib^{2,28}, Nle¹⁴) Exendin-4(1-39)NH₂; (SEQ ID NO:7)
(Aib^{2,29}, Nle¹⁴) Exendin-4(1-39)NH₂; (SEQ ID NO:8)
(Aib², Nle¹⁴, *β*-Ala²⁹) Exendin-4(1-39)NH₂; (SEQ ID NO:9)
(Aib², Nle¹⁴, A6c²⁶) Exendin-4(1-39)NH₂; (SEQ ID NO:10)
(Aib², Nle¹⁴, *β*-Ala²⁹, Arg³⁰) Exendin-4(1-30)NH₂; (SEQ ID NO:45)
(Aib^{2,29}, Me¹⁴, Arg³⁰) Exendin-4(1-30)NH₂; (SEQ ID NO:46)
(Aib², A6c¹⁴) Exendin-4(1-39)NH₂; (SEQ ID NO:5)
(Aib², Arg^{12,27}, Nle¹⁴, *β*-Ala²⁹, Acp⁴⁰) Exendin-4(1-40)NH₂; (SEQ ID NO:88) or
(Aib², Nle¹⁴, Lys²⁷(N^{ε}-Acp-decanoyl), *β*-Ala²⁹) Exendin-4(1-39)NH₂; (SEQ ID NO:92)
or a pharmaceutically acceptable salt thereof..

A most preferred embodiment of the foregoing aspect of the invention features a compound according to the formula:
(Aib², A6c¹⁴)Exendin-4(1-39)-NH₂; (SEQ ID NO:5)
(Aib², Nle¹⁴)Exendin-4(1-39)-NH₂; (SEQ ID NO:5)
(Aib², Nle¹⁴, *β*-Ala²⁹, Arg³⁰)Exendin-4(1-30)-NH₂; (SEQ ID NO:45)
(Aib^{2,28}, Nle¹⁴ )Exendin-4(1-39)-NH₂; (SEQ ID NO:7)
(Aib², Nle¹⁴, A6c26)Exendin-4(1-39)-NH₂; (SEQ ID NO:10)
(Aib^{2,29}, Nle¹⁴)Exendin-4(1-39)-NH₂; (SEQ ID NO:8)
(Aib², Leu¹⁴)Exendin-4(1-39)-NH₂; (SEQ ID NO:5)
(Aib², Nle¹⁴, *β*-Ala²⁹)Exendin-4(1-39)-NH₂; (SEQ ID NO:9) or
(Aib^{2,29}, Nle¹⁴, Arg³⁰)Exendin-4(1-30)-NH₂ (SEQ ID NO:46)

Another more preferred compound of formula (I) comprises each of the compounds that are specifically enumerated in the Examples section of the present disclosure, or a pharmaceutically acceptable salt thereof.

In another the present invention provides a method of binding a GLP-1 receptor which comprises the step of contacting said GLP-1 receptor with a compound of claim 1 or a pharmaceutically acceptable salt thereof. In one embodiment of this aspect the said GLP-1 receptor is in a human or non-human animal subject.

In another aspect, the present invention provides a pharmaceutical composition comprising an effective amount of a compound of formula (I) as defined hereinabove or a pharmaceutically acceptable salt thereof and a pharmaceutically acceptable carrier or diluent.

In another aspect the present invention provides a method of eliciting a GLP-1 agonist effect from a cell or cells, wherein said cell or cells comprises one or more GLP-1 receptors, said method comprising contacting said cell or cells with an effective amount of a compound of claim 1 or a pharmaceutically acceptable salt thereof.

In one embodiment of the immediately foregoing aspect, the present invention provides a method of eliciting a GLP-1 agonist effect in a cell or cells of a human or non-human animal subject in need thereof which comprises administering to said subject an effective amount of a compound of formula (I) as defined hereinabove or a pharmaceutically acceptable salt thereof.

In another embodiment of the immediately foregoing aspect, the present invention provides a method of treating a disease selected from the group consisting of Type I diabetes, Type II diabetes, obesity, hyperorexia, glucagonomas, secretory disorders of the airway, metabolic disorder, arthritis, osteoporosis, central nervous system disease, restenosis and neurodegenerative disease, in a subject in need thereof which comprises administering to said subject an effective amount of a compound of formula (I) as defined hereinabove or a pharmaceutically acceptable salt thereof. A preferred method of the immediately foregoing method is where the disease being treated is Type I diabetes or Type II diabetes.

In another embodiment of the immediately foregoing aspect the present invention provides a method of stimulating insulin release in a human or non-human animal subject in need thereof, which comprises administering to said subject an effective amount of a compound according to claim 1 or a pharmaceutically acceptable salt thereof.

In another embodiment of the immediately foregoing aspect the present invention provides a method of enhancing insulin sensitivity in a human or non-human animal subject in need thereof, which comprises administering to said subject an effective amount of a compound according to claim 1 or a pharmaceutically acceptable salt thereof.

In still another embodiment of the immediately foregoing aspect the present invention provides a method of lowering blood glucose levels in a human or non-human animal subject in need thereof, which comprises administering to said subject an effective amount of a compound according to claim 1 or a pharmaceutically acceptable salt thereof.

In yet still another embodiment of the immediately foregoing aspect the present invention provides a method of stimulating cAMP production in beta cells of a human or non-human animal subject in need thereof, which comprises administering to said subject an effective amount of a compound according to claim 1 or a pharmaceutically acceptable salt thereof.

In another aspect, the present invention provides a method of eliciting a GLP-1 antagonist effect from a cell or cells, wherein said cell or cells comprise one or more GLP-1 receptors, said method comprising contacting said cell or cells with an effective amount of a compound of claim 1 or a pharmaceutically acceptable salt thereof.

In one embodiment of the immediately foregoing aspect, the present invention provides a method of eliciting a GLP-1 antagonist effect in a cell or cells of a human or non-human animal subject in need thereof which comprises administering to said subject an effective amount of a compound of formula (I) as defined hereinabove or a pharmaceutically acceptable salt thereof.

In another embodiment of the immediately foregoing aspect, the present invention provides a method of treating a disease selected from the group consisting of cachexia, anorexia, hypoglycemia and malabsorption syndrome, in a human or non-human animal subject in need thereof which comprises administering to said subject an effective amount of a compound according to claim 1 or a pharmaceutically acceptable salt thereof.

In yet a further aspect the invention provides a method of imaging cells having GLP-1 receptors comprising contacting said cells with an effective amount of a compound according to claim 1, or a pharmaceutically acceptable salt thereof, wherein said compound comprises one or more radio-iodinated tyrosine residues.

Unless otherwise defined, and with the exception of the N-terminal amino acid, abbreviations (e.g. Ala) of amino acids in this disclosure generally stand for the structure -NH-C(R)(R')-CO-, wherein each of R and R' is, independently, H or the side chain of an amino acid (e.g., R = CH₃ and R' = H for Ala). For the N-terminal amino acid, the abbreviation generally stands for the structure of (R²R³)-N-CH(R)-CO-, wherein R is a side chain of an amino acid and R² and R³ are as defined above. In the case of certain amino acids at the N-terminal (e.g., Ura, Paa, Pta and the like) R², R³ and N are not present since such amino acids are considered here as des-amino amino acids.

### DETAILED DESCRIPTION OF THE INVENTION

The peptides of this invention can be prepared by standard solid phase peptide synthesis. See, e.g., Stewart, J.M., et al., Solid Phase Synthesis (Pierce Chemical Co., 2d ed. 1984). The substituents R² and R³ of the above generic formula may be attached to the free amine of the N-terminal amino acid by standard methods known in the art. For example, alkyl groups, e.g., (C₁-C₃₀)alkyl, may be attached using reductive alkylation. Hydroxyalkyl groups, e.g., (C₁-C₃₀)hydroxyalkyl, may also be attached using reductive alkylation wherein the free hydroxy group is protected with a t-butyl ester. Acyl groups, e.g., COE¹, may be attached by coupling the free acid, e.g., E¹COOH, to the free amine of the N-terminal amino acid by mixing the resin with 3 molar equivalents of both the free acid and diisopropylcarbodiimide in methylene chloride for about one hour. If the free acid contains a free hydroxy group, e.g., p-hydroxyphenylpropionic acid, then the coupling should be performed with an additional 3 molar equivalents of HOBT.

Unless defined otherwise, all technical and scientific terms used herein have the same meaning as commonly understood by one of ordinary skill in the art to which this invention belongs. Also, all publications, patent applications, patents and other references mentioned herein are incorporated by reference.

### Nomenclature and Abbreviations

| **Symbol** | **Meaning** |
|---|---|
| A3c | is 1-amino-1-cyclopropanecarboxylic acid |
| A4c | is 1-amino-1-cyclobutanecarboxylic acid |
| A5c | is 1-amino-1-cyclopentanecarboxylic acid |
| A6c | is 1-amino-1-cyclohexanecarboxylic acid |
| A7c | is 1-amino-1-cycloheptanecarboxylic acid |
| A8c | is 1-amino-1-cyclooctanecarboxylic acid |
| A9c | is 1-amino-1-cyclononanecarboxylic acid |
| Abu | is α-aminobutyric acid |
| Acc | is an amino acid selected from the group A3c, A4c, A5c, A6c, A7c, A8c and A9c |
| Acp | is 4-(2-aminoethyl)-1-carboxymethyl-piperazine |
| Ado | is 12-aminododecanoic acid |
| Aib | is α-aminoisobutyric acid |
| Aic | is 2-aminoindane-2-carboxylic acid |
| *β-*Ala | is *β-*alanine |
| Amp | is 4-amino-phenylalanine |
| Aun | is 11-aminoundecanoic acid |
| Ava | is 5-aminovaleric acid |
| Boc | is t-butyloxycarbonyl |
| 2BrZ | is 2-bromobenzyloxycarbonyl |
| Bzl | is benzyl |
| Cha | is *β*-cyclohexylalanine |
| 2ClZ | is 2-chlorobenzyloxycarbonyl |
| Dab | is *α,γ*-diaminobutyric acid |
| Dap | is *α,β*-diaminopropionic acid |
| DCM | is dichloromethane |
| DIC | is 1,3-diisopropylcarbodiimide |
| DMAP | is 4-(dimethylamino)pyridine |
| DIEA | is diisopropylethylamine |
| DMF | is N,N-dimethylformamide |
| DNP | is 2,4-dinitrophenyl |
| Fm | is formyl |
| Fmoc | is 9-fluorenylmethoxycarbonyl |
| Gaba | is *γ*-aminobutyric acid |
| HBTU | is 2-(1H-benzotriazol-1-yl)-1,1,3,3-tetramethyl uronium hexafluorophosphate |
| HF | is hydrogen fluoride |
| HOAc | is acetic acid |
| HOBt | is N-hydroxybenzotriazole |
| Hppa | is 3-(*p*-hydroxyphenyl)propionic acid |
| hPro | is homoproline |
| N-Me-Ala | is N-methyl-alanine |
| N-Me-Glu | is N-methyl-glutamic acid |
| N-Me-Gly | is N-methyl-glycine |
| β-1-Nal | is *β*(1-naphthyl)alanine |
| β-2-Nal | is *β*-(2-naphthyl)alanine |
| Nle | is norleucine |
| OcHex | is O-cyclohexyl |
| Orn | is ornithine |
| Paa | is *trans*-3-(3-pyridyl) acrylic acid |
| 3-Pal | is *β-*(3-pyridinyl)alanine |
| 4-Pal | is *β*-(4-pyridinyl)alanine |
| PAM resin | is 4-hydroxymethylphenylacetamidomethyl resin |
| Pta | is (4-pyridylthio) acetic acid |
| TFA | is trifluoroacetic acid |
| TIS | is triisopropylsilane |
| Tle | is *tert*-butylglycine |
| Tma-His | is N,N-tetramethylamidino-histidine |
| Tos | is tosyl |
| Trt | is trityl |
| Ura | is urocanic acid |
| Xan | is xanthyl |
| Z | is benzyloxycarbonyl |

In the above formula, hydroxyalkyl, hydroxyphenylalkyl and hydroxynaphthylalkyl may contain 1-4 hydroxy substituents. C(O)X³ stands for -C=O·X³, wherein X³ is as defined hereinabove. Thus examples of -C=O·X³ include, but are not limited to, acetyl and phenylpropionyl.

What is meant by Lys(N^{ε}-alkanoyl) is represented by the following structure: 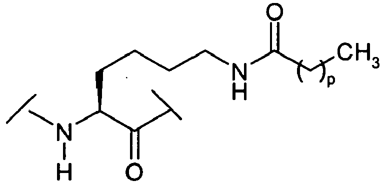 where p is 0 or an integer from 1 to 28, inclusive.

What is meant by Ser(O-alkanoyl) is represented by the following structure: 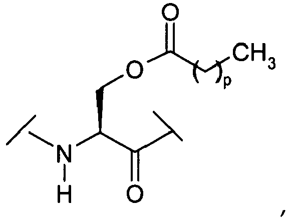 where p is 0 or an integer from 1 to 28, inclusive.

What is meant by Acp is represented by the following structure:

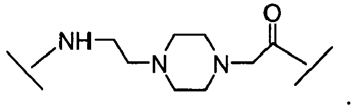

What is meant by Lys(N-Acp-alkanoyl) is represented by the following structure: 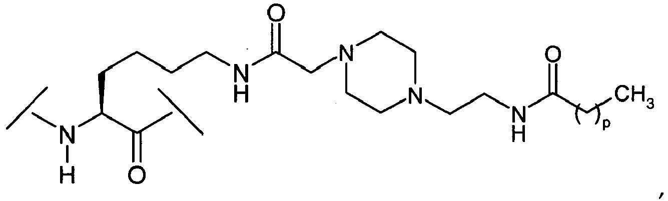 where p is 0 or an integer from 1 to 28, inclusive.

In the moiety -C(NH)NH₂ the nitrogen of the (NH) group is bonded to the carbon via a double bond.

The term "halogen" encompasses fluoro, chloro, bromo and iodo, including radioactive and non-radioactive isotopes. The term "(C₁-C₁₂)hydrocarbon moiety" encompasses alkyl, alkenyl and alkynyl moieties; alkenyl and alkynyl moieties having C₂-C₁₂.

The phrase "independently for each occurrence" means that any substituent member to which the phrase applies may appear without regard to whether another substituent member appears. Thus the definition of X¹ᵤ-Phe as provided herein encompasses, e.g., (¹²⁵I)Tyr; i.e., where u is 2, X¹ is OH in the first occurrence, and X¹ is ¹²⁵I in the second occurrence.

A peptide of this invention is also denoted herein by another format, e.g., (Aib²)Exendin-4(1-39)NH₂ (SEQ ID NO:4), with the substituted amino acids from the natural sequence placed between parentheses prior to the abbreviation for the peptide, (e.g., Aib² for Gly² in Exendin-4). The numbers between the parentheses following the abbreviation for the peptide refer to the number of amino acids present in the peptide; e.g., Exendin-4(1-39) (SEQ ID NO:93) is amino acids 1 through 39 of the peptide sequence for Exendin-4 (SEQ ID NO:2). The designation "NH₂" in, e.g., "Exendin-4(1-39)NH₂" (SEQ ID NO:93), indicates that the C-terminus of the peptide is amidated. Conversely, "Exendin-4(1-39)" (SEQ ID NO:93) means that the C-terminus is the free acid.

The sequence for Exendin-4 is listed in John Eng et al., The Journal of Biological Chemistry, vol. 267, pp. 7402-7405, (1992). The sequence for Exendin-3 is listed in Eng, J. et al., The Journal of Biological Chemistry, 265:20259-62, (1990).

### Synthesis

The protected amino acid 1-(N-tert-butoxycarbonyl-amino)-1-cyclohexanecarboxylic acid (Boc-A6c-OH) was synthesized as follows. 19.1 g (0.133 mol) of 1-amino-1-cyclohexanecarboxylic acid (Acros Organics, Fisher Scientific, Pittsburgh, PA) was dissolved in 200 ml of dioxane and 100 ml of water. To it was added 67 ml of 2N NaOH. The solution was cooled in an ice-water bath. 32.0 g (0.147 mol) of di-tert-butyl-dicarbonate was added to this solution. The reaction mixture was stirred overnight at room temperature. Dioxane was then removed under reduced pressure. 200 ml of ethyl acetate were added to the remaining aqueous solution. The mixture was cooled in an ice-water bath. The pH of the aqueous layer was adjusted to about 3 by adding 4N HCl. The organic layer was separated. The aqueous layer was extracted with ethyl acetate (1 x 100 ml). The two organic layers were combined and washed with water (2 x 150 ml), dried over anhydrous MgSO₄, filtered, and concentrated to dryness under reduced pressure. The residue was recrystallized in ethyl acetate/hexanes. 9.2 g of the pure product was obtained. 29% yield.

Boc-A5c-OH was synthesized in an analogous manner to that of Boc-A6c-OH. Other protected Acc amino acids can be prepared in an analogous manner by a person of ordinary skill in the art as enabled by the teachings herein.

In the synthesis of analogue of either Exendin-4 or -3 of this invention containing A5c, A6c and/or Aib, the coupling time is about 2 hours for these residues and the residue immediately following them. For the synthesis of (Tma-His⁷) Exendin-4 (1-39)NH₂ (SEQ ID NO:101), HBTU (2 mmol) and DIEA (1.0 ml) in 4 ml DMF are used to react with the N-terminal free amine of the peptide-resin in the last coupling reaction; the coupling time is about 2 hours.

The substituents R² and R³ of the above generic formula can be attached to the free amine of the N-terminal amino acid by standard methods known in the art. For example, alkyl groups, e.g., (C₁-C₃₀)alkyl, can be attached using reductive alkylation. Hydroxyalkyl groups, e.g., (C₁-C₃₀)hydroxyalkyl, can also be attached using reductive alkylation wherein the free hydroxy group is protected with a t-butyl ester. Acyl groups, e.g., COX¹, may be attached by coupling the free acid, e.g., X¹COOH, to the free amine of the N-terminal amino acid by mixing the resin with 3 molar equivalents of both the free acid and diisopropylcarbodiimide in methylene chloride for about one hour. If the free acid contains a free hydroxy group, e.g., *p-*hydroxyphenylpropionic acid, then the coupling should be performed with an additional 3 molar equivalents of HOBT.

### Methods

Compounds of the present invention can be and were tested for activity as a GLP-1 binding compound, and for GLP-1 agonist and/or antagonist activity, according to the following procedures.

### Receptor Expression and Transfection

### Functional Expression of the Cloned Human Somatostatin Receptors

A genomic clone containing the human GLP-1 (hGLP-1) receptor was obtained from Dr. Andreas Wilmen, University of Marburg, Germany. The coding regions were cloned into the *EcoRI* site of the mammalian expression plasmid, pTEJ8 (5). DNA transfection of a Chinese hamster ovary cell line, CHO-K1 (American Type Culture Collection, Rockville, MD), was carried out by the calcium phosphate precipitation method as previously described (6). Clonal cell lines stably expressing the rat and the human GLP-1 receptors were obtained by selection of the DNA-transfected cells in RPMI 1640 medium supplemented with 10% fetal bovine serum, 1 mM sodium pyruvate, 1% non-essential amino acids, containing 0.8 mg/ml G418 (Gibco BRL, Grand island, NY), ring cloned, and expanded in culture. For receptor and binding characterizations, the rat GLP-1 receptor expressing line CHO-N1 and the human GLP-1 receptor expressing line CHO-Y2 were used.

Transfection was performed by the calcium phosphate method. CHO-K1 cells were maintained in α-minimum essential medium (α-MEM; Gibco) supplemented with 10% fetal calf serum and transfected with the expression plasmid using calcium phosphate precipitation. Clones that had inherited the expression plasmid were selected in α-MEM supplemented with 500 µg mL⁻¹ of geneticin (G418; Gibco). Independent CHO-K1 clones were picked by glass-ring cloning and expanded in culture in the selective media. Membranes were prepared from the isolated clones and hGLP-1 expression was initially assessed for binding with (¹²⁵I)GLP-1(7-36).

CHO-K1 cells expressing the human recombinant GLP-1 receptor were cultured in Dulbecco's modified Eagle's medium (DMEM) containing 10% fetal calf serum and maintained at about 37 °C in a humidified atmosphere of 5% CO₂/95% air.

The following examples describe synthetic methods for making a peptide of this invention, which methods are well-known to those skilled in the art. Other methods are also known to those skilled in the art. The examples are provided for the purpose of illustration and are not meant to limit the scope of the present invention in any manner.

### Example 1: (Aib², Nle¹⁴, β-Ala²⁹) Exendin-4(1-39)NH₂ (SEQ ID NO:9)

The title peptide was synthesized on a model 430A peptide synthesizer (Applied Biosystems, Foster City, CA), modified to conduct accelerated Boc-chemistry solid phase peptide synthesis, was selected. See Schnolzer et al., Int. J. Peptide Protein Res., 40:180 (1992). 4-Methylbenzhydrylamine (MBHA) resin (Peninsula, Belmont, CA) with the loading of 0.91 mmol/g was used.

The Boc amino acids (Midwest Bio-tech, Fishers, IN) were used with the following side chain protection: Boc-Ala-OH, Boc-Arg(Tos)-OH, Boc-Asp(OcHex)-OH, Boc-His(DNP)-OH, Boc-Val-OH, Boc-Leu-OH, Boc-Gly-OH, Boc-Gln-OH, Boc-Ile-OH, Boc-Lys(2ClZ)-OH, Boc-Thr(Bzl)-OH, Boc-Ser(Bzl)-OH, Boc-Phe-OH, Boc-Aib-OH, Boc-Glu(OcHex)-OH, Boc-Trp(Fm)-OH, Boc-*β*-Ala-OH, Boc-Pro-OH, Boc-Nle-OH and Boc-Asn(Xan)-OH. The synthesis was carried out on a 0.20 mmol scale. The Boc groups were removed by treatment with 100% TFA for 2 x about 1 minutes. Boc amino acids (2.5 mmol) were pre-activated with HBTU (2.0 mmol) and DIEA (1.0 mL) in 4 mL of DMF and were coupled without prior neutralization of the peptide-resin TFA salt. Coupling times were about 5 minutes, except for the Boc-Aib-OH residues and the Boc-His(DNP)-OH residue, for which the coupling times were about 2 hours.

At the end of the assembly of the peptide chain, the resin was treated with a solution of 20% mercaptoethanol/10% DIEA in DMF for 2 x about 30 minutes to remove the DNP group on the His side chain. The N-terminal Boc group was then removed by treatment with 100% TFA for 2 x about 2 minutes. After neutralization of the peptide-resin with 10% DIEA in DMF (1 x about 1 min), the formyl group on the side of the chain of Trp was removed by treatment with a solution of 15% ethanolamine/ 15% water/ 70% DMF for 2 x about 30 minutes. The partially-deprotected peptide-resin was washed with DMF and DCM and dried under reduced pressure. The final cleavage was completed by stirring the peptide-resin in 10 mL of HF containing 1 mL of anisole and dithiothreitol (24 mg) at 0°C for about 75 minutes. HF was removed by a flow of nitrogen. The residue was washed with ether (6 x 10 mL) and extracted with 4N HOAc (6 x 10 mL).

The peptide mixture in the aqueous extract was purified on reverse-phase preparative high-pressure liquid chromatography (HPLC) using a reverse phase VYDAC C₁₈ column (Nest Group, Southborough, MA). The column was eluted with a linear gradient (20% to 50% of solution B over 105 minutes) at a flow rate of 10 mL/min (Solution A = water containing 0.1% TFA; Solution B = acetonitrile containing 0.1% of TFA). Fractions were collected and checked on analytical HPLC. Those containing pure product were combined and lyophilized to dryness. 31.1mg. of a white solid was collected. Purity was about 98% based on analytical HPLC analysis. Electro-spray mass spectrometer analysis gave the molecular weight at 4209.1 (in agreement with the calculated molecular weight of 4210.07).

### Example 2: (Aib², Nle¹⁴, β-Ala²⁹, Arg³⁰) Exendin-4(1-30)NH₂ (SEQ ID NO:45)

The title compound was synthesized according to the procedure described in Example 1 with substitution of appropriate Boc-amino acids. 92.9 mg of the title compound was collected from 0.2 mmol of the MBHA resin (13% yield). Purity was >99%. Electro-spray mass spectrometer analysis gave 3531.4 (in agreement with the calculated molecular weight of 3532.03).

### Example 3: (Aib², Arg^{12,27}, Nle¹⁴,β-Ala²⁹, Acp⁴⁰ ) Exendin-4(1-40)NH₂(SEQ ID NO:88)

Fmoc-Acp-OH (Neosystem Laboratoire, Princeton, NJ) is coupled to the MBHA resin (0.2mmol, substitution=0.91mmol/g) by shaking the resin with a mixture of Fmoc-Acp-OH (0.40g, 0.829 mmol), HBTU (1.57 mL of 0.5 M HBTU solution in DMF, 0.787 mmol) and DIEA (0.5 mL) for about 4 hours at room temperature on a shaker. The resin is then washed with DMF and treated with 25% piperidine in DMF for 2 x about 20 minutes. The resin is washed with DMF and DCM and dried under vacuum. This H-Acp-MBHA resin is then used for the synthesis of the title compound according to the procedure described in Example 1, with substitution of appropriate Boc-amino acids.

### Example 4: (Aib², Nle¹⁴, Lys²⁷(N^{ε}-Acp-decanoyl),β-Ala²⁹) Exendin-4(1-39)NH₂ (SEQ ID NO:92)

The assembly of Boc-Asn(Xan)-*β*-Ala-Gly-Pro-Ser(Bzl)-Ser(Bzl)-Gly-Ala-Pro-Pro-Pro-Ser(Bzl)-MBHA (0.2 mmol; (SEQ ID NO:98)) sequence is done on a modified ABI 430A peptide synthesizer according to the procedure described in Example 1. The resin is transferred to a reaction vessel on a shaker and shaken with 100% TFA for 2 x about 2 minutes. The resin is washed with DMF and shaken with a mixture of Boc-Lys(Fmoc)-OH (1.17g, 2.5 mmol, Novabiochem, San Diego, CA), 4 mL of 0.5 M HBTU solution in DMF and 1 mL of DIEA for about 5 min at room temperature. The resin is washed with DMF and treated with 25% piperidine in DMF for 2 x about 20 minutes. The resin is washed with DMF and shaken with a mixture of Fmoc-Acp-OH (289 mg, 0.6 mmol), 1.12 mL of 0.5 M HBTU solution in DMF (0.56 mmol) and 0.4 mL of DIEA for about 2 hours at room temperature. The resin is washed with DMF and treated with 25% piperidine in DMF for 2 x about 20 minutes. The resin was washed with DMF and then shaken with a mixture of decanoic acid (431 mg, 2.5 mmol), 4 mL of 0.5 M of HBTU solution in DMF and 1 mL of DIEA for about 10 min at room temperature. The resin is washed with DMF and treated with 100% TFA for 2 x about 2 minutes. The resin is washed with DMF and DCM and dried under vacuum. The dry resin is then transferred to a reaction vessel of the modified ABI 430A peptide synthesizer for further synthesis. The rest of the synthetic and purification procedures of making the title compound are the same as those described in Example 1.

### Example 5: [Aib², Nle¹⁴, Ser³²(O-decanoyl)]Exendin-4(1-39)NH₂ (SEQ ID NO:17)

The assembly of Boc-Ser(Bzl)-Gly-Ala-Pro-Pro-Pro-Ser(Bzl)-MBHA (0.2 mmol; (SEQ ID NO:99)) sequence is performed on a modified ABI 430A peptide synthesizer according to the procedure described in Example 1. The resin is transferred to a reaction vessel on a shaker and shaken with 100% TFA for 2 x about 2 minutes. The resin is washed with DMF and shaken with a mixture of Fmoc-Ser(Trt)-OH (1.42g, 2.5 mmol, Novabiochem, San Diego, CA), 4 mL of 0.5 M HBTU solution in DMF and 1 mL of DIEA for about 5 minutes at room temperature. The resin is washed with DMF and treated with 4 mL of TFA containing 0.2 mL of TIS for about 5 minutes. The resin is washed with DMF and neutralized with 2% DIEA in DMF (5mL) for 2 x about 2 minutes. The resin is washed with DMF and shaken with a mixture of decanoic acid (431 mg, 2.5mmol), DIC (316 mg, 2.5 mmol) and DMAP (5 mg) for about 16 hours at room temperature. The resin was washed with DMF and treated with 25% piperidine in DMF for about 30 minutes. The resin is washed with DMF and DCM and dried under vacuum. The dry resin is then transferred to a reaction vessel of the modified ABI 430A peptide synthesizer for further synthesis. The rest of the synthesis and purification procedures are the same as those described in Example 1.

### Example 6: [Aib², Nle¹⁴, Lys²⁷(N^{ε}-octanoyl)]Exendin-4(1-39)NH₂ (SEQ ID NO:20)

The assembly of Boc-Asn(Xan)-Gly-Gly-Pro-Ser(Bzl)-Ser(Bzl)-Gly-Ala-Pro-Pro-Pro-Ser(Bzl)-MBHA (0.2 mmol; (SEQ ID NO:100)) sequence was performed on a modified ABI 430A peptide synthesizer according to the procedure described in Example 1. The resin is transferred to a reaction vessel on a shaker and shaken with 100% TFA for 2 x about 2 minutes. The resin is washed with DMF and shaken with a mixture of Boc-Lys-(Fmoc)-OH (1.17g, 2.5 mmol), 4 mL of 0.5 M HBTU solution in DMF and 1 mL of DIEA for about 5 minutes at room temperature. The resin was washed with DMF and treated with 25% piperidine in DMF for 2 x about 20 minutes. The resin was washed with DMF and shaken with a mixture of octanoic acid (361 mg, 2.5 mmol), 4 mL of 0.5 M of HBTU solution in DMF and 1 mL of DIEA for about 10 minutes at room temperature. The resin was washed with DMF and treated with 100% TFA for 2 x about 2 minutes. The resin was washed with DMF and DCM and dried under vacuum. The dry resin was then transferred to a reaction vessel of the modified ABI 430A peptide synthesizer for further synthesis. The rest of the synthesis and purification procedures are the same as those described in Example 1.

Other peptides of the invention can be prepared by a person of ordinary skill in the art using synthetic procedures analogous to those disclosed generally hereinabove and/or to those disclosed specifically in the foregoing examples, as were the compounds depicted in Table 1.

**TABLE 1-Molecular Weight and Purity for Selected Embodiments**

| Peptide Sequence | Purity (%) | Exact Weight | Calculate |
|---|---|---|---|
| [Aib^{2,29}, Nle¹⁴, Arg³⁰]Exendin-4(1-30)-NH₂ (SEQ ID NO:46) | 93.0 | 3545.98 | 3546.00 |
| [Aib²]Exendin-4(1-39)-NH₂ (SEQ ID NO:4) | 100.0 | 3531.40 | 3531.96 |
| [Aib², Nle¹⁴]Exendin-4(1-39)-NH₂ (SEQ ID NO:5) | 98.0 | 4166.90 | 4167.62 |
| [Aib², Leu¹⁴]Exendin-4(1-39)-NH₂ (SEQ ID NO:5) | 99.0 | 4196.30 | 4196.62 |
| [Aib^{2,28}, Nle¹⁴]Exendin-4(1-39)-NH₂ (SEQ ID NO:7) | 94.0 | 4214.70 | 4214.66 |
| [Aib², Nle¹⁴, A6c²⁶]Exendin-4(1-39)-NH₂ (SEQ ID NO:10) | 97.0 | 4195.30 | 4196.62 |
| [Aib², Nle¹⁴, *β*-Ala²⁹, Arg³⁰]Exendin-4(1-30)-NH₂ (SEQ ID NO:45) | 94.0 | 4207.50 | 4208.63 |
| [Aib², Nle¹⁴, *β*-Ala²⁹]Exendin-4(1-39)-NH₂ (SEQ ID NO:9) | 98.0 | 4209.10 | 4210.65 |
| [Aib^{2,29}, Nle¹⁴]Exendin-4(1-39)-NH₂ (SEQ ID NO:8) | 99.0 | 4224.00 | 4224.68 |
| [Aib², A6c¹⁴]Exendin-4(1-39)-NH₂ (SEQ ID NO:5) | 89.0 | 4208.30 | 4208.63 |
| Exendin-4(1-39)-NH₂ (SEQ ID NO:2) | 92.0 | 4186.63 | 4186.61 |

### Example 7: in vitro studies

### Biological Assay for AgonistlAntagonist Activity:

Biological activity was determined by assaying the ability of an analog to stimulate cyclic AMP formation *in vitro* or to inhibit GLP-1-stimulated cyclic AMP formation *in vitro.* CHO-K1 cells were seeded into 24-well plates and cultured for 1-2 days. Subsequently, the culture media was removed, replaced with Hank's-buffered saline (HBSS) containing 0.5 mM isobutylmethylxanthine (IBMX), and preincubated for 30 min at 37 °C. At the end of the pre-incubation period, the analogs were added and the cells were incubated for an additional 30 minutes. When screening for antagonist activity, hGLP-1 was also added to the cells. The incubation was terminated by the addition of 0.5 ml ice-cold absolute ethanol. Aliquots were taken from the cells and analyzed for cyclic AMP content using a commercial cyclic AMP radioimmunoassay kit, following the instructions provided (catalog # NEK-033, New England Nuclear, Boston, MA).

### Radioligand Binding:

Membranes were prepared for radioligand binding studies by homogenization of the CHO-K1 cells in 20 ml of ice-cold 50 mM Tris-HCl with a Brinkman Polytron (Westbury, NY) (setting 6, 15 sec). The homogenates were washed twice by centrifugation (39,000 g / 10 min), and the final pellets were resuspended in 50 mM Tris-HCl, containing 2.5 mM MgCl₂ and 0.1% BSA. For assay, aliquots (0.4 ml) were incubated with 0.05 nM (¹²⁵I)GLP-1(7-36) (~2200 Ci/mmol, New England Nuclear, Boston, MA) with and without 0.05 ml of unlabeled competing test peptides. After a 100 min incubation (25 °C), the bound (¹²⁵I)GLP-1(7-36) was separated from the free by rapid filtration through GF/C filters (Brandel, Gaithersburg, MD) which had been previously soaked in 0.5% polyethyleneimine. The filters were washed three times with 5 ml aliquots of ice-cold 50 mM Tris-HCl and the bound radioactivity trapped on the filters was counted by gamma spectrometry (Wallac LKB, Gaithersburg, MD). Specific binding was defined as the total (¹²⁵I)GLP-1(7-36) bound minus that bound in the presence of 1000 nM GLP1(7-36) (Bachem, Torrence, CA).

The peptides of this invention may be provided in the form of pharmaceutically acceptable salts. Examples of such salts include, but are not limited to, those formed with organic acids (e.g., acetic, lactic, maleic, citric, malic, ascorbic, succinic, benzoic, methanesulfonic, toluenesulfonic, or pamoic acid), inorganic acids (e.g., hydrochloric acid, sulfuric acid, or phosphoric acid), and polymeric acids (e.g., tannic acid, carboxymethyl cellulose, polylactic, polyglycolic, or copolymers of polylactic-glycolic acids). A typical method of making a salt of a peptide of the present invention is well known in the art and can be accomplished by standard methods of salt exchange. Accordingly, the TFA salt of a peptide of the present invention (the TFA salt results from the purification of the peptide by using preparative HPLC, eluting with TFA containing buffer solutions) can be converted into another salt, such as an acetate salt by dissolving the peptide in a small amount of 0.25 N acetic acid aqueous solution. The resulting solution is applied to a semi-prep HPLC column (Zorbax, 300 SB, C-8). The column is eluted with (1) 0.1N ammonium acetate aqueous solution for 0.5 hours, (2) 0.25N acetic acid aqueous solution for 0.5 hours and (3) a linear gradient (20% to 100% of solution B over 30 minutes) at a flow rate of 4 ml/minute (solution A is 0.25N acetic acid aqueous solution; solution B is 0.25N acetic acid in acetonitrile/water, 80:20). The fractions containing the peptide are collected and lyophilized to dryness.

A selection of the preferred embodiments was tested using the above-discussed assay and the binding constants (Ki in nM) are reported in Table 2(a-d).

**TABLE 2A: Radioligand Binding Assay Data for Selected Compounds**

| Peptide Sequence | hGLP-1 Binding Ki(nM) | SEM |
|---|---|---|
| [Aib², A6c¹⁴]Exendin-4(1-39)-NH₂ (SEQ ID NO:5) | 1.200 | 0.263 |
| Exendin-4(1-39)-NH₂ (SEQ ID NO:2) | 0.455 | 0.095 |

**TABLE 2B: Radioligand Binding Assay Data for Selected Compounds**

| Peptide Sequence | hGLP-1 Binding Ki(nM) | SEM | hGLP-1 cAMP* | hGLP-1 cAMP* |
|---|---|---|---|---|
| [Aib², Nle¹⁴]Exendin-4(1-39)-NH₂ (SEQ ID NO:5) | 0.755 | 0.015 | Agonist | 62 |
| [Aib², Nle¹⁴, *β*-Ala²⁹, Arg³⁰] Exendin-4(1-30)-NH₂ (SEQ ID NO:45) | 1.143 | 0.157 | Agonist | 90 |
| [Aib^{2,28}, Nle¹⁴]Exendin-4(1-39)-NH₂ (SEQ ID NO:7) | 1.547 | 0.061 | Agonist | 77 |
| [Aib², Nle¹⁴, A6c26]Exendin-4(1-39)-NH₂ (SEQ ID NO:10) | 0.980 | 0.056 | Agonist | 103 |
| [Aib^{2,29}, Nle¹⁴]Exendin-4(1-39)-NH₂(SEQ ID NO:8) | 0.785 | 0.055 | Agonist | 116 |
| [Aib², Leu¹⁴]Exendin-4(1-39)-NH₂ (SEQ ID NO:5) | 0.835 | 0.005 | Agonist | 106 |
| [Aib², Nle¹⁴, *β*-Ala²⁹]Exendin-4(1-39)-NH₂ (SEQ ID NO:9) | 0.463 | 0.019 | Agonist | 104 |

**TABLE 2C: Radioligand Binding Assay Data for Selected Compounds**

| Peptide Sequence | rGLP-1 Ki(nM) | hGLP-1 Binding Ki(nM) | SEM | hGLP-1 cAMP* | hGLP-1 cAMP* |
|---|---|---|---|---|---|
| [Aib², Nle¹⁴, *β-*Ala²⁹] Exendin-4(1-39)-NH₂ (SEQ ID NO:9) | 0.470 | 0.463 | 0.019 | Agonist | 139 |
| Exendin-4 (SEQ ID NO:2) | 0.440 | 0.887 | 0.023 | Agonist | 100 |

**TABLE 2D: Radioligand Binding Assay Data for Selected Compounds**

| Peptide Sequence | RIN Ki(nM) | hGLP-1 Binding Ki(nM) | SEM | hGLP-1 cAMP* | hGLP-1 cAMP* |
|---|---|---|---|---|---|
| [Aib^{2,29}, Nle¹⁴, Arg³⁰] Exendin-4(1-30)-NH₂ (SEQ ID NO:46) | 0.350 | 1.840 | 0.177 | Agonist | 95 |

### Administration and Use

As is well known to those skilled in the art, the known and potential uses of GLP-1 receptor ligands is varied and multitudinous (See, Todd, J.F. et al., Clinical Science, 1998, 95:325-9; and Todd, J.F. et al., European Journal of Clinical Investigation, 1997, 27:533-6). Thus, the administration of the compounds of this invention for purposes of eliciting an agonist effect can have the same effects and uses as GLP-1 itself. These varied uses include, but are not limited to, treatment of: Type I diabetes, Type II diabetes, hyperglylcemia, obesity, glucagonomas, cachexia, secretory disorders of the airway, metabolic disorder, arthritis, osteoporosis, central nervous system diseases, restenosis and neurodegenerative diseases. The analogues of the present invention that elicit an antagonist effect from a subject can be used for treating the following: anorexia, hypoglycemia and malabsorption syndrome associated with gastroectomy or small bowel resection.

Accordingly, the present invention includes within its scope pharmaceutical compositions comprising, as an active ingredient, at least one of the compounds of formula (I) in association with a pharmaceutically acceptable carrier.

The dosage of active ingredient in the compositions of this invention may be varied; however, it is necessary that the amount of the active ingredient be such that a suitable dosage form is obtained. The selected dosage depends upon the desired therapeutic effect, on the route of administration, and on the duration of the treatment. In general, an effective dosage for the activities of this invention is in the range of 1x10⁻⁷ to 200 mg/kg/day, preferably 1x10⁻⁴ to 100 mg/kg/day, which can be administered as a single dose, divided into multiple doses, or continuously as with, e.g., an INFUSAID implantable pump.

The compounds of this invention can be administered by oral, parenteral (e.g., intramuscular, intraperitoneal, intravenous or subcutaneous injection, or implant), nasal, vaginal, rectal, sublingual or topical routes of administration and can be formulated with pharmaceutically acceptable carriers to provide dosage forms appropriate for each route of administration.

Solid dosage forms for oral administration include capsules, tablets, pills, powders and granules. In such solid dosage forms, the active compound is admixed with at least one inert pharmaceutically acceptable carrier such as sucrose, lactose, or starch. Such dosage forms can also comprise, as is normal practice, additional substances other than such inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups, the elixirs containing inert diluents commonly used in the art, such as water. Besides such inert diluents, compositions can also include adjuvants, such as wetting agents, emulsifying and suspending agents, and sweetening, flavoring and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the active substance, excipients such as coca butter or a suppository wax.

Compositions for nasal or sublingual administration are also prepared with standard excipients well known in the art.

Further, a compound of this invention can be administered in a sustained release composition such as those described in the following patents and patent applications. U.S. Patent No. 5,672,659 teaches sustained release compositions comprising a bioactive agent and a polyester. U.S. Patent No. 5,595,760 teaches sustained release compositions comprising a bioactive agent in a gelable form. U.S. Patent No. 5,821,221 teaches polymeric sustained release compositions comprising a bioactive agent and chitosan. U.S. Patent No. 5,916,883 teaches sustained release compositions comprising a bioactive agent and cyclodextrin. International Patent Publication No. WO 99/38536 teaches absorbable sustained release compositions of a bioactive agent. International Patent Publication No. WO 00/04916 teaches a process for making microparticles comprising a therapeutic agent such as a peptide in an oil-in-water process. International Patent Publication No. WO 00/09166 teaches complexes comprising a therapeutic agent such as a peptide and a phosphorylated polymer. International Patent Publication No. WO 00/25826 teaches complexes comprising a therapeutic agent such as a peptide and a polymer bearing a non-polymerizable lactone. The teachings of the foregoing patents and applications are incorporated herein by reference.

Unless defined otherwise, all technical and scientific terms used herein have the same meaning as commonly understood by one of ordinary skill in the art to which this invention belongs. Also, all publications, patent applications, patents and other references mentioned herein are incorporated by reference.

### OTHER EMBODIMENTS

From the above description, one skilled in the art can easily ascertain the essential characteristics of the present invention, and without departing from the spirit and scope thereof, can make various changes and modifications of the invention to adapt it to various uses and conditions. Thus, other embodiments are also within the claims.

The following numbered paragraphs are not claims but merely serve to further define particular aspects of the invention:
1. A compound according to formula (I):

   (R²R³)A¹-A²-A³-A⁴-A⁵-A⁶-A⁷-A⁸-A⁹-A¹⁰-A¹¹-A¹²-A-¹³-A¹⁴-A-¹⁵-A¹⁶-A¹⁷-A¹⁸-A¹⁹-A²⁰-A²¹-A²²-A²³-A²⁴-A²⁵-A²⁶-A²⁷-A²⁸-A²⁹-A³⁰-A³¹-A³²-A³³-A³⁴-A³⁵-A³⁶-A³⁷-A³⁸-A³⁹-A⁴⁰-A⁴¹-A⁴²-R¹ (I)

   or a pharmaceutically acceptable salt thereof,
   wherein:
   A¹ is His or Ala, Amp, Arg, Dab, Dap, D-His, Tma-His, Hppa, Lys, Orn, Paa, 3-Pal, 4-Pal, Pta, Tyr, Ura or deleted;
   A² is Gly or Ser or Acc, Aib, Ala, *β*-Ala, N-Me-Ala, N-Me-D-Ala, Arg, Dab, Dap, N-Me-Gly, Lys, Orn, HN-CH(CH₂OC(O)R⁴)-C(O), a D-Amino Acid or deleted;
   A³ is Asp or Glu or Acc, Aib, Ala, Arg, N-Me-Asp, Dab, Dap, N-Me-Glu, Lys, Orn, HN-CH((CH₂)_{q}-C(O)-O-R⁵)-C(O), a D-Amino acid or deleted;
   A⁴ is Gly or Acc, Aib, Ala, *β*-Ala, Arg, Dab, Dap, Lys, Orn, Ser, a D-Amino acid or deleted;
   A⁵ is Thr or Acc, Aib, Ala, Arg, Dab, Dap, Lys, Orn, Ser, HN-CH(CH(CH₃)-OC(O)R⁴)-C(O), HN-CH(CH₂OC(O)R⁴)-C(O) or deleted;
   A⁶ is Phe or Acc, Aib, Aic, Arg, Cha, Dab, Dap, Lys, *β*-1-Nal, *β*-2-Nal, Orn, 3-Pal, 4-Pal, X¹ᵤ-Phe, Trp or deleted;
   A⁷ is Thr or Acc, Aib, Ala, Arg, Dab, Dap, Lys, Orn, Ser, HN-CH(CH(CH₃)-OC(O)R⁴)-C(O), HN-CH(CH₂OC(O)R⁴)-C(O) or deleted;
   A⁸ is Ser or Acc, Aib, Ala, Arg, Dab, Dap, Lys, Orn, Thr, HN-CH(CH₂OC(O)R⁴)-C(O), HN-CH(CH(CH₃)-OC(O)R⁴)-C(O) or deleted;
   A⁹ is Asp or Aib, Ala, Arg, Dab, Dap, Glu, Lys, Orn or HN-CH((CH₂)_{q}-C(O)-O-R⁵)-C(O);
   A¹⁰ is Leu or Abu, Acc, Aib, Ala, Arg, Cha, Dab, Dap, Ile, Lys, Nle, Orn, Phe, X¹ᵤ-Phe, Tle or Val;
   A¹¹ is Ser or Acc, Aib, Ala, Arg, Dab, Dap, Lys, Orn, Thr, HN-CH(CH₂OC(O)R⁴)-C(O) or HN-CH(CH(CH₃)-OC(O)R⁴)-C(O);
   A¹² is Lys or Acc, Aib, Ala, Amp, Arg, hArg, Dab, Dap, Orn, Ser, Thr or HN-CH((CH₂)ₙ-NR⁶R⁷)-C(O);
   A¹³ is Gln or Acc, Aib, Arg, Asn, Cha, Dab, Dap, Lys, *β*-1-Nal, *β*-2-Nal, Orn, 3-Pal, 4-Pal, Phe or Tyr;
   A¹⁴ is Met or Abu, Acc, Aib, Ala, Arg, Cha, Dab, Dap, Ile, Leu, Lys, Nle, Orn, Phe, X¹ᵤ-Phe, Tle or Val;
   A¹⁵ is Glu or Acc, Aib, Ala, Arg, Asp, Dab, Dap, Lys, Orn or HN-CH((CH₂)_{q}-C(O)-O-R⁵)-C(O);
   A¹⁶ is Glu or Acc, Aib, Ala, *β*-Ala, D-Ala, Arg, Asp, Dab, Dap, Gly, Lys, Orn or HN-CH((CH₂)_{q}-C(O)-O-R⁵)-C(O);
   A¹⁷ is Glu or Acc, Aib, Ala, Arg, Asn, Asp, Dab, Dap, Gln, Lys, Orn or HN-CH((CH₂)_{q}-C(O)-O-R⁵)-C(O);
   A¹⁸ is Ala or Abu, Acc, Aib, Arg, Dab, Dap, Lys, Orn or Val;
   A¹⁹ is Val or Abu, Acc, Aib, Ala, Arg, hArg, Cha, Dab, Dap, Ile, Leu, Lys, Nle, Nva, Orn, Phe, X¹ᵤ-Phe, Tle or HN-CH((CH₂)n-NR⁶R⁷)-C(O);
   A²⁰ is Arg or Amp, hArg, Dab, Dap, Lys, Orn, 3-Pal, 4-Pal or HN-CH((CH₂)ₙ-NR⁶R⁷)-C(O);
   A²¹ is Leu or Abu, Acc, Aib, Ala, Arg, Asp, Cha, Dab, Dap, Glu, Ile, Lys, Nle, Orn, Phe, X¹ᵤ-Phe, Tle or Val;
   A²² is Phe or Acc, Aib, Aic, Ala, Arg, Cha, Dab, Dap, Lys, *β*-1-Nal, *β*-2-Nal, Orn, 3-Pal, 4-Pal, X¹ᵤ-Phe or Trp;
   A²³ is Ile or Abu, Acc, Aib, Ala, Arg, Cha, Dab, Dap, Leu, Lys, Nle, Orn, Phe, X¹ᵤ-Phe, Tle or Val;
   A²⁴ is Glu or Abu, Acc, Aib, Ala, Arg, Asp, Dab, Dap, Orn, Val or deleted,
   A²⁵ is Trp or Acc, Aib, Ala, Amp, Arg, Cha, Dab, Dap, Lys, *β*-1-Nal, *β*-2-Nal, Orn, 3-Pal, 4-Pal, Phe, X¹ᵤ-Phe, HN-CH((CH₂)_{q}-C(O)-O-R⁵)-C(O) or deleted;
   A²⁶ is Leu or Abu, Acc, Aib, Ala, Arg, Cha, Dab, Dap, Ile, Lys, Nle, Orn, Phe, X¹ᵤ-Phe, Tle, Val or deleted;
   A²⁷ is Lys or Abu, Acc, Aib, Ala, Amp, Arg, hArg, Cha, Dab, Dap, Ile, Leu, Nle, Nva, Orn, Phe, X¹ᵤ-Phe, Tle, Val, HN-CH((CH₂)ₙ-NR⁶R⁷)-C(O) or deleted;
   A²⁸ is Asn or Acc, Aib, Ala, Amp, Arg, hArg, Dab, Dap, Gln, Lys, Orn, HN-CH((CH₂)ₙ-NR⁶R⁷)-C(O), a D-amino acid or deleted;
   A²⁹ is Gly or Acc, Aib, Ala, *β*-Ala, Arg, Dab, Dap, Lys, Orn, HN-(CH₂)ₘ-C(O), a D-Amino acid or deleted;
   A³⁰ is Gly or Acc, Aib, Ala, *β*-Ala, Amp, Arg, hArg, Dap, Dab, Lys, Orn, Val, HN-(CH₂)ₘ-C(O), HN-CH((CH₂)ₙ-NR⁶R⁷)-C(O), a D-Amino acid or deleted;
   A³¹ is Pro or Acc, Aib, Ala, Arg, Dab, Dap, Lys, Orn, D-Pro, hPro, R⁹ or deleted,
   A³² is Ser or Acc, Aib, Ala, Arg, Dab, Dap, Lys, Orn, D-Ser, Thr, HN-CH(CH₂OC(O)R⁴)-C(O), HN-CH(CH(CH₃)-OC(O)R⁴)-C(O), R⁹, a D-amino acid or deleted;
   A³³ is Ser or Acc, Aib, Ala, Arg, Dab, Dap, Lys, Orn, D-Ser, Thr, HN-CH(CH₂OC(O)R⁴)-C(O), HN-CH(CH(CH₃)-OC(O)R⁴)-C(O), R⁹, a D-amino acid or deleted;
   A³⁴ is Gly or Acc, Aib, Ala, *β*-Ala, Arg, Dab, Dap, Lys, Om, HN-(CH₂)ₘ-C(O), R⁹, a D-Amino acid or deleted;
   A³⁵ is Ala or Abu, Acc, Aib, *β*-Ala, D-Ala, Arg, Dab, Dap, Lys, Orn, Val, HN-(CH₂)ₘ-C(O), R⁹, a D-amino acid or deleted;
   A³⁶ is Pro or Acc, Aib, Ala, Arg, Dab, Dap, Lys, Orn, D-Pro, hPro, R⁹, a D-amino acid or deleted;
   A³⁷ is Pro or Acc, Aib, Ala, Arg, Dab, Dap, Lys, Orn, D-Pro, hPro, R⁹, a D-amino acid or deleted;
   A³⁸ is Pro or Acc, Aib, Ala, Arg, Dab, Dap, Lys, Orn, D-Pro, hPro, R⁹, a D-amino acid or deleted;
   A³⁹ is Ser or Acc, Aib, Ala, Arg, Dab, Dap, Lys, Orn, Thr, HN-CH(CH₂OC(O)R⁴)-C(O), HN-CH(CH(CH₃)-OC(O)R⁴)-C(O), R⁹, a D-Amino Acid or deleted;
   A⁴⁰ is Asp, D-Asp, Glu, D-Glu, HN-(CH₂)ₘ-C(O), HN-CH((CH₂)_{q}-C(O)-O-R⁵)-C(O), R⁹ or deleted;
   A⁴¹ is HN-(CH₂)ₘ-C(O), R⁹ or deleted;
   A⁴² is HN-(CH₂)ₘ-C(O), R⁹ or deleted;
   R¹ is OH, NH₂, or (C₁-C₁₂)alkoxy;
   R² and R³ each is independently selected from the group consisting of H, (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl, hydroxynaphthyl(C₁-C₃₀)alkyl and C(O)X³;
   R⁴ and R⁵ each is independently (C₁-C₃₀)alkyl;
   R⁶ and R⁷ each is, independently for each occurrence, H, (C₁-C₃₀)alkyl, (C₁-C₃₀)acyl, (C₁-C₃₀)alkylsulfonyl, or -C(NH)NH₂;
   R⁹ is 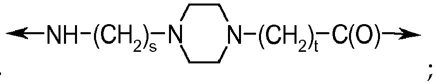
   X¹ is, independently for each occurrence, (C₁-C₆)alkyl, OH or halogen;
   X³ is (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl, or hydroxynaphthyl(C₁-C₃₀)alkyl;
   m is 1 to 20;
   n is 1 to 5;
   q is 1 or 2;
   s is 1 to 5; and
   t is 1 to 5;
   provided that when R⁶ is (C₁-C₃₀)acyl, (C₁-C₃₀)alkylsulfonyl, or -C(NH)NH₂, then R⁷ is H or (C₁-C₃₀)alkyl; and further provided that said compound is not a compound selected from the group consisting of Exendin 4(v-w)-OH, Exendin 4(v-w)-NH₂, Exendin 3(v-w)-OH, and Exendin 3(v-w)-NH₂, wherein v is 1 - 8 and w is 24-39;
   or a pharmaceutically-acceptable salt thereof.
2. A compound of paragraph 1, or a pharmaceutically acceptable salt thereof, wherein:
   A¹ is His, Hppa, Paa, Pta, Ura or deleted;
   A² is Gly, Aib, D-Ala or deleted;
   A¹⁰ is Leu or A6c;
   A¹¹ is Ser or Aib;
   A¹² is Lys, Arg, Lys(N^{ε}-octanoyl) or Lys(N^{ε}-Acp-octanoyl);
   A¹⁴ is Met, Nle, Leu or A6c;
   A¹⁷ is Glu or Ser(O-decanoyl);
   A¹⁸ is Ala or Aib;
   A²⁵ is Trp, Phe or deleted;
   A²⁶ is Leu, A6c or deleted;
   A²⁷ is Lys, Arg, Lys(N^{ε}-octanoyl), Lys(N^{ε}-Acp-octanoyl), Lys(N^{ε}-Acp-decanoyl) or deleted;
   A²⁸ is Asn, Aib or deleted;
   A²⁹ is Gly, Aib, *β*-Ala or deleted;
   A³⁰ is Gly, Arg, D-Arg or deleted;
   A³¹ is Acp, Asp, D-Asp, Ava, Pro, Ser(O-decanoyl) or deleted;
   A³² is Ser, Ser(O-decanoyl), Aun, Ava or deleted;
   A³³ is Ser, Ser(O-decanoyl), Aun or deleted;
   A³⁹ is Ser, Ser(O-decanoyl) or deleted;
   A⁴⁰ is Acp, Asp, D-Asp, Ava or deleted;
   A⁴¹ is Aun, Ava or deleted; and
   A⁴² is Aun or deleted;
   or a pharmaceutically-acceptable salt thereof.
3. A compound according to paragraph 2, wherein:
   A² is Aib;
   A¹² is Arg or Lys;
   A¹⁸ is Ala, or Aib;
   A²⁶ is A6c or Leu;
   A²⁷ is Arg, Lys or Lys²⁷(N^{ε}-Acp-decanoyl);
   A²⁸ is Aib or Asn;
   A²⁹ is Aib, *β*-Ala or Gly;
   A³⁰ is Arg or Gly; and
   A⁴⁰ is Acp or deleted;
   or a pharmaceutically-acceptable salt thereof.
4. A compound according to paragraph 2, wherein said compound is:
   (Aib²) Exendin-4(1-39)NH₂; (SEQ ID NO:4)
   (Aib², Nle¹⁴) Exendin-4(1-39)NH₂; (SEQ ID NO:5)
   (Aib², Leu¹⁴) Exendin-4(1-39)NH₂; (SEQ ID NO:5)
   (Aib², A6c¹⁴) Exendin-4(1-39)NH₂; (SEQ ID NO:5)
   (Aib², Arg^{12,27}, Nle¹⁴) Exendin-4(1-39)NH₂; (SEQ ID NO:6)
   (Aib²,Arg^{12,27}, Leu¹⁴) Exendin-4(1-39)NH₂; (SEQ ID NO:6)
   (Aib^{2,28}, Nle¹⁴) Exendin-4(1-39)NH₂; (SEQ ID NO:7)
   (Aib^{2,28}, Leu¹⁴) Exendin-4(1-39)NH₂; (SEQ ID NO:7)
   (Aib^{2,29}, Nle¹⁴) Exendin-4(1-39)NH₂; (SEQ ID NO:8)
   (Aib^{2,29}, Leu¹⁴) Exendin-4(1-39)NH₂; (SEQ ID NO:8)
   (Aib², Nle¹⁴ *β*-Ala²⁹) Exendin-4(1-39)NH₂; (SEQ ID NO:9)
   (Aib², Leu¹⁴, *β*-Ala²⁹) Exendin-4(1-39)NH₂; (SEQ ID NO:9)
   (Aib², *β*-Ala²⁹) Exendin-4(1-39)NH₂; (SEQ ID NO:9)
   (Aib^{2,29}) Exendin-4(1-39)NH₂; (SEQ ID NO:8)
   (Aib², Nle¹⁴ A6c²⁶) Exendin-4(1-39)NH₂; (SEQ ID NO:10)
   (Aib², Leu¹⁴, A6c²⁶) Exendin-4(1-39)NH₂; (SEQ ID NO:10)
   (Aib², A6c²⁶) Exendin-4(1-39)NH₂; (SEQ ID NO:10)
   (Aib², A6c¹⁴) Exendin-4(1-39)NH₂; (SEQ ID NO:5)
   (Aib², A6c¹⁰, Leu¹⁴) Exendin-4(1-39)NH₂; (SEQ ID NO:11)
   (Aib², A6c¹⁰, Nle¹⁴) Exendin-4(1-39)NH₂; (SEQ ID NO:11)
   (Aib², A6c^{10,14}) Exendin-4(1-39)NH₂; (SEQ ID NO:11)
   (Aib^{2,18}, Nle¹⁴) Exendin-4(1-39)NH₂; (SEQ ID NO:12)
   (Aib^{2,18}, Leu¹⁴) Exendin-4(1-39)NH₂; (SEQ ID NO:12)
   (Aib^{2,18}) Exendin-4(1-39)NH₂; (SEQ ID NO:12)
   (Aib^{2,11}, Nle¹⁴) Exendin-4(1-39)NH₂; (SEQ ID NO:13)
   (Aib^{2,11}, Leu¹⁴) Exendin-4(1-39)NH₂; (SEQ ID NO:13)
   (Aib^{2,11}) Exendin-4(1-39)NH₂; (SEQ ID NO:13)
   (D-Ala², Nle¹⁴) Exendin-4(1-39)NH₂; (SEQ ID NO:14)
   (D-Ala², Leu¹⁴) Exendin-4(1-39)NH₂; (SEQ ID NO:14)
   (D-Ala²) Exendin-4(1-39)NH₂; (SEQ ID NO:14)
   (Aib², A6c^{14,26}) Exendin-4(1-39)NH₂; (SEQ ID NO:10)
   (Aib^{2,18}, Nle¹⁴) Exendin-4(1-39)NH₂; (SEQ ID NO:12)
   (Hppa¹, Nle¹⁴) Exendin-4(1-39)NH₂; (SEQ ID NO:15)
   (Hppa¹, Leu¹⁴) Exendin-4(1-39)NH₂; (SEQ ID NO:15)
   (Hppa¹) Exendin-4(1-39)NH₂; (SEQ ID NO:15)
   (Ura¹, Nle¹⁴) Exendin-4(1-39)NH₂; (SEQ ID NO:16
   (Paa¹, Nle¹⁴) Exendin-4(1-39)NH₂; (SEQ ID NO:16)
   (Pta¹, Nle¹⁴) Exendin-4(1-39)NH₂; (SEQ ID NO:16)
   (Aib², Nle¹⁴, Ser³²(O-decanoyl)) Exendin-4(1-39)NH₂; (SEQ ID NO:17)
   (Aib², Leu¹⁴, Ser³²(O-decanoyl)) Exendin-4(1-39)NH₂; (SEQ ID NO:17)
   (Aib², Nle¹⁴, Ser³³(O-decanoyl)) Exendin-4(1-39)NH₂; (SEQ ID NO:18)
   (Aib², Leu¹⁴, Ser³³(O-decanoyl)) Exendin-4(1-39)NH₂; (SEQ ID NO:18)
   (Aib², Nle¹⁴, Ser³⁹(O-decanoyl)) Exendin-4(1-39)NH₂; (SEQ ID NO:19)
   (Aib², Leu¹⁴, Ser³⁹(O-decanoyl)) Exendin-4(1-39)NH₂; (SEQ ID NO:19)
   (Aib², Nle¹⁴ Lys²⁷(N^{ε}-octanoyl)) Exendin-4(1-39)NH₂; (SEQ ID NO:20)
   (Aib², Leu¹⁴, Lys²⁷(N^{ε}-octanoyl)) Exendin-4(1-39)NH₂; (SEQ ID NO:20)
   (Aib², Arg¹², Nle¹⁴ Lys²⁷(N^{ε}-octanoyl)) Exendin-4(1-39)NH₂; (SEQ ID NO:21)
   (Aib², Arg¹², Leu¹⁴, Lys²⁷(N^{ε}-octanoyl)) Exendin-4(1-39)NH₂; (SEQ ID NO:21)
   (Aib², Lys¹²(N^{ε}-octanoyl), Nle¹⁴, Arg²⁷) Exendin-4(1-39)NH₂; (SEQ ID NO:22)
   (Aib², Lys¹²(N^{ε}-octanoyl), Leu¹⁴, Arg²⁷) Exendin-4(1-39)NH₂; (SEQ ID NO:22)
   (Aib², Arg^{12,27}, Nle¹⁴, Ava⁴⁰, Aun⁴¹) Exendin-4(1-41)NH₂; (SEQ ID NO:94)
   (Aib², Arg^{12,27}, Nle¹⁴, Asp⁴⁰, Ava⁴¹, Aun⁴²) Exendin-4(1-42)NH₂; (SEQ ID NO:23)
   (Aib², Arg^{12,27}, Nle¹⁴, D-Asp⁴⁰, Ava⁴¹, Aun⁴²) Exendin-4(1-42)NH₂; (SEQ ID NO:23)
   (Aib²) Exendin-3(1-39)NH₂; (SEQ ID NO:24)
   (Aib², Nle¹⁴) Exendin-3(1-39)NH₂; (SEQ ID NO:25)
   (Aib², Leu¹⁴) Exendin-3(1-39)NH₂; (SEQ ID NO:25)
   (Aib², A6c¹⁴) Exendin-3(1-39)NH₂; (SEQ ID NO:25)
   (Aib², Arg^{12,27}, Nle¹⁴) Exendin-3(1-39)NH₂; (SEQ ID NO:26)
   (Aib², Arg^{12,27}, Leu¹⁴) Exendin-3(1-39)NH₂; (SEQ ID NO:26)
   (Aib^{2,28}, Nle¹⁴) Exendin-3(1-39)NH₂; (SEQ ID NO:27)
   (Aib^{2,28}, Leu¹⁴) Exendin-3(1-39)NH₂; (SEQ ID NO:27)
   (Aib^{2,29}, Nle¹⁴) Exendin-3(1-39)NH₂; (SEQ ID NO:28)
   (Aib^{2,29}, Leu¹⁴) Exendin-3(1-39)NH₂; (SEQ ID NO:28)
   (Aib², Nle¹⁴, *β*-Ala²⁹) Exendin-3(1-39)NH₂; (SEQ ID NO:29)
   (Aib², Leu¹⁴, *β*-Ala²⁹) Exendin-3(1-39)NH₂; (SEQ ID NO:29)
   (Aib², *β*-Ala²⁹) Exendin-3(1-39)NH₂; (SEQ ID NO:29)
   (Aib^{2,29}) Exendin-3(1-39)NH₂; (SEQ ID NO:28)
   (Aib², Nle¹⁴ A6c²⁶) Exendin-3(1-39)NH₂; (SEQ ID NO:30)
   (Aib², Leu¹⁴, A6c²⁶) Exendin-3(1-39)NH₂; (SEQ ID NO:30)
   (Aib², A6c²⁶) Exendin-3(1-39)NH₂; (SEQ ID NO:30)
   (Aib², A6c¹⁴) Exendin-3(1-39)NH₂; (SEQ ID NO:25)
   (Aib², A6c¹⁰, Leu¹⁴) Exendin-3(1-39)NH₂; (SEQ ID NO:31)
   (Aib², A6c¹⁰, Nle¹⁴) Exendin-3(1-39)NH₂; (SEQ ID NO:31)
   (Aib², A6c^{10,14}) Exendin-3(1-39)NH₂; (SEQ ID NO:31)
   (Aib^{2,18}, Me¹⁴) Exendin-3(1-39)NH₂; (SEQ ID NO:32)
   (Aib^{2,18}, Leu¹⁴) Exendin-3(1-39)NH₂; (SEQ ID NO:32)
   (Aib^{2,11}, Nle¹⁴) Exendin-3(1-39)NH₂; (SEQ ID NO:33)
   (Aib^{2,11}, Leu¹⁴) Exendin-3(1-39)NH₂; (SEQ ID NO:33)
   (Aib^{2,11}) Exendin-3(1-39)NH₂; (SEQ ID NO:33)
   (D-Ala², Nle¹⁴) Exendin-3(1-39)NH₂; (SEQ ID NO:34)
   (D-Ala², Leu¹⁴) Exendin-3(1-39)NH₂; (SEQ ID NO:34)
   (D-Ala²) Exendin-3(1-39)NH₂; (SEQ ID NO:34)
   (Aib², A6c^{14,26}) Exendin-3(1-39)NH₂; (SEQ ID NO:30)
   (Aib^{2,18}) Exendin-3(1-39)NH₂; (SEQ ID NO:32)
   (Hppa¹, Nle¹⁴) Exendin-3(1-39)NH₂; (SEQ ID NO:35)
   (Hppa¹, Leu¹⁴) Exendin-3(1-39)NH₂; (SEQ ID NO:35)
   (Hppa¹) Exendin-3(1-39)NH₂; (SEQ ID NO:35)
   (Ura¹, Nle¹⁴) Exendin-3(1-39)NH₂; (SEQ ID NO:36)
   (Paa¹, Nle¹⁴) Exendin-3(1-39)NH₂; (SEQ ID NO:36)
   (Pta¹, Nle¹⁴) Exendin-3(1-39)NH₂; (SEQ ID NO:36)
   (Aib², Nle¹⁴, Ser³²(O-decanoyl)) Exendin-3(1-39)NH₂; (SEQ ID NO:37)
   (Aib², Leu¹⁴, Ser³²(O-decanoyl)) Exendin-3(1-39)NH₂; (SEQ ID NO:37)
   (Aib², Nle¹⁴, Ser³³(O-decanoyl)) Exendin-3(1-39)NH₂; (SEQ ID NO:38)
   (Aib², Leu¹⁴, Ser³³(O-decanoyl)) Exendin-3(1-39)NH₂; (SEQ ID NO:38)
   (Aib², Nle¹⁴, Ser³⁹(O-decanoyl)) Exendin-3(1-39)NH₂; (SEQ ID NO:39)
   (Aib², Leu¹⁴, Ser³⁹(O-decanoyl)) Exendin-3(1-39)NH₂; (SEQ ID NO:39)
   (Aib², Nle¹⁴, Ser¹⁷(O-decanoyl)) Exendin-3(1-39)NH₂; (SEQ ID NO:40)
   (Aib², Leu¹⁴, Ser¹⁷(O-decanoyl)) Exendin-3(1-39)NH₂; (SEQ ID NO:40)
   (Aib², Nle¹⁴ Lys²⁷(N^{ε}-octanoyl)) Exendin-3(1-39)NH₂; (SEQ ID NO:41)
   (Aib², Leu¹⁴, Lys²⁷(N^{ε}-octanoyl)) Exendin-3(1-39)NH₂; (SEQ ID NO:41)
   (Aib², Arg¹², Nle¹⁴ Lys²⁷(N^{ε}-octanoyl)) Exendin-3(1-39)NH₂; (SEQ ID NO:42)
   (Aib², Arg¹², Leu¹⁴, Lys²⁷(N^{ε}-octanoyl)) Exendin-3(1-39)NH₂; (SEQ ID NO:42)
   (Aib², Lys¹² (N^{ε}-octanoyl), Nle¹⁴, Arg²⁷) Exendin-3(1-39)NH₂; (SEQ ID NO:43)
   (Aib², Lys¹² (N^{ε}-octanoyl), Leu¹⁴, Arg²⁷) Exendin-3(1-39)NH₂; (SEQ ID NO:43)
   (Aib², Arg^{12,27}, Nle¹⁴, Ava⁴⁰, Aun⁴¹) Exendin-3(1-41)NH₂; (SEQ ID NO:95)
   (Aib², Arg^{12,27}, Nle¹⁴, Asp⁴⁰, Ava⁴¹, Aun⁴²) Exendin-3(1-42)NHz; (SEQ ID NO:44)
   (Aib², Arg^{12,27}, Nle¹⁴, D-Asp⁴⁰, Ava⁴¹, Aun⁴²) Exendin-3(1-42)NH₂; (SEQ ID NO:44)
   (Aib², Nle¹⁴, *β*-Ala²⁹, Arg³⁰) Exendin-4(1-30)NH₂; (SEQ ID NO:45)
   (Aib², Leu¹⁴, *β*-Ala²⁹, Arg³⁰) Exendin-4(1-30)NH₂; (SEQ ID NO:45)
   (Aib², A6c¹⁴, *β*-Ala²⁹, Arg³⁰) Exendin-4(1-30)NH₂; (SEQ ID NO:45)
   (Aib^{2,29}, Nle¹⁴, Arg³⁰) Exendin-4(1-30)NH₂; (SEQ ID NO:46)
   (Aib^{2,29}, Leu¹⁴, Arg³⁰) Exendin-4(1-30)NH₂; (SEQ ID NO:46)
   (Aib^{2,29}, A6c¹⁴, Arg³⁰) Exendin-4(1-30)NH₂; (SEQ ID NO:46)
   (Aib², Nle¹⁴, *β*-Ala²⁹, D-Arg³⁰) Exendin-4(1-30)NH₂; (SEQ ID NO:47)
   (Aib², Leu¹⁴, *β*-Ala²⁹, D-Arg³⁰) Exendin-4(1-30)NH₂; (SEQ ID NO:47)
   (Aib^{2,29}, Nle¹⁴, D-Arg³⁰) Exendin-4(1-30)NH₂; (SEQ ID NO:48)
   (Aib^{2,29}, Leu¹⁴, D-Arg³⁰) Exendin-4(1-30)NH₂; (SEQ ID NO:48)
   (Aib², Art^{12,27,30}, Nle¹⁴, *β*-Ala²⁹) Exendin-4(1-30)NH₂; (SEQ ID NO:49)
   (Aib², Arg^{12,27,30}, Leu¹⁴, *β*-Ala²⁹) Exendin-4(1-30)NH₂; (SEQ ID NO:49)
   (Aib^{2,29}, Arg^{12,27,30}_{,} Nle¹⁴) Exendin-4(1-30)NH₂; (SEQ ID NO:50)
   (Aib^{2,29}, Arg^{12,27,30}, Leu¹⁴) Exendin-4(1-30)NH₂; (SEQ ID NO:50)
   (Nle¹⁴, *β*-Ala²⁹, Arg³⁰) Exendin-4(1-30)NH₂; (SEQ ID NO:51)
   (Leu¹⁴, *β*-Ala²⁹, Arg³⁰) Exendin-4(1-30)NH₂; (SEQ ID NO:51)
   (Nle¹⁴, Aib²⁹, Arg³⁰) Exendin-4(1-30)NH₂; (SEQ ID NO:52)
   (Leu¹⁴, Aib²⁹, Arg³⁰) Exendin-4(1-30)NH₂; (SEQ ID NO:52)
   (Aib², *β*-Ala²⁹, Arg³⁰) Exendin-4(1-30)NH₂; (SEQ ID NO:45)
   (Aib^{2,29}, Arg³⁰) Exendin-4(1-30)NH₂; (SEQ ID NO:46)
   (*β*-Ala²⁹, Arg³⁰) Exendin-4(1-30)NH₂; (SEQ ID NO:51)
   (Aib²⁹, Arg³⁰) Exendin-4(1-30)NH₂; (SEQ ID NO:52)
   (Aib², Nle¹⁴, *β*-Ala²⁹) Exendin-4(1-30)NH₂; (SEQ ID NO:53)
   (Aib², Leu¹⁴, *β*-Ala²⁹) Exendin-4(1-30)NH₂; (SEQ ID NO:53)
   (Aib^{2,29}, Nle¹⁴) Exendin-4(1-30)NH₂; (SEQ ID NO:54)
   (Aib^{2,29}, Leu¹⁴) Exendin-4(1-30)NH₂; (SEQ ID NO:54)
   (Nle¹⁴, *β*-Ala²⁹) Exendin-4(1-30)NH₂; (SEQ ID NO:55)
   (Leu¹⁴, *β*-Ala²⁹) Exendin-4(1-30)NH₂; (SEQ ID NO:55)
   (Nle¹⁴, Aib²⁹) Exendin-4(1-30)NH₂; (SEQ ID NO:56)
   (Leu¹⁴, Aib²⁹) Exendin-4(1-30)NH₂; (SEQ ID NO:56)
   (Aib^{2,29}) Exendin-4(1-30)NH₂; (SEQ ID NO:54)
   (*β*-Ala²⁹) Exendin-4(1-30)NH₂; (SEQ ID NO:55)
   (Aib²⁹) Exendin-4(1-30)NH₂; (SEQ ID NO:56)
   (Aib², Nle¹⁴, Ser³¹(O-decanoyl)) Exendin-4(1-31)NH₂; (SEQ ID NO:57)
   (Aib², Leu¹⁴, Ser³¹(O-decanoyl)) Exendin-4(1-31)NH₂; (SEQ ID NO:57)
   (Aib², Nle¹⁴, Ser¹⁷(O-decanoyl)) Exendin-4(1-30)NH₂; (SEQ ID NO:58)
   (Aib², Leu¹⁴, Ser¹⁷(O-decanoyl)) Exendin-4(1-30)NH₂; (SEQ ID NO:58)
   (Aib², Nle¹⁴, Lys²⁷(N^{ε}-octanoyl)) Exendin-4(1-30)NH₂; (SEQ ID NO:59)
   (Aib², Leu¹⁴, Lys²⁷(N^{ε}-octanoyl)) Exendin-4(1-30)NH₂; (SEQ ID NO:59)
   (Aib², Arg¹², Nle¹⁴ Lys²⁷(N^{ε}-octanoyl)) Exendin-4(1-30)NH₂; (SEQ ID NO:60)
   (Aib², Arg¹², Leu¹⁴, Lys²⁷(N^{ε}-octanoyl)) Exendin-4(1-30)NH₂; (SEQ ID NO:60)
   (Aib², Lys¹²(N^{ε}-octanoyl), Me¹⁴, Arg²⁷) Exendin-4(1-30)NH₂; (SEQ ID NO:61)
   (Aib², Lys¹² (N^{ε}-octanoyl), Leu¹⁴, Arg²⁷) Exendin-4(1-30)NH₂; (SEQ ID NO:61)
   (Aib², Arg^{12, 27, 30}, Nle¹⁴, Ava³¹, Aun³²) Exendin-4(1-32)NH2; (SEQ ID NO:96)
   (Aib², Arg^{12, 27, 30}, Nle¹⁴, Asp³¹, Ava³², Aun³³) Exendin-4(1-32)NH₂; (SEQ ID NO:62)
   (Aib², Arg¹², ^{27, 30}, Nle¹⁴, D-Asp³¹, Ava³², Aun³³) Exendin-4(1-32)NH₂; (SEQ ID NO:62)
   (Aib², Nle¹⁴, *β*-Ala²⁹, Arg³⁰) Exendin-3(1-30)NH₂; (SEQ ID NO:63)
   (Aib², Leu¹⁴, *β*-Ala²⁹, Arg³⁰) Exendin-3(1-30)NH₂; (SEQ ID NO:63)
   (Aib², A6c¹⁴, *β*-Ala²⁹, Arg³⁰) Exendin-3(1-30)NH₂; (SEQ ID NO:63)
   (Aib^{2,29}, Nle¹⁴, Arg³⁰) Exendin-3(1-30)NH₂; (SEQ ID NO:64)
   (Aib^{2,29}, Leu¹⁴, Arg³⁰) Exendin-3(1-30)NH₂; (SEQ ID NO:64)
   (Aib^{2,29}, A6c¹⁴, Arg³⁰) Exendin-3(1-30)NH₂; (SEQ ID NO:64)
   (Aib², Nle¹⁴, *β*-Ala²⁹, D-Arg³⁰) Exendin-3(1-30)NH₂; (SEQ ID NO:65)
   (Aib², Leu¹⁴, *β*-Ala²⁹, D-Arg³⁰) Exendin-3(1-30)NH₂; (SEQ ID NO:65)
   (Aib^{2,29}, Nle¹⁴, D-Arg³⁰) Exendin-3(1-30)NH₂; (SEQ ID NO:66)
   (Aib^{2,29}, Leu¹⁴, D-Arg³⁰) Exendin-3(1-30)NH₂; (SEQ ID NO:66)
   (Aib², Arg^{12,27,30} , Nle¹⁴, *β*-Ala²⁹) Exendin-3(1-30)NH₂; (SEQ ID NO:67)
   (Aib², Arg^{12,27,30}, Leu¹⁴, *β*-Ala²⁹) Exendin-3(1-30)NH₂; (SEQ ID NO:67)
   (Aib^{2,29}, Arg^{12,27,30}, Nle¹⁴) Exendin-3(1-30)NH₂; (SEQ ID NO:68)
   (Aib^{2,29}, Arg^{12,27,30}, Leu¹⁴) Exendin-3(1-30)NH₂; (SEQ ID NO:68)
   (Nle¹⁴, *β*-Ala²⁹, Arg³⁰) Exendin-3(1-30)NH₂; (SEQ ID NO:69)
   (Leu¹⁴, *β*-Ala²⁹, Arg³⁰) Exendin-3(1-30)NH₂; (SEQ ID NO:69)
   (Nle¹⁴, Aib²⁹, Arg³⁰) Exendin-3(1-30)NH₂; (SEQ ID NO:70)
   (Leu¹⁴ Aib²⁹, Arg³⁰) Exendin-3(1-30)NH₂; (SEQ ID NO:70)
   (Aib², *β*-Ala²⁹, Arg³⁰) Exendin-3(1-30)NH₂; (SEQ ID NO:63)
   (Aib^{2,29}, Arg³⁰) Exendin-3(1-30)NH₂; (SEQ ID NO:64)
   (*β*-Ala²⁹, Arg³⁰) Exendin-3(1-30)NH₂; (SEQ ID NO:69)
   (Aib²⁹, Arg³⁰) Exendin-3(1-30)NH₂; (SEQ ID NO:70)
   (Aib², Nle¹⁴, *β*-Ala²⁹) Exendin-3(1-30)NH₂; (SEQ ID NO:71)
   (Aib², Leu¹⁴, *β*-Ala²⁹) Exendin-3(1-30)NH₂;(SEQ ID NO:71)
   (Aib^{2,29}, Nle¹⁴) Exendin-3(1-30)NH₂; (SEQ ID NO:72)
   (Aib^{2,29}, Leu¹⁴) Exendin-3(1-30)NH₂; (SEQ ID NO:72)
   (Nle¹⁴, *β*-Ala²⁹) Exendin-3(1-30)NH₂; (SEQ ID NO:73)
   (Leu¹⁴, *β*-Ala²⁹) Exendin-3(1-30)NH₂; (SEQ ID NO:73)
   (Nle¹⁴, Aib²⁹) Exendin-3(1-30)NH₂; (SEQ ID NO:74)
   (Leu¹⁴, Aib²⁹) Exendin-3(1-30)NH₂; (SEQ ID NO:74)
   (Aib^{2,29}) Exendin-3(1-30)NH₂; (SEQ ID NO:72)
   (*β*-Ala²⁹) Exendin-3(1-30)NH₂; (SEQ ID NO:73)
   (Aib²⁹) Exendin-3(1-30)NH₂; (SEQ ID NO:74)
   (Aib², Nle¹⁴, Ser³¹(O-decanoyl)) Exendin-3(1-31)NH₂; (SEQ ID NO:75)
   (Aib², Leu¹⁴, Ser³¹(O-decanoyl)) Exendin-3(1-31)NH₂; (SEQ ID NO:75)
   (Aib², Nle¹⁴, Ser¹⁷(O-decanoyl)) Exendin-3(1-30)NH₂; (SEQ ID NO:76)
   (Aib², Leu¹⁴, Ser¹⁷(O-decanoyl)) Exendin-3(1-30)NH₂; (SEQ ID NO:76)
   (Aib², Nle¹⁴, Lys²⁷(N^{ε}-octanoyl)) Exendin-3(1-30)NH₂; (SEQ ID NO:77)
   (Aib², Leu¹⁴, Lys²⁷(N^{ε}-octanoyl)) Exendin-3(1-30)NH₂; (SEQ ID NO:77)
   (Aib², Arg¹², Nle¹⁴ Lys²⁷(N^{ε}-octanoyl)) Exendin-3(1-30)NH₂; (SEQ ID NO:78)
   (Aib², Arg¹², Leu¹⁴, Lys²⁷(N^{ε}-octanoyl)) Exendin-3(1-30)NH₂; (SEQ ID NO:78)
   (Aib², Lys¹² (N^{ε}-octanoyl), Nle¹⁴, Arg²⁷) Exendin-3(1-30)NH₂; (SEQ ID NO:79)
   (Aib², Lys¹² (N^{ε}-octanoyl), Leu¹⁴, Arg²⁷) Exendin-3(1-30)NH₂; (SEQ ID NO:79)
   (Aib², Arg^{12,27,30}, Nle¹⁴, Ava³¹, Aun³²) Exendin-3(1-32)NH₂;(SEQ ID NO:97)
   (Aib², Arg^{12,27,30}, Nle¹⁴, Asp³¹, Ava³², Aun³³) Exendin-3(1-33)NH₂; (SEQ ID NO:80)
   (Aib², Arg^{12,27,30}, Nle¹⁴, D-Asp³¹, Ava³², Aun³³) Exendin-3(1-33)NH₂; (SEQ ID NO:80)
   (Aib², Arg^{12,27}, Nle¹⁴, *β*-Ala²⁹) Exendin-4(1-39)NH₂; (SEQ ID NO:81)
   (Aib², Arg^{12,27}, Leu¹⁴, *β*-Ala²⁹) Exendin-4(1-39)NH₂; (SEQ ID NO:81)
   (Aib², Arg^{12,27}, A6c¹⁴, *β*-Ala²⁹) Exendin-4(1-39)NH₂; (SEQ ID NO:81)
   (Aib², Arg^{12,27}, *β*-Ala²⁹) Exendin-4(1-39)NH₂; (SEQ ID NO:81)
   (Nle¹⁴, *β*-Ala²⁹) Exendin-4(1-39)NH₂; (SEQ ID NO:82)
   (Leu¹⁴ Aib²⁹) Exendin-4(1-39)NH₂; (SEQ ID NO:83)
   (*β*-Ala²⁹) Exendin-4(1-39)NH₂; (SEQ ID NO:82)
   (Aib²⁹) Exendin-4(1-39)NH₂; (SEQ ID NO:83)
   (Nle¹⁴, Aib²⁹) Exendin-4(1-39)NH₂; (SEQ ID NO:83)
   (Leu¹⁴ Aib²⁹) Exendin-4(1-39)NH₂; (SEQ ID NO:83)
   (Aib², Arg^{12,27}, Nle¹⁴, Phe²⁵, *β*-Ala²⁹) Exendin-4(1-39)NH₂; (SEQ ID NO:84)
   (Aib², Arg^{12,27}, Leu¹⁴, Phe²⁵, *β*-Ala²⁹) Exendin-4(1-39)NH₂; (SEQ ID NO:84)
   (Aib², Arg^{12,27}, A6c¹⁴, Phe²⁵, *β*-Ala²⁹) Exendin-4(1-39)NH₂; (SEQ ID NO:84)
   (Aib², Arg^{12,27}, Phe²⁵, *β*-Ala²⁹) Exendin-4(1-39)NH₂; (SEQ ID NO:84)
   (Aib², Nle¹⁴, Phe²⁵, *β*-Ala²⁹) Exendin-4(1-39)NH₂; (SEQ ID NO:85)
   (Aib², Leu¹⁴, Phe²⁵, *β*-Ala²⁹) Exendin-4(1-39)NH₂; (SEQ ID NO:85)
   (Aib², A6c¹⁴, Phe²⁵, *β*-Ala²⁹) Exendin-4(1-39)NH₂; (SEQ ID NO:85)
   (Aib², Phe²⁵, β-Ala²⁹) Exendin-4(1-39)NH₂; (SEQ ID NO:85)
   (Aib², Arg^{12,27,30}, Nle¹⁴, *β*-Ala²⁹, Acp³¹) Exendin-4(1-31)NH₂; (SEQ ID NO:86)
   (Aib², Nle¹⁴, *β*-Ala²⁹, Arg³⁰, Acp³¹) Exendin-4(1-31)NH₂; (SEQ ID NO:87)
   (Aib², Arg^{12,27}, Nle¹⁴, *β*-Ala²⁹, Acp⁴⁰) Exendin-4(1-40)NH₂; (SEQ ID NO:88)
   (Aib², Nle¹⁴ *β*-Ala²⁹, Acp⁴⁰) Exendin-4(1-40)NH₂; (SEQ ID NO:89)
   (Aib², Lys¹²(N^{ε}-Acp-octanoyl), Nle¹⁴, *β*-Ala²⁹) Exendin-4(1-39)NH₂; (SEQ ID NO:90)
   (Aib², Lys¹²(N^{ε}-Acp-octanoyl), Nle¹⁴, Arg²⁷, *β*-Ala²⁹) Exendin-4(1-39)NH₂; (SEQ ID NO:90)
   (Aib², Nle¹⁴, Lys²⁷(N^{ε}-Acp-octanoyl), *β*-Ala²⁹) Exendin-4(1-39)NH₂; (SEQ ID NO:91)
   (Aib², Arg¹², Nle¹⁴, Lys²⁷(N^{ε}-Acp-octanoyl), *β*-Ala²⁹) Exendin-4(1-39)NH₂; (SEQ ID NO:91)
   (Aib², Arg^{12,27}, Nle¹⁴, *β*-Ala²⁹, Acp⁴⁰) Exendin-4(1-40)NH₂; (SEQ ID NO:88) or (Aib², Nle¹⁴, Lys²⁷(N^{ε}-Acp-decanoyl), *β*-Ala²⁹) Exendin-4(1-39)NH₂; (SEQ ID NO:92)
   or a pharmaceutically acceptable salt thereof.
5. A compound of paragraph 2, wherein said compound is:
   (Aib²) Exendin-4(1-39)NH₂; (SEQ ID NO:4)
   (Aib², Nle¹⁴) Exendin-4(1-39)NH₂; (SEQ ID NO:5)
   (Aib², Leu¹⁴) Exendin-4(1-39)NH₂; (SEQ ID NO:5)
   (Aib², A6c¹⁴) Exendin-4(1-39)NH₂; (SEQ ID NO:5)
   (Aib², Arg^{12,27}, Nle¹⁴) Exendin-4(1-39)NH₂; (SEQ ID NO:6)
   (Aib², Arg^{12,27}, Leu¹⁴) Exendin-4(1-39)NH₂; (SEQ ID NO:6)
   (Aib^{2,28}, Nle¹⁴) Exendin-4(1-39)NH₂; (SEQ ID NO:7)
   (Aib^{2,28}, Leu¹⁴) Exendin-4(1-39)NH₂; (SEQ ID NO:7)
   (Aib^{2,29}, Nle¹⁴) Exendin-4(1-39)NH₂; (SEQ ID NO:8)
   (Aib^{2,29}, Leu¹⁴) Exendin-4(1-39)NH₂; (SEQ ID NO:8)
   (Aib², Nle¹⁴ *β*-Ala²⁹) Exendin-4(1-39)NH₂; (SEQ ID NO:9)
   (Aib², Leu¹⁴, *β*-Ala²⁹) Exendin-4(1-39)NH₂; (SEQ ID NO:9)
   (Aib², *β*-Ala²⁹) Exendin-4(1-39)NH2; (SEQ ID NO:9)
   (Aib^{2,29}) Exendin-4(1-39)NH₂; (SEQ ID NO:8)
   (Aib², Nle¹⁴ A6c²⁶) Exendin-4(1-39)NH₂; (SEQ ID NO:10)
   (Aib², Leu¹⁴, A6c²⁶) Exendin-4(1-39)NH₂; (SEQ ID NO:10)
   (Aib², A6c¹⁴) Exendin-4(1-39)NH₂; (SEQ ID NO:5)
   (Aib², Nle¹⁴, Ser³⁹(O-decanoyl)) Exendin-4(1-39)NH₂; (SEQ ID NO:19)
   (Aib², Leu¹⁴, Sex³⁹(O-decanoyl)) Exendin-4(1-39)NH₂; (SEQ ID NO:19)
   (Aib², Nle¹⁴, Lys²⁷(N^{ε}-octanoyl)) Exendin-4(1-39)NH₂; (SEQ ID NO:20)
   (Aib², Leu¹⁴, Lys²⁷(N^{ε}-octanoyl)) Exendin-4(1-39)NH₂; (SEQ ID NO:20)
   (Aib², Arg¹², Nle¹⁴ Lys²⁷(N^{ε}-octanoyl)) Exendin-4(1-39)NH₂; (SEQ ID NO:21)
   (Aib², Arg¹², Leu¹⁴, Lys²⁷(N^{ε}-octanoyl)) Exendin-4(1-39)NH₂; (SEQ ID NO:21)
   (Aib², Lys¹² (N^{ε}-octanoyl), Nle¹⁴, Arg²⁷) Exendin-4(1-39)NH₂; (SEQ ID NO:22)
   (Aib², Arg^{12,27}, Nle¹⁴) Exendin-3(1-39)NH₂; (SEQ ID NO:26) (SEQ ID NO:26)
   (Aib², Arg^{12,27}, Leu¹⁴) Exendin-3(1-39)NH₂; (SEQ ID NO:26) (SEQ ID NO:26)
   (Aib², Arg¹², Nle¹⁴, Lys²⁷(N^{ε}-octanoyl)) Exendin-3(1-39)NH₂; (SEQ ID NO:42)
   (Aib², Nle¹⁴, *β*-Ala²⁹, Arg³⁰) Exendin-4(1-30)NH₂; (SEQ ID NO:45)
   (Aib², Leu¹⁴, *β*-Ala²⁹, Arg³⁰) Exendin-4(1-30)NH₂; (SEQ ID NO:45)
   (Aib^{2,29}, Nle¹⁴, Arg³⁰) Exendin-4(1-30)NH₂; (SEQ ID NO:46)
   (Aib^{2,29}, Leu¹⁴, Arg³⁰) Exendin-4(1-30)NH₂; (SEQ ID NO:46)
   (Aib², Arg^{12,27,30}, Nle¹⁴, *β*-Ala²⁹) Exendin-4(1-30)NH₂; (SEQ ID NO:49)
   (Aib², Arg^{12,27,30}, Leu¹⁴, *β*-Ala²⁹) Exendin-4(1-30)NH₂; (SEQ ID NO:49)
   (Aib^{2,29}, Arg^{12,27,30}, Nle¹⁴) Exendin-4(1-30)NH₂; (SEQ ID NO:50)
   (Aib^{2,29}, Arg^{12,27,30}, Leu¹⁴) Exendin-4(1-30)NH₂; (SEQ ID NO:50)
   (Nle¹⁴, *β*-Ala²⁹, Arg³⁰) Exendin-4(1-30)NH₂; (SEQ ID NO:51)
   (Leu¹⁴, *β*-Ala²⁹, Arg³⁰) Exendin-4(1-30)NH₂; (SEQ ID NO:51)
   (Nle¹⁴ Aib²⁹, Arg³⁰) Exendin-4(1-30)NH₂; (SEQ ID NO:52)
   (Leu¹⁴ Aib²⁹, Arg³⁰) Exendin-4(1-30)NH₂; (SEQ ID NO:52)
   (Aib², *β*-Ala²⁹, Arg³⁰) Exendin-4(1-30)NH₂; (SEQ ID NO:45)
   (Aib^{2,29}, Arg³⁰) Exendin-4(1-30)NH₂; (SEQ ID NO:46)
   (Aib², Nle¹⁴, *β*-Ala²⁹) Exendin-4(1-30)NH₂; (SEQ ID NO:53)
   (Aib², Leu¹⁴, *β*-Ala²⁹) Exendin-4(1-30)NH₂; (SEQ ID NO:53)
   (Aib^{2,29}, Nle¹⁴) Exendin-4(1-30)NH₂; (SEQ ID NO:54)
   (Aib², Nle¹⁴, Ser³¹(O-decanoyl)) Exendin-4(1-31)NH₂; (SEQ ID NO:57)
   (Aib², Arg¹², Nle¹⁴, Lys²⁷(N^{ε}-octanoyl)) Exendin-4(1-30)NH₂; (SEQ ID NO:60)
   (Aib², Arg¹², Leu¹⁴, Lys²⁷(N^{ε}-octanoyl)) Exendin-4(1-30)NH₂; (SEQ ID NO:60)
   (Aib², Nle¹⁴, *β*-Ala²⁹, Arg³⁰) Exendin-3(1-30)NH₂; (SEQ ID NO:63)
   (Aib², Leu¹⁴, *β*-Ala²⁹, Arg³⁰) Exendin-3(1-30)NH₂; (SEQ ID NO:63)
   (Aib^{2,29}, Nle¹⁴, Arg³⁰) Exendin-3(1-30)NH₂; (SEQ ID NO:64)
   (Aib², Arg^{12,27}, Nle¹⁴, *β*-Ala²⁹) Exendin-4(1-39)NH₂; (SEQ ID NO:81)
   (Aib², Arg^{12,27}, Leu¹⁴, *β*-Ala²⁹) Exendin-4(1-39)NH₂; (SEQ ID NO:81)
   (Aib², Arg^{12,27}, Nle¹⁴, Phe²⁵, *β*-Ala²⁹) Exendin-4(1-39)NH₂; (SEQ ID NO:84)
   (Aib², Arg^{12,27}, Leu¹⁴, Phe²⁵, *β*-Ala²⁹) Exendin-4(1-39)NH₂; (SEQ ID NO:84)
   (Aib², Nle¹⁴, Phe²⁵, *β*-Ala²⁹) Exendin-4(1-39)NH₂; (SEQ ID NO:85)
   (Aib², Leu¹⁴, Phe²⁵, *β*-Ala²⁹) Exendin-4(1-39)NH₂; (SEQ ID NO:85)
   (Aib², Arg^{12,27,30}, Nle¹⁴, *β*-Ala²⁹, Acp³¹) Exendin-4(1-31)NH₂; (SEQ ID NO:86)
   (Aib², Nle¹⁴, *β*-Ala²⁹, Arg³⁰, Acp³¹) Exendin-4(1-31)NH₂; (SEQ ID NO:87)
   (Aib², Arg^{12,27}, Nle¹⁴, *β*-Ala²⁹, Acp⁴⁰) Exendin-4(1-40)NH₂; (SEQ ID NO:88)
   (Aib², Nle¹⁴, *β*-Ala²⁹, Acp⁴⁰) Exendin-4(1-40)NH₂; (SEQ ID NO:89)
   (Aib², Nle¹⁴, Lys²⁷(N^{ε}-Acp-octanoyl), *β*-Ala²⁹) Exendin-4(1-39)NH₂; (SEQ ID NO:91)
   (Aib², Arg¹², Nle¹⁴, Lys²⁷(N^{ε}-Acp-octanoyl), *β*-Ala²⁹) Exendin-4(1-39)NH₂; (SEQ ID NO:91)
   (Aib², Arg^{12,27}, Nle¹⁴, *β*-Ala²⁹, Acp⁴⁰) Exendin-4(1-40)NH₂; (SEQ ID NO:88) or
   (Aib², Nle¹⁴, Lys²⁷(N^{ε}-Acp-decanoyl), *β*-Ala²⁹) Exendin-4(1-39)NH₂; (SEQ ID NO:92)
   or a pharmaceutically acceptable salt thereof.
6. A compound according to paragraph 5, wherein said compound is
   (Aib²) Exendin-4(1-39)NH₂; (SEQ ID NO:4)
   (Aib², Nle¹⁴) Exendin-4(1-39)NH₂; (SEQ ID NO:5)
   (Aib², Leu¹⁴) Exendin-4(1-39)NH₂; (SEQ ID NO:5)
   (Aib², A6c¹⁴) Exendin-4(1-39)NH₂; (SEQ ID NO:5)
   (Aib², Arg^{12,27}, Nle¹⁴) Exendin-4(1-39)NH₂; (SEQ ID NO:6)
   (Aib², Arg^{12,27}, Leu¹⁴) Exendin-4(1-39)NH₂; (SEQ ID NO:6)
   (Aib^{2,28}, Nle¹⁴) Exendin-4(1-39)NH₂; (SEQ ID NO:7)
   (Aib^{2,29}, Nle¹⁴) Exendin-4(1-39)NH₂; (SEQ ID NO:8)
   (Aib², Nle¹⁴, *β*-Ala²⁹) Exendin-4(1-39)NH₂; (SEQ ID NO:9)
   (Aib², Leu¹⁴, *β*-Ala²⁹) Exendin-4(1-39)NH₂; (SEQ ID NO:9)
   (Aib², Nle¹⁴ A6c²⁶) Exendin-4(1-39)NH₂; (SEQ ID NO:10)
   (Aib², Nle¹⁴, Ser³⁹(O-decanoyl)) Exendin-4(1-39)NH₂; (SEQ ID NO:19)
   (Aib², Nle¹⁴, Lys²⁷(N^{ε}-octanoyl)) Exendin-4(1-39)NH₂; (SEQ ID NO:20)
   (Aib², Arg¹², Nle¹⁴ Lys²⁷(N^{ε}-octanoyl)) Exendin-4(1-39)NH₂; (SEQ ID NO:21)
   (Aib², Nle¹⁴, *β*-Ala²⁹, Arg³⁰) Exendin-4(1-30)NH₂; (SEQ ID NO:45)
   (Aib², Leu¹⁴, *β*-Ala²⁹, Arg³⁰) Exendin-4(1-30)NH₂; (SEQ ID NO:45)
   (Aib^{2,29}, Nle¹⁴, Arg³⁰) Exendin-4(1-30)NH₂; (SEQ ID NO:46)
   (Aib², Arg^{12,27,30}, Nle¹⁴, *β*-Ala²⁹) Exendin-4(1-30)NH₂; (SEQ ID NO:49)
   (Aib², Arg^{12,27,30}, Leu^{14,} *β*-Ala²⁹) Exendin-4(1-30)NH₂; (SEQ ID NO:49)
   (Aib², Nle¹⁴, Ser³¹(O-decanoyl)) Exendin-4(1-31)NH₂; (SEQ ID NO:57)
   (Aib², Arg¹², Nle¹⁴, Lys²⁷(N^{ε}-octanoyl)) Exendin-4(1-30)NH₂; (SEQ ID NO:60)
   (Aib², Arg^{12,27}, Nle¹⁴, *β*-Ala²⁹) Exendin-4(1-39)NH2; (SEQ ID NO:81)
   (Aib², Arg^{12,27}, Leu¹⁴, *β*-Ala²⁹) Exendin-4(1-39)NH2; (SEQ ID NO:81)
   (Aib², Arg^{12,27}, Nle¹⁴, Phe²⁵, *β*-Ala²⁹) Exendin-4(1-39)NH2; (SEQ ID NO:84)
   (Aib², Arg^{12,27}, Leu¹⁴, Phe²⁵, *β*-Ala²⁹) Exendin-4(1-39)NH2; (SEQ ID NO:84)
   (Aib², Arg^{12,27,30}, Nle¹⁴, *β*-Ala²⁹, Acp³¹) Exendin-4(1-31)NH₂; (SEQ ID NO:86)
   (Aib², Arg^{12,27}, Nle¹⁴, *β*-Ala²⁹, Acp⁴⁰) Exendin-4(1-40)NH₂; (SEQ ID NO:88)
   (Aib², Arg¹², Nle¹⁴, Lys²⁷(N-Acp-octanoyl), *β*-Ala²⁹) Exendin-4(1-39)NH2; (SEQ ID NO:91)
   (Aib², Arg^{12,27}, Nle¹⁴, *β*-Ala²⁹, Acp⁴⁰) Exendin-4(1-40)NH₂; (SEQ ID NO:88) or
   (Aib², Nle¹⁴, Lys²⁷(N^{ε}-Acp-decanoyl), *β*-Ala²⁹) Exendin-4(1-39)NH2; (SEQ ID NO:92)
   or a pharmaceutically acceptable salt thereof.
7. A compound according to paragraph 6, wherein said compound is
   (Aib²) Exendin-4(1-39)NH₂; (SEQ ID NO:4)
   (Aib², Nle¹⁴) Exendin-4(1-39)NH₂; (SEQ ID NO:5)
   (Aib², Leu¹⁴) Exendin-4(1-39)NH₂; (SEQ ID NO:5)
   (Aib^{2,28}, Nle¹⁴) Exendin-4(1-39)NH₂; (SEQ ID NO:7)
   (Aib^{2,29}, Nle¹⁴) Exendin-4(1-39)NH₂; (SEQ ID NO:8)
   (Aib², Nle¹⁴, *β*-Ala²⁹) Exendin-4(1-39)NH₂; (SEQ ID NO:9)
   (Aib², Nle¹⁴, A6c²⁶) Exendin-4(1-39)NH₂; (SEQ ID NO:10)
   (Aib², Nle¹⁴, *β*-Ala²⁹, Arg³⁰) Exendin-4(1-30)NH₂; (SEQ ID NO:45)
   (Aib^{2,29}, Nle¹⁴, Arg³⁰) Exendin-4(1-30)NH₂; (SEQ ID NO:46)
   (Aib², A6c¹⁴) Exendin-4(1-39)NH₂; (SEQ ID NO:5)
   (Aib², Arg^{12,27}, Nle¹⁴, *β*-Ala²⁹, Acp⁴⁰) Exendin-4(1-40)NH₂; (SEQ ID NO:88) or
   (Aib², Nle¹⁴, Lys²⁷(N^{ε}-Acp-decanoyl), *β*-Ala²⁹) Exendin-4(1-39)NH₂; (SEQ ID NO:92)
   or a pharmaceutically acceptable salt thereof.
8. A compound according to paragraph 7, wherein said compound is
   (Aib², A6c¹⁴)Exendin-4(1-39)-NH₂; (SEQ ID NO:5)
   (Aib², Nle¹⁴)Exendin-4(1-39)-NH₂; (SEQ ID NO:5)
   (Aib², Nle¹⁴, *β* -Ala²⁹, Arg³⁰)Exendin-4(1-30)-NH₂; (SEQ ID NO:45)
   (Aib^{2,28}, Nle¹⁴)Exendin-4(1-39)-NH₂; (SEQ ID NO:7)
   (Aib², Nle¹⁴, A6c²⁶)Exendin-4(1-39)-NH₂; (SEQ ID NO:10)
   (Aib^{2,29}, Nle¹⁴)Exendin-4(1-39)-NH₂; (SEQ ID NO:8)
   (Aib², Leu¹⁴)Exendin-4(1-39)-NH₂; (SEQ ID NO:5)
   (Aib², Nle¹⁴, *β* -Ala²⁹)Exendin-4(1-39)-NH₂; (SEQ ID NO:9) or
   (Aib^{2,29}, Nle¹⁴, Arg³⁰)Exendin-4(1-30)-NH₂; (SEQ ID NO:46)
   or a pharmaceutically-acceptable salt thereof.
9. A pharmaceutical composition comprising an effective amount of a compound according to paragraph 1 or a pharmaceutically acceptable salt thereof and a pharmaceutically acceptable carrier or diluent.
10. A method of eliciting a GLP-1 agonist effect in a human or non-human animal subject in need thereof which comprises administering to said subject an effective amount of a compound according to paragraph 1 or a pharmaceutically acceptable salt thereof.
11. A method of treating a disease selected from the group consisting of Type I diabetes, Type II diabetes, obesity, glucagonomas, secretory disorders of the airway, metabolic disorder, arthritis, osteoporosis, central nervous system disease, restenosis and neurodegenerative disease, in a human or non-human animal subject in need thereof which comprises administering to said subject an effective amount of a compound according to paragraph 1 or a pharmaceutically acceptable salt thereof.
12. A method according to paragraph 11 wherein said disease is Type I diabetes or Type II diabetes.
13. A method of eliciting a GLP-1 antagonist effect in a human or non-human animal subject in need thereof which comprises administering to said subject an effective amount of a compound according to paragraph 1 or a pharmaceutically acceptable salt thereof.
14. A method of treating a disease selected from the group consisting of cachexia, anorexia, hypoglycemia and malabsorption syndrome, in a human or non-human animal subject in need thereof which comprises administering to said subject an effective amount of a compound according to paragraph 1 or a pharmaceutically acceptable salt thereof.
15. A method of binding a GLP-1 receptor which comprises the step of contacting said GLP-1 receptor with a compound of paragraph 1 or a pharmaceutically acceptable salt thereof.
16. The method of paragraph 15 wherein said GLP-1 receptor is in a human or non-human animal subject.
17. A method of eliciting a GLP-1 agonist effect from a cell, wherein said cell comprises one or more GLP-1 receptors, said method comprising contacting said cell with an effective amount of a compound of paragraph 1 or a pharmaceutically acceptable salt thereof.
18. A method of eliciting a GLP-1 antagonist effect from a cell, wherein said cell comprises one or more GLP-1 receptors, said method comprising contacting said cell with an effective amount of a compound of paragraph 1 or a pharmaceutically acceptable salt thereof.
19. A method of eliciting a GLP-1 agonist effect in a human or non-human animal subject in need thereof, which comprises the step of administering an effective amount of a compound of paragraph 1 or a pharmaceutically acceptable salt thereof to said subject.
20. A method of eliciting a GLP-1 antagonist effect in a human or non-human animal subject in need thereof, which comprises the step of administering an effective amount of a compound of paragraph 1 or a pharmaceutically acceptable salt thereof to said subject.
21. A method of stimulating insulin release in a human or non-human animal subject in need thereof, which comprises administering to said subject an effective amount of a compound according to paragraph 1 or a pharmaceutically acceptable salt thereof.
22. A method of enhancing insulin sensitivity in a human or non-human animal subject in need thereof, which comprises administering to said subject an effective amount of a compound according to paragraph 1 or a pharmaceutically acceptable salt thereof.
23. A method of lowering blood glucose levels in a human or non-human animal subject in need thereof, which comprises administering to said subject an effective amount of a compound according to paragraph 1 or a pharmaceutically acceptable salt thereof.
24. A method of stimulating cAMP production in beta cells of a human or non-human animal subject in need thereof, which comprises administering to said subject an effective amount of a compound according to paragraph 1 or a pharmaceutically acceptable salt thereof.
25. A method of imaging cells having GLP-1 receptors which comprises contacting said cells with an effective amount of a compound according to paragraph 1, or a pharmaceutically acceptable salt thereof, wherein said compound comprises one or more radioiodinated tyrosine residues.

## Claims

1. A compound according to formula (I):
(R²R³)A¹-A²-A³-A⁴-A⁵-A⁶-A⁷-A⁸-A⁹-A¹⁰-A¹¹-A¹²-A¹³-A¹⁴-A¹⁵-A¹⁶-A¹⁷-A¹⁸-A¹⁹-A²⁰-A²¹-A²²-A²³-A²⁴-A²⁵-A²⁶-A²⁷-A²⁸-A²⁹-A³⁰-A³¹-A³²-A³³-A³⁴-A³⁵-A³⁶-A³⁷-A³⁸-A³⁹-A⁴⁰-A⁴¹-A⁴²-R¹ (I)
or a pharmaceutically acceptable salt thereof,
wherein:
A² is Aib or D-Ala;
A¹⁴ is Nle, Leu or A6c;
A²⁹ is *β*-Ala, Gly, Aib or deleted;
A¹ is His, Hppa, Paa, Pta, Ura or deleted;
A³ is Glu or Asp or Acc, Aib, Ala, Arg, N-Me-Asp, Dab, Dap, N-Me-Glu, Lys,
Orn, HN-CH((CH₂)_{q}-C(O)-O-R⁵)-C(O), a D-Amino acid or deleted;
A⁴ is Gly or Acc, Aib, Ala, *β*-Ala, Arg, Dab, Dap, Lys, Orn, Ser, a D-Amino acid or deleted;
A⁵ is Thr or Acc, Aib, Ala, Arg, Dab, Dap, Lys, Orn, Ser, HN-CH(CH(CH₃)-OC(O)R⁴)-C(O), HN-CH(CH₂OC(O)R⁴)-C(O) or deleted;
A⁶ is Phe or Acc, Aib, Aic, Arg, Cha, Dab, Dap, Lys, *β*-1-Nal, *β*-2-Nal, Orn, 3-Pal, 4-Pal, X¹ᵤ-Phe, Trp or deleted;
A⁷ is Thr or Acc, Aib, Ala, Arg, Dab, Dap, Lys, Orn, Ser, HN-CH(CH(CH₃)-OC(O)R⁴)-C(O), HN-CH(CH₂OC(O)R⁴)-C(O) or deleted;
A⁸ is Ser or Acc, Aib, Ala, Arg, Dab, Dap, Lys, Orn, Thr, HN-CH(CH₂OC(O)R⁴)-C(O), HN-CH(CH(CH₃)-OC(O)R⁴)-C(O) or deleted;
A⁹ is Asp or Aib, Ala, Arg, Dab, Dap, Glu, Lys, Orn or HN-CH((CH₂)_{q}-C(O)-O-R⁵)-C(O);
A¹⁰ is Leu or A6c;
A¹¹ is Ser or Aib;
A¹² is Lys, Arg, hArg, Lys(N^{ε}-octanoyl) or Lys(N^{ε}-Acp-octanoyl);
A¹³ is Gln or Acc, Aib, Arg, Asn, Cha, Dab, Dap, Lys, *β*-1-Nal, *β*-2-Nal, Orn, 3-Pal, 4-Pal, Phe or Tyr;
A¹⁵ is Glu or Acc, Aib, Ala, Arg, Asp, Dab, Dap, Lys, Orn or HN-CH((CH₂)_{q}-C(O)-O-R⁵)-C(O);
A¹⁶ is Glu or Acc, Aib, Ala, *β*-Ala, D-Ala, Arg, Asp, Dab, Dap, Gly, Lys, Orn or HN-CH((CH₂)_{q}-C(O)-O-R⁵)-C(O);
A¹⁷ is Glu or Ser(O-decanoyl);
A¹⁸ is Ala or Aib;
A¹⁹ is Val or Abu, Acc, Aib, Ala, Arg, hArg, Cha, Dab, Dap, Ile, Leu, Lys, Nle, Nva, Orn, Phe, X¹ᵤ-Phe, Tle or HN-CH((CH₂)ₙ-NR⁶R⁷)-C(O);
A²⁰ is Arg or Amp, hArg, Dab, Dap, Lys, Orn, 3-Pal, 4-Pal or HN-CH((CH₂)ₙ-NR⁶R⁷)-C(O);
A²¹ is Leu or Abu, Acc, Aib, Ala, Arg, Asp, Cha, Dab, Dap, Glu, Ile, Lys, Nle, Orn, Phe, X¹ᵤ-Phe, Tle or Val;
A²² is Phe or Acc, Aib, Aic, Ala, Arg, Cha, Dab, Dap, Lys, *β*-1-Nal, *β*-2-Nal, Orn, 3-Pal, 4-Pal, X¹ᵤ-Phe or Trp;
A²³ is Ile or Abu, Acc, Aib, Ala, Arg, Cha, Dab, Dap, Leu, Lys, Nle, Orn, Phe, X¹ᵤ-Phe, Tle or Val;
A²⁴ is Glu or Abu, Acc, Aib, Ala, Arg, Asp, Dab, Dap, Orn, Val or deleted,
A²⁵ is Trp, Phe or deleted;
A²⁶ is Leu, A6c or deleted;
A²⁷ is Lys, Arg, Lys(N^{ε}-octanoyl), Lys(N^{ε}-Acp-octanoyl), Lys(N^{ε}-Acp-decanoyl) or deleted;
A²⁸ is Asn, Aib or deleted;
A³⁰ is Gly, Arg, D-Arg or deleted;
A³¹ is Pro, Acp, Asp, D-Asp, Ava, Ser(O-decanoyl) or deleted,
A³² is Ser, Ser(O-decanoyl), Aun, Ava or deleted;
A³³ is Ser, Ser(O-decanoyl), Aun or deleted;
A³⁴ is Gly or Acc, Aib, Ala, *β*-Ala, Arg, Dab, Dap, Lys, Orn, HN-(CH₂)ₘ-C(O), R⁹, a D-Amino acid or deleted;
A³⁵ is Ala or Abu, Acc, Aib, *β*-Ala, D-Ala, Arg, Dab, Dap, Lys, Orn, Val, HN-(CH₂)ₘ-C(O), R⁹, a D-amino acid or deleted;
A³⁶ is Pro or Acc, Aib, Ala, Arg, Dab, Dap, Lys, Orn, D-Pro, hPro, R⁹, a D-amino acid or deleted;
A³⁷ is Pro or Acc, Aib, Ala, Arg, Dab, Dap, Lys, Orn, D-Pro, hPro, R⁹, a D-amino acid or deleted;
A³⁸ is Pro or Acc, Aib, Ala, Arg, Dab, Dap, Lys, Orn, D-Pro, hPro, R⁹, a D-amino acid or deleted;
A³⁹ is Ser, Ser(O-decanoyl) or deleted;
A⁴⁰ is deleted, Acp, Asp, D-Asp or Ava ;
A⁴¹ is deleted, Aun or Ava;
A⁴² is deleted or Aun;
R¹ is NH₂, OH, or (C₁-C₁₂)alkoxy;
R² and R³ each is independently selected from the group consisting of H, (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl, hydroxynaphthyl(C₁-C₃₀)alkyl and C(O)X³;
R⁴ and R⁵ each is independently (C₁-C₃₀)alkyl;
R⁶ and R⁷ each is, independently for each occurrence, H, (C₁-C₃₀)alkyl, (C₁-C₃₀)acyl, (C₁-C₃₀)alkylsulfonyl, or -C(NH)NH₂;
R⁹ is 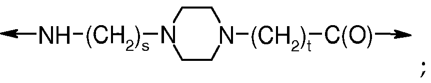
X¹ is, independently for each occurrence, (C₁-C₆)alkyl, OH or halogen;
X³ is (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl, or hydroxynaphthyl(C₁-C₃₀)alkyl;
m is 1 to 20;
n is 1 to 5;
q is 1 or 2;
s is 1 to 5; and
t is 1 to 5;
provided that when R⁶ is (C₁-C₃₀)acyl, (C₁-C₃₀)alkylsulfonyl, or -C(NH)NH₂, then R⁷ is H or (C₁-C₃₀)alkyl; and further provided that said compound is not a compound selected from the group consisting of Exendin 4(v-w)-OH, Exendin 4(v-w)-NH₂, Exendin 3(v-w)-OH, and Exendin 3(v-w)-NH₂, wherein v is 1 - 8 and w is 24-39;
or a pharmaceutically-acceptable salt thereof.

2. A compound according to claim 1, wherein:
A² is Aib;
A²⁹ is *β*-Ala, Aib or Gly;
A¹² is Lys or Arg;
A¹⁸ is Ala, or Aib;
A²⁶ is Leu or A6c;
A²⁷ is Lys, Arg or Lys²⁷(N^{ε}-Acp-decanoyl);
A²⁸ is Asn or Aib;
A³⁰ is Gly or Arg; and
A⁴⁰ is deleted or Acp;
or a pharmaceutically-acceptable salt thereof.

3. A compound according to claim 1, wherein said compound is:
(Aib², Nle¹⁴, *β*-Ala²⁹) Exendin-4(1-39)NH₂; (SEQ ID NO:9)
(Aib²) Exendin-4(1-39)NH₂; (SEQ ID NO:4)
(Aib², Nle¹⁴) Exendin-4(1-39)NH₂; (SEQ ID NO:5)
(Aib², Leu¹⁴) Exendin-4(1-39)NH₂; (SEQ ID NO:5)
(Aib², A6c¹⁴) Exendin-4(1-39)NH₂; (SEQ ID NO:5)
(Aib², Arg^{12,27}, Nle¹⁴) Exendin-4(1-39)NH₂; (SEQ ID NO:6)
(Aib², Arg^{12,27}, Leu¹⁴) Exendin-4(1-39)NH₂; (SEQ ID NO:6)
(Aib^{2,28}, Nle¹⁴) Exendin-4(1-39)NH₂; (SEQ ID NO:7)
(Aib^{2,28}, Leu¹⁴) Exendin-4(1-39)NH₂; (SEQ ID NO:7)
(Aib^{2,29}, Nle¹⁴) Exendin-4(1-39)NH₂; (SEQ ID NO:8)
(Aib^{2,29}, Leu¹⁴) Exendin-4(1-39)NH₂; (SEQ ID NO:8)
(Aib², Leu¹⁴,*β*-Ala²⁹) Exendin-4(1-39)NH₂; (SEQ ID NO:9)
(Aib², Nle¹⁴, A6c²⁶) Exendin-4(1-39)NH₂; (SEQ ID NO:10)
(Aib², Leu¹⁴, A6c²⁶) Exendin-4(1-39)NH₂; (SEQ ID NO:10)
(Aib², A6c¹⁴) Exendin-4(1-39)NH₂; (SEQ ID NO:5)
(Aib², A6c¹⁰, Leu¹⁴) Exendin-4(1-39)NH₂; (SEQ ID NO:11)
(Aib², A6c¹⁰, Nle¹⁴) Exendin-4(1-39)NH₂; (SEQ ID NO:11)
(Aib², A6c^{10,14}) Exendin-4(1-39)NH₂; (SEQ ID NO:11)
(Aib^{2,18}, Nle¹⁴) Exendin-4(1-39)NH₂; (SEQ ID NO:12)
(Aib^{2,18}, Leu¹⁴) Exendin-4(1-39)NH₂; (SEQ ID NO:12)
(Aib^{2,11}, Nle¹⁴) Exendin-4(1-39)NH₂; (SEQ ID NO:13)
(Aib^{2,11}, Leu¹⁴) Exendin-4(1-39)NH₂; (SEQ ID NO:13)
(D-Ala², Nle¹⁴) Exendin-4(1-39)NH₂; (SEQ ID NO:14)
(D-Ala², Leu¹⁴) Exendin-4(1-39)NH₂; (SEQ ID NO:14)
(Aib², A6c^{14,26}) Exendin-4(1-39)NH₂; (SEQ ID NO:10)
(Aib^{2,18}, Nle¹⁴) Exendin-4(1-39)NH₂; (SEQ ID NO:12)
(Aib², Nle¹⁴, Ser³²(O-decanoyl)) Exendin-4(1-39)NH₂; (SEQ ID NO:17)
(Aib², Leu¹⁴, Ser³²(O-decanoyl)) Exendin-4(1-39)NH₂; (SEQ ID NO:17)
(Aib², Nle¹⁴, Ser³³(O-decanoyl)) Exendin-4(1-39)NH₂; (SEQ ID NO:18)
(Aib², Leu¹⁴, Ser³³(O-decanoyl)) Exendin-4(1-39)NH₂; (SEQ ID NO:18)
(Aib², Nle¹⁴, Ser³⁹(O-decanoyl)) Exendin-4(1-39)NH₂; (SEQ ID NO:19)
(Aib², Leu¹⁴, Ser³⁹(O-decanoyl)) Exendin-4(1-39)NH₂; (SEQ ID NO:19)
(Aib², Nle¹⁴, Lys²⁷(N^{ε}-octanoyl)) Exendin-4(1-39)NH₂; (SEQ ID NO:20)
(Aib², Leu¹⁴, Lys²⁷(N^{ε}-octanoyl)) Exendin-4(1-39)NH₂; (SEQ ID NO:20)
(Aib², Arg¹², Nle¹⁴, Lys²⁷(N^{ε}-octanoyl)) Exendin-4(1-39)NH₂; (SEQ ID NO:21)
(Aib², Arg¹², Leu¹⁴, Lys²⁷(N^{ε}-octanoyl)) Exendin-4(1-39)NH₂; (SEQ ID NO:21)
(Aib², Lys¹²(N^{ε}-octanoyl), Nle¹⁴, Arg²⁷) Exendin-4(1-39)NH₂; (SEQ ID NO:22)
(Aib², Lys¹²(N^{ε}-octanoyl), Leu¹⁴, Arg²⁷) Exendin-4(1-39)NH₂; (SEQ ID NO:22)
(Aib², Arg^{12,27}, Nle¹⁴, Ava⁴⁰, Aun⁴¹) Exendin-4(1-41)NH₂; (SEQ ID NO:94)
(Aib², Arg^{12,27}, Nle¹⁴, Asp⁴⁰, Ava⁴¹, Aun⁴²) Exendin-4(1-42)NH₂; (SEQ ID NO:23)
(Aib², Arg^{12,27}, Nle¹⁴, D-Asp⁴⁰, Ava⁴¹, Aun⁴²) Exendin-4(1-42)NH₂; (SEQ ID NO:23)
(Aib², Nle¹⁴) Exendin-3(1-39)NH₂; (SEQ ID NO:25)
(Aib², Leu¹⁴) Exendin-3(1-39)NH₂; (SEQ ID NO:25)
(Aib², A6c¹⁴) Exendin-3(1-39)NH₂; (SEQ ID NO:25)
(Aib², Arg^{12,27}, Nle¹⁴) Exendin-3(1-39)NH₂; (SEQ ID NO:26)
(Aib², Arg^{12,27}, Leu¹⁴) Exendin-3(1-39)NH₂; (SEQ ID NO:26)
(Aib^{2,28}, Nle¹⁴) Exendin-3(1-39)NH₂; (SEQ ID NO:27)
(Aib^{2,28}, Leu¹⁴) Exendin-3(1-39)NH₂; (SEQ ID NO:27)
(Aib^{2,29}, Nle¹⁴) Exendin-3(1-39)NH₂; (SEQ ID NO:28)
(Aib^{2,29}, Leu¹⁴) Exendin-3(1-39)NH₂; (SEQ ID NO:28)
(Aib², Nle¹⁴, *β*-Ala²⁹) Exendin-3(1-39)NH₂; (SEQ ID NO:29)
(Aib², Leu¹⁴, *β*-Ala²⁹) Exendin-3(1-39)NH₂; (SEQ ID NO:29)
(Aib², Nle¹⁴, A6c²⁶) Exendin-3(1-39)NH₂; (SEQ ID NO:30)
(Aib², Leu¹⁴, A6c²⁶) Exendin-3(1-39)NH₂; (SEQ ID NO:30)
(Aib², A6c¹⁴) Exendin-3(1-39)NH₂; (SEQ ID NO:25)
(Aib², A6c¹⁰, Leu¹⁴) Exendin-3(1-39)NH₂; (SEQ ID NO:31)
(Aib², A6c¹⁰, Nle¹⁴) Exendin-3(1-39)NH₂; (SEQ ID NO:31)
(Aib², A6c^{10,14}) Exendin-3(1-39)NH₂; (SEQ ID NO:31)
(Aib^{2,18}, Nle¹⁴) Exendin-3(1-39)NH₂; (SEQ ID NO:32)
(Aib^{2,18}, Leu¹⁴) Exendin-3(1-39)NH₂; (SEQ ID NO:32)
(Aib^{2,11}, Nle¹⁴) Exendin-3(1-39)NH₂; (SEQ ID NO:33)
(Aib^{2,11}, Leu¹⁴) Exendin-3(1-39)NH₂; (SEQ ID NO:33)
(D-Ala², Nle¹⁴) Exendin-3(1-39)NH₂; (SEQ ID NO:34)
(D-Ala², Leu¹⁴) Exendin-3(1-39)NH₂; (SEQ ID NO:34)
(Aib², A6c^{14,26}) Exendin-3(1-39)NH₂; (SEQ ID NO:30)
(Aib², Nle¹⁴, Ser³²(O-decanoyl)) Exendin-3(1-39)NH₂; (SEQ ID NO:37)
(Aib², Leu¹⁴, Ser³²(O-decanoyl)) Exendin-3(1-39)NH₂; (SEQ ID NO:37)
(Aib², Nle¹⁴, Ser³³(O-decanoyl)) Exendin-3(1-39)NH₂; (SEQ ID NO:38)
(Aib², Leu¹⁴, Ser³³(O-decanoyl)) Exendin-3(1-39)NH₂; (SEQ ID NO:38)
(Aib², Nle¹⁴, Ser³⁹(O-decanoyl)) Exendin-3(1-39)NH₂; (SEQ ID NO:39)
(Aib², Leu¹⁴, Ser³⁹(O-decanoyl)) Exendin-3(1-39)NH₂; (SEQ ID NO:39)
(Aib², Nle¹⁴, Ser¹⁷(O-decanoyl)) Exendin-3(1-39)NH₂; (SEQ ID NO:40)
(Aib², Leu¹⁴, Ser¹⁷(O-decanoyl)) Exendin-3(1-39)NH₂; (SEQ ID NO:40)
(Aib², Nle¹⁴, Lys²⁷(N^{ε}-octanoyl)) Exendin-3(1-39)NH₂; (SEQ ID NO:41)
(Aib², Leu¹⁴, Lys²⁷(N^{ε}-octanoyl)) Exendin-3(1-39)NH₂; (SEQ ID NO:41)
(Aib², Arg¹², Nle¹⁴, Lys²⁷(N^{ε}-octanoyl)) Exendin-3(1-39)NH₂; (SEQ ID NO:42)
(Aib², Arg¹², Leu¹⁴, Lys²⁷(N^{ε}-octanoyl)) Exendin-3(1-39)NH₂; (SEQ ID NO:42)
(Aib², LyS¹²(N^{ε}-octanoyl), Nle¹⁴, Arg²⁷) Exendin-3(1-39)NH₂; (SEQ ID NO:43)
(Aib², LyS¹²(N^{ε}-octanoyl), Leu¹⁴, Arg²⁷) Exendin-3(1-39)NH₂; (SEQ ID NO:43) (Aib², Arg^{12,27}, Nle¹⁴, Ava⁴⁰, Aun⁴¹) Exendin-3(1-41)NH₂; (SEQ ID NO:95) (Aib², Arg^{12,27}, Nle¹⁴, Asp⁴⁰, Ava⁴¹, Aun⁴²) Exendin-3(1-42)NH₂; (SEQ ID NO:44)
(Aib², Arg^{12,27}, Nle¹⁴, D-Asp⁴⁰, Ava⁴¹, Aun⁴²) Exendin-3(1-42)NH₂; (SEQ ID NO:44)
(Aib², Nle¹⁴, *β*-Ala²⁹, Arg³⁰) Exendin-4(1-30)NH₂; (SEQ ID NO:45) (Aib², Leu¹⁴, *β*-Ala²⁹, Arg³⁰) Exendin-4(1-30)NH₂; (SEQ ID NO:45) (Aib², A6c¹⁴, *β*-Ala²⁹, Arg³⁰) Exendin-4(1-30)NH₂; (SEQ ID NO:45) (Aib^{2,29}, Nle¹⁴, Arg³⁰) Exendin-4(1-30)NH₂; (SEQ ID NO:46) (Aib^{2,29}, Leu¹⁴, Arg³⁰) Exendin-4(1-30)NH₂; (SEQ ID NO:46) (Aib^{2,29}, A6c¹⁴, Arg³⁰) Exendin-4(1-30)NH₂; (SEQ ID NO:46) (Aib², Nle¹⁴, *β*-Ala²⁹, D-Arg³⁰) Exendin-4(1-30)NH₂; (SEQ ID NO:47) (Aib², Leu¹⁴,*β*-Ala²⁹, D-Arg³⁰) Exendin-4(1-30)NH₂; (SEQ ID NO:47) (Aib^{2,29}, Nle¹⁴, D-Arg³⁰) Exendin-4(1-30)NH₂; (SEQ ID NO:48) (Aib^{2,29}, Leu¹⁴, D-Arg³⁰) Exendin-4(1-30)NH₂; (SEQ ID NO:48) (Aib², Arg^{12,27,30}, Nle¹⁴, *β*-Ala²⁹) Exendin-4(1-30)NH₂; (SEQ ID NO:49) (Aib², Arg^{12,27,30}, Leu¹⁴, *β*-Ala²⁹) Exendin-4(1-30)NH₂; (SEQ ID NO:49) (Aib^{2,29}, Arg^{12,27,30}, Nle¹⁴) Exendin-4(1-30)NH₂; (SEQ ID NO:50) (Aib^{2,29}, Arg^{12,27,30}, Leu¹⁴) Exendin-4(1-30)NH₂; (SEQ ID NO:50)
(Aib², Nle¹⁴, *β*-Ala²⁹) Exendin-4(1-30)NH₂; (SEQ ID NO:53) (Aib², Leu¹⁴,*β*-Ala²⁹) Exendin-4(1-30)NH₂; (SEQ ID NO:53) (Aib^{2,29}, Nle¹⁴) Exendin-4(1-30)NH₂; (SEQ ID NO:54) (Aib^{2,29}, Leu¹⁴) Exendin-4(1-30)NH₂; (SEQ ID NO:54) (Aib², Nle¹⁴, Ser³¹(O-decanoyl)) Exendin-4(1-31)NH₂; (SEQ ID NO:57) (Aib², Leu¹⁴, Ser³¹(O-decanoyl)) Exendin-4(1-31)NH₂; (SEQ ID NO:57) (Aib², Nle¹⁴, Ser¹⁷(O-decanoyl)) Exendin-4(1-30)NH₂; (SEQ ID NO:58) (Aib², Leu¹⁴, Ser¹⁷(O-decanoyl)) Exendin-4(1-30)NH₂; (SEQ ID NO:58) (Aib², Nle¹⁴, Lys²⁷(N^{ε}-octanoyl)) Exendin-4(1-30)NH₂; (SEQ ID NO:59) (Aib², Leu¹⁴, Lys²⁷(N^{ε}-octanoyl)) Exendin-4(1-30)NH₂; (SEQ ID NO:59) (Aib², Arg¹², Nle¹⁴, Lys²⁷(N^{ε}-octanoyl)) Exendin-4(1-30)NH₂; (SEQ ID NO:60)
(Aib², Arg¹², Leu¹⁴, Lys²⁷(N^{ε}-octanoyl)) Exendin-4(1-30)NH₂; (SEQ ID NO:60)
(Aib², Lys¹²(N^{ε}-octanoyl), Nle¹⁴, Arg²⁷) Exendin-4(1-30)NH₂; (SEQ ID NO:61)
(Aib², Lys¹²(N^{ε}-octanoyl), Leu¹⁴, Arg²⁷) Exendin-4(1-30)NH₂; (SEQ ID NO:61)
(Aib², Arg¹²,^{27,30},Nle¹⁴, Ava³¹, Aun³²) Exendin-4(1-32)NH₂; (SEQ ID NO:96)
(Aib², Arg¹², ²⁷, ³⁰, Nle¹⁴, Asp³¹, Ava³², Aun³³) Exendin-4(1-32)NH₂; (SEQ ID NO:62)
(Aib², Arg¹², ^{27, 30}, Nle¹⁴, D-Asp³¹, Ava³², Aun³³) Exendin-4(1-32)NH₂; (SEQ ID NO:62)
(Aib², Nle¹⁴, *β*-Ala²⁹, Arg³⁰) Exendin-3(1-30)NH₂; (SEQ ID NO:63)
(Aib², Leu¹⁴, *β-*Ala²⁹, Arg³⁰) Exendin-3(1-30)NH₂; (SEQ ID NO:63)
(Aib², A6c¹⁴ *β*-Ala²⁹, Arg³⁰) Exendin-3(1-30)NH₂; (SEQ ID NO:63)
(Aib^{2,29}, Nle¹⁴, Arg³⁰) Exendin-3(1-30)NH₂; (SEQ ID NO:64)
(Aib^{2,29}, Leu¹⁴, Arg³⁰) Exendin-3(1-30)NH₂; (SEQ ID NO:64)
(Aib^{2,29}, A6c¹⁴, Arg³⁰) Exendin-3(1-30)NH₂; (SEQ ID NO:64)
(Aib², Nle¹⁴, *β*-Ala²⁹, D-Arg³⁰) Exendin-3(1-30)NH₂; (SEQ ID NO:65)
(Aib², Leu¹⁴, *β*-Ala²⁹, D-Arg³⁰) Exendin-3(1-30)NH₂; (SEQ ID NO:65)
(Aib^{2,29}, Nle¹⁴, D-Arg³⁰) Exendin-3(1-30)NH₂; (SEQ ID NO:66)
(Aib^{2,29}, Leu¹⁴, D-Arg³⁰) Exendin-3(1-30)NH₂; (SEQ ID NO:66)
(Aib², Arg^{12,27,30}, Nle¹⁴, *β*-Ala²⁹) Exendin-3(1-30)NH₂; (SEQ ID NO:67)
(Aib², Arg^{12,27,30}, Leu¹⁴, *β*-Ala²⁹) Exendin-3(1-30)NH₂; (SEQ ID NO:67)
(Aib^{2,29}, Arg^{12,27,30}, Nle¹⁴) Exendin-3(1-30)NH₂; (SEQ ID NO:68)
(Aib^{2,29}, Arg^{12,27,30}, Leu¹⁴) Exendin-3(1-30)NH₂; (SEQ ID NO:68)
(Aib², Nle¹⁴, *β*-Ala²⁹) Exendin-3(1-30)NH₂; (SEQ ID NO:71)
(Aib², Leu¹⁴, *β*-Ala²⁹) Exendin-3(1-30)NH₂;(SEQ ID NO:71)
(Aib^{2,29}, Nle¹⁴) Exendin-3(1-30)NH₂; (SEQ ID NO:72)
(Aib^{2,29}, Leu¹⁴) Exendin-3(1-30)NH₂; (SEQ ID NO:72)
(Aib², Nle¹⁴, Ser³¹(O-decanoyl)) Exendin-3(1-31)NH₂; (SEQ ID NO:75)
(Aib², Leu¹⁴, Ser³¹(O-decanoyl)) Exendin-3(1-31)NH₂; (SEQ ID NO:75)
(Aib², Nle¹⁴, Ser¹⁷(O-decanoyl)) Exendin-3(1-30)NH₂; (SEQ ID NO:76)
(Aib², Leu¹⁴, Ser¹⁷(O-decanoyl)) Exendin-3(1-30)NH₂; (SEQ ID NO:76)
(Aib², Nle¹⁴, Lys²⁷(N^{ε}-octanoyl)) Exendin-3(1-30)NH₂; (SEQ ID NO:77)
(Aib², Leu¹⁴, Lys²⁷(N^{ε}-octanoyl)) Exendin-3(1-30)NH₂; (SEQ ID NO:77)
(Aib², Arg¹², Nle¹⁴, Lys²⁷(N^{ε}-octanoyl)) Exendin-3(1-30)NH₂; (SEQ ID NO:78)
(Aib², Arg¹², Leu¹⁴, Lys²⁷(N^{ε}-octanoyl)) Exendin-3(1-30)NH₂; (SEQ ID NO:78)
(Aib², Lys¹² (N^{ε}-octanoyl), Nle¹⁴, Arg²⁷) Exendin-3(1-30)NH₂; (SEQ ID NO:79)
(Aib², Lys¹² (N^{ε}-octanoyl), Leu¹⁴, Arg²⁷) Exendin-3(1-30)NH₂; (SEQ ID NO:79)
(Aib², Arg^{12,27,30}, Nle¹⁴, Ava³¹, Aun³²) Exendin-3(1-32)NH₂;(SEQ ID NO:97)
(Aib², Arg^{12,27,30}, Nle¹⁴, Asp³¹, Ava³², Aun³³) Exendin-3(1-33)NH₂; (SEQ ID NO:80)
(Aib², Arg^{12,27,30}, Nle¹⁴, D-Asp³¹, Ava³², Aun³³) Exendin-3(1-33)NH₂; (SEQ ID NO:80)
(Aib², Arg^{12,27}, Nle¹⁴, *β*-Ala²⁹) Exendin-4(1-39)NH₂; (SEQ ID NO:81)
(Aib², Arg^{12,27}, Leu¹⁴, *β*-Ala²⁹) Exendin-4(1-39)NH₂; (SEQ ID NO:81)
(Aib², Arg^{12,27}, A6c¹⁴, *β*-Ala²⁹) Exendin-4(1-39)NH₂; (SEQ ID NO:81)
(Aib², Arg^{12,27}, Nle¹⁴, Phe²⁵, *β*-Ala²⁹) Exendin-4(1-39)NH₂; (SEQ ID NO:84)
(Aib², Arg^{12,27}, Leu¹⁴, Phe²⁵, *β*-Ala²⁹) Exendin-4(1-39)NH₂; (SEQ ID NO:84)
(Aib², Arg^{12,27}, A6c¹⁴, Phe²⁵, *β*-Ala²⁹) Exendin-4(1-39)NH₂; (SEQ ID NO:84)
(Aib², Nle¹⁴, Phe²⁵, *β*-Ala²⁹) Exendin-4(1-39)NH₂; (SEQ ID NO:85)
(Aib², Leu¹⁴, Phe²⁵, *β*-Ala²⁹) Exendin-4(1-39)NH₂; (SEQ ID NO:85)
(Aib², A6c¹⁴, Phe²⁵, *β*-Ala²⁹) Exendin-4(1-39)NH₂; (SEQ ID NO:85)
(Aib², Arg^{12,27,30}, Nle¹⁴, *β*-Ala²⁹, Acp³¹) Exendin-4(1-31)NH₂; (SEQ ID NO:86)
(Aib², Nle¹⁴, *β*-Ala²⁹, Arg³⁰, Acp³¹) Exendin-4(1-31)NH₂; (SEQ ID NO:87)
(Aib², Arg^{12,27}, Nle¹⁴, *β*-Ala²⁹, Acp⁴⁰) Exendin-4(1-40)NH₂; (SEQ ID NO:88)
(Aib², Nle¹⁴, *β*-Ala²⁹, Acp⁴⁰) Exendin-4(1-40)NH₂; (SEQ ID NO:89)
(Aib², Lys¹²(N^{ε}-Acp-octanoyl), Nle¹⁴, *β*-Ala²⁹) Exendin-4(1-39)NH₂; (SEQ ID NO:90)
(Aib², Lys¹²(N^{ε}-Acp-octanoyl), Nle¹⁴, Arg²⁷, *β*-Ala²⁹) Exendin-4(1-39)NH₂; (SEQ ID NO:90)
(Aib², Nle¹⁴, Lys²⁷(N^{ε}-Acp-octanoyl), *β*-Ala²⁹) Exendin-4(1-39)NH₂; (SEQ ID NO:91)
(Aib², Arg¹², Nle¹⁴, Lys²⁷(N^{ε}-Acp-octanoyl), *β*-Ala²⁹) Exendin-4(1-39)NH₂; (SEQ ID NO:91)
(Aib², Arg^{12,27}, Nle¹⁴, *β*-Ala²⁹, Acp⁴⁰) Exendin-4(1-40)NH₂; (SEQ ID NO:88) or
(Aib², Nle¹⁴, Lys²⁷(N^{ε}-Acp-decanoyl), *β*-Ala²⁹) Exendin-4(1-39)NH₂; (SEQ ID NO:92)
or a pharmaceutically acceptable salt thereof.

4. A compound of claim 1, wherein said compound is:
(Aib², Nle¹⁴, *β*-Ala²⁹) Exendin-4(1-39)NH₂; (SEQ ID NO:9)
(Aib², Nle¹⁴) Exendin-4(1-39)NH₂; (SEQ ID NO:5)
(Aib², Leu¹⁴) Exendin-4(1-39)NH₂; (SEQ ID NO:5)
(Aib², A6c¹⁴) Exendin-4(1-39)NH₂; (SEQ ID NO:5)
(Aib², Arg^{12,27}, Nle¹⁴) Exendin-4(1-39)NH₂; (SEQ ID NO:6)
(Aib², Arg^{12,27}, Leu¹⁴) Exendin-4(1-39)NH₂; (SEQ ID NO:6)
(Aib^{2,28}, Nle¹⁴) Exendin-4(1-39)NH₂; (SEQ ID NO:7)
(Aib^{2,28}, Leu¹⁴) Exendin-4(1-39)NH₂; (SEQ ID NO:7)
(Aib^{2,29}, Nle¹⁴) Exendin-4(1-39)NH₂; (SEQ ID NO:8)
(Aib^{2,29}, Leu¹⁴) Exendin-4(1-39)NH₂; (SEQ ID NO:8)
(Aib², Leu¹⁴, *β*-Ala²⁹) Exendin-4(1-39)NH₂; (SEQ ID NO:9)
(Aib², Nle¹⁴, A6c²⁶) Exendin-4(1-39)NH₂; (SEQ ID NO:10)
(Aib², Leu¹⁴, A6c²⁶) Exendin-4(1-39)NH₂; (SEQ ID NO:10)
(Aib², A6c¹⁴) Exendin-4(1-39)NH₂; (SEQ ID NO:5)
(Aib², Nle¹⁴, Ser³⁹(O-decanoyl)) Exendin-4(1-39)NH₂; (SEQ ID NO:19)
(Aib², Leu¹⁴, Ser³⁹(O-decanoyl)) Exendin-4(1-39)NH₂; (SEQ ID NO:19)
(Aib², Nle¹⁴, Lys²⁷(N^{ε}-octanoyl)) Exendin-4(1-39)NH₂; (SEQ ID NO:20)
(Aib², Leu¹⁴, Lys²⁷(N^{ε}-octanoyl)) Exendin-4(1-39)NH₂; (SEQ ID NO:20)
(Aib², Arg¹², Nle¹⁴, Lys²⁷(N^{ε}-octanoyl)) Exendin-4(1-39)NH₂; (SEQ ID NO:21)
(Aib², Arg¹², Leu¹⁴, Lys²⁷(N^{ε}-octanoyl)) Exendin-4(1-39)NH₂; (SEQ ID NO:21)
(Aib², Lys¹² (N^{ε}-octanoyl), Nle¹⁴, Arg²⁷) Exendin-4(1-39)NH₂; (SEQ ID NO:22)
(Aib², Arg^{12,27}, Nle¹⁴) Exendin-3(1-39)NH₂; (SEQ ID NO:26) (SEQ ID NO:26)
(Aib², Arg^{12,27}, Leu¹⁴) Exendin-3(1-39)NH₂; (SEQ ID NO:26) (SEQ ID NO:26)
(Aib², Arg¹², Nle¹⁴, Lys²⁷(N^{ε}-octanoyl)) Exendin-3(1-39)NH₂; (SEQ ID NO:42)
(Aib², Nle¹⁴, *β*-Ala²⁹, Arg³⁰) Exendin-4(1-30)NH₂; (SEQ ID NO:45)
(Aib², Leu¹⁴, *β*-Ala²⁹, Arg³⁰) Exendin-4(1-30)NH₂; (SEQ ID NO:45)
(Aib^{2,29}, Nle¹⁴, Arg³⁰) Exendin-4(1-30)NH₂; (SEQ ID NO:46)
(Aib^{2,29}, Leu¹⁴, Arg³⁰) Exendin-4(1-30)NH₂; (SEQ ID NO:46)
(Aib², Arg^{12,27,30}, Nle¹⁴, *β*-Ala²⁹) Exendin-4(1-30)NH₂; (SEQ ID NO:49)
(Aib², Arg^{12,27,30}, Leu¹⁴, *β*-Ala²⁹) Exendin-4(1-30)NHz; (SEQ ID NO:49)
(Aib^{2,29}, Arg^{12,27,30}, Nle¹⁴) Exendin-4(1-30)NH₂; (SEQ ID NO:50)
(Aib^{2,29}, Arg^{12,27,30}, Leu¹⁴) Exendin-4(1-30)NHz; (SEQ ID NO:50)
(Aib², Nle¹⁴, *β*-Ala²⁹) Exendin-4(1-30)NH₂; (SEQ ID NO:53)
(Aib², Leu¹⁴, *β*-Ala²⁹) Exendin-4(1-30)NH₂; (SEQ ID NO:53)
(Aib^{2,29}, Nle¹⁴) Exendin-4(1-30)NH₂; (SEQ ID NO:54)
(Aib², Nle¹⁴, Ser³¹(O-decanoyl)) Exendin-4(1-31)NH₂; (SEQ ID NO:57)
(Aib², Arg¹², Nle¹⁴, Lys²⁷(N^{ε}-octanoyl)) Exendin-4(1-30)NH₂; (SEQ ID NO:60)
(Aib², Arg¹², Leu¹⁴, Lys²⁷(N^{ε}-octanoyl)) Exendin-4(1-30)NHz; (SEQ ID NO:60)
(Aib², Nle¹⁴, *β*-Ala²⁹, Arg³⁰) Exendin-3(1-30)NH₂; (SEQ ID NO:63)
(Aib², Leu¹⁴, *β*-Ala²⁹, Arg³⁰) Exendin-3(1-30)NH₂; (SEQ ID NO:63)
(Aib^{2,29}, Nle¹⁴, Arg³⁰) Exendin-3(1-30)NH₂; (SEQ ID NO:64)
(Aib², Arg^{12,27}, Nle¹⁴, *β*-Ala²⁹) Exendin-4(1-39)NH₂; (SEQ ID NO:81)
(Aib², Arg^{12,27}, Leu¹⁴, *β*-Ala²⁹) Exendin-4(1-39)NHz; (SEQ ID NO:81)
(Aib², Arg^{12,27}, Nle¹⁴, Phe²⁵, *β*-Ala²⁹) Exendin-4(1-39)NH₂; (SEQ ID NO:84)
(Aib², Arg^{12,27}, Leu¹⁴, Phe²⁵, *ß*-Ala²⁹) Exendin-4(1-39)NHz; (SEQ ID NO:84)
(Aib², Nle¹⁴, Phe²⁵, *β*-Ala²⁹) Exendin-4(1-39)NH₂; (SEQ ID NO:85)
(Aib², Leu¹⁴, Phe²⁵, *β*-Ala²⁹) Exendin-4(1-39)NH₂; (SEQ ID NO:85)
(Aib², Arg^{12,27,30}, Nle¹⁴, *β*-Ala²⁹, Acp³¹) Exendin-4(1-31)NH₂; (SEQ ID NO:86)
(Aib², Nle¹⁴, *β*-Ala²⁹, Arg³⁰, Acp³¹) Exendin-4(1-31)NH₂; (SEQ ID NO:87)
(Aib², Arg^{12,27}, Nle¹⁴, *β*-Ala²⁹, Acp⁴⁰) Exendin-4(1-40)NH₂; (SEQ ID NO:88)
(Aib², Nle¹⁴, *β*-Ala²⁹, Acp⁴⁰) Exendin-4(1-40)NH₂; (SEQ ID NO:89)
(Aib², Nle¹⁴, Lys²⁷(N^{ε}-Acp-octanoyl), *β*-Ala²⁹) Exendin-4(1-39)NH₂; (SEQ ID NO:91)
(Aib², Arg¹², Nle¹⁴, Lys²⁷(N^{ε}-Acp-octanoyl), *β*-Ala²⁹) Exendin-4(1-39)NHz; (SEQ ID NO:91)
(Aib², Arg^{12,27}, Nle¹⁴, *β*-Ala²⁹, Acp⁴⁰) Exendin-4(1-40)NH₂; (SEQ ID NO:88) or
(Aib², Nle¹⁴, Lys²⁷(N^{ε}-Acp-decanoyl), *β*-Ala²⁹) Exendin-4(1-39)NH₂; (SEQ ID NO:92)
or a pharmaceutically acceptable salt thereof.

5. A compound according to claim 4, wherein said compound is
(Aib², Nle¹⁴, *β*-Ala²⁹) Exendin-4(1-39)NH₂; (SEQ ID NO:9)
(Aib², Nle¹⁴) Exendin-4(1-39)NH₂; (SEQ ID NO:5)
(Aib², Leu¹⁴) Exendin-4(1-39)NHz; (SEQ ID NO:5)
(Aib², A6c¹⁴) Exendin-4(1-39)NH₂; (SEQ ID NO:5)
(Aib², Arg^{12,27}, Nle¹⁴) Exendin-4(1-39)NH₂; (SEQ ID NO:6)
(Aib², Arg^{12,27}, Leu¹⁴) Exendin-4(1-39)NH₂; (SEQ ID NO:6)
(Aib^{2,28}, Nle¹⁴) Exendin-4(1-39)NH₂; (SEQ ID NO:7)
(Aib^{2,29}, Nle¹⁴) Exendin-4(1-39)NH₂; (SEQ ID NO:8)
(Aib², Leu¹⁴, *β*-Ala²⁹) Exendin-4(1-39)NH₂; (SEQ ID NO:9)
(Aib², Nle¹⁴, A6c²⁶) Exendin-4(1-39)NHz; (SEQ ID NO:10)
(Aib², Nle¹⁴, Ser³⁹(O-decanoyl)) Exendin-4(1-39)NHz; (SEQ ID NO:19)
(Aib², Nle¹⁴, Lys²⁷(N^{ε}-octanoyl)) Exendin-4(1-39)NH₂; (SEQ ID NO:20)
(Aib², Arg¹², Nle¹⁴, Lys²⁷(N^{ε}-octanoyl)) Exendin-4(1-39)NH₂; (SEQ ID NO:21)
(Aib², Nle¹⁴, *β*-Ala²⁹, Arg³⁰) Exendin-4(1-30)NHz; (SEQ ID NO:45)
(Aib², Leu¹⁴, *β*-Ala²⁹, Arg³⁰) Exendin-4(1-30)NH₂; (SEQ ID NO:45)
(Aib^{2,29}, Nle¹⁴, Arg³⁰) Exendin-4(1-30)NH₂; (SEQ ID NO:46)
(Aib², Arg^{12,27,30}, Nle¹⁴, *β*-Ala²⁹) Exendin-4(1-30)NH₂; (SEQ ID NO:49)
(Aib², Arg^{12,27,30}, Leu¹⁴, *β-*Ala²⁹) Exendin-4(1-30)NH₂; (SEQ ID NO:49)
(Aib², Nle¹⁴, Ser³¹(O-decanoyl)) Exendin-4(1-31)NH₂; (SEQ ID NO:57)
(Aib², Arg¹², Nle¹⁴, Lys²⁷(N^{ε}-octanoyl)) Exendin-4(1-30)NH₂; (SEQ ID NO:60)
(Aib², Arg^{12,27}, Nle¹⁴, *β*-Ala²⁹) Exendin-4(1-39)NH₂; (SEQ ID NO:81)
(Aib², Arg^{12,27}, Leu¹⁴, *β*-Ala²⁹) Exendin-4(1-39)NHz; (SEQ ID NO:81)
(Aib², Arg^{12,27}, Nle¹⁴, Phe²⁵, *β*-Ala²⁹) Exendin-4(1-39)NH₂; (SEQ ID NO:84)
(Aib², Arg^{12,27}, Leu¹⁴, Phe²⁵, *β*-Ala²⁹) Exendin-4(1-39)NH₂; (SEQ ID NO:84)
(Aib², Arg^{12,27,30}, Nle¹⁴, *β*-Ala²⁹, Acp³¹) Exendin-4(1-31)NH₂; (SEQ ID NO:86)
(Aib², Arg^{12,27}, Nle¹⁴, *β*-Ala²⁹, Acp⁴⁰) Exendin-4(1-40)NH₂; (SEQ ID NO:88)
(Aib², Arg¹², Nle¹⁴, Lys²⁷(N-Acp-octanoyl), *β*-Ala²⁹) Exendin-4(1-39)NHz; (SEQ ID NO:91)
(Aib², Arg^{12,27}, Nle¹⁴, *β*-Ala²⁹, Acp⁴⁰) Exendin-4(1-40)NH₂; (SEQ ID NO:88) or
(Aib², Nle¹⁴, Lys²⁷(N^{ε}-Acp-decanoyl), *β*-Ala²⁹) Exendin-4(1-39)NH₂; (SEQ ID NO:92)
or a pharmaceutically acceptable salt thereof.

6. A compound according to claim 5, wherein said compound is
(Aib², Nle¹⁴, *β*-Ala²⁹) Exendin-4(1-39)NHz; (SEQ ID NO:9)
(Aib², Nle¹⁴) Exendin-4(1-39)NH₂; (SEQ ID NO:5)
(Aib², Leu¹⁴) Exendin-4(1-39)NHz; (SEQ ID NO:5)
(Aib^{2,28}, Nle¹⁴) Exendin-4(1-39)NH₂; (SEQ ID NO:7)
(Aib^{2,29}, Nle¹⁴) Exendin-4(1-39)NH₂; (SEQ ID NO:8)
(Aib², Nle¹⁴, A6c²⁶) Exendin-4(1-39)NHz; (SEQ ID NO:10)
(Aib², Nle¹⁴, *β*-Ala²⁹, Arg³⁰) Exendin-4(1-30)NH₂; (SEQ ID NO:45)
(Aib^{2,29}, Nle¹⁴, Arg³⁰) Exendin-4(1-30)NH₂; (SEQ ID NO:46)
(Aib², A6c¹⁴) Exendin-4(1-39)NH₂; (SEQ ID NO:5)
(Aib², Arg^{12,27}, Nle¹⁴, *β*-Ala²⁹, Acp⁴⁰) Exendin-4(1-40)NH₂; (SEQ ID NO:88) or
(Aib², Nle¹⁴, Lys²⁷(N^{ε}-Acp-decanoyl), *β*-Ala²⁹) Exendin-4(1-39)NH₂; (SEQ ID NO:92)
or a pharmaceutically acceptable salt thereof.

7. A compound according to claim 6, wherein said compound is
(Aib², Nle¹⁴, *β*-Ala²⁹)Exendin-4(1-39)-NH₂; (SEQ ID NO:9)
(Aib², A6c¹⁴)Exendin-4(1-39)-NH₂; (SEQ ID NO:5)
(Aib², Nle¹⁴)Exendin-4(1-39)-NH₂; (SEQ ID NO:5)
(Aib², Nle¹⁴, *β*-Ala²⁹, Arg³⁰)Exendin-4(1-30)-NH₂; (SEQ ID NO:45)
(Aib^{2,28}, Nle¹⁴)Exendin-4(1-39)-NH₂; (SEQ ID NO:7)
(Aib², Nle¹⁴, A6c²⁶)Exendin-4(1-39)-NH₂; (SEQ ID NO:10)
(Aib^{2,29}, Nle¹⁴)Exendin-4(1-39)-NH₂; (SEQ ID NO:8)
(Aib², Leu¹⁴)Exendin-4(1-39)-NH₂; (SEQ ID NO:5) or
(Aib^{2,29}, Nle¹⁴, Arg³⁰)Exendin-4(1-30)-NH₂; (SEQ ID NO:46)
or a pharmaceutically-acceptable salt thereof.

8. A compound according to claim 7 which is (Aib², Nle¹⁴, *β-*Ala²⁹)Exendin-4(1-39)-NH₂; (SEQ ID NO:9) or a pharmaceutically acceptable salt thereof.

9. A pharmaceutical composition comprising an effective amount of a compound according to any one of claims 1 to 8 or a pharmaceutically acceptable salt thereof and a pharmaceutically acceptable carrier or diluent.

10. A compound according to any of claims 1 to 8 or a pharmaceutically acceptable salt thereof, or a composition according to claim 9 for use in eliciting a GLP-1 agonist effect in a human or non-human animal subject in need thereof.

11. A compound according to any of claims 1 to 8 or a pharmaceutically acceptable salt thereof, or a composition according to claim 9 for use in treating a disease selected from the group consisting of Type I diabetes, Type II diabetes, obesity, glucagonomas, secretory disorders of the airway, metabolic disorder, arthritis, osteoporosis, central nervous system disease, restenosis and neurodegenerative disease, in a human or non-human animal subject in need thereof.

12. A compound according to any of claims 1 to 8 or a pharmaceutically acceptable salt thereof, or a composition according to claim 9 for use in treating a disease selected from the group consisting of cachexia, anorexia, hypoglycemia and malabsorption syndrome, in a human or non-human animal subject in need thereof.

13. A compound according to any of claims 1 to 8 or a pharmaceutically acceptable salt thereof, or a composition according to claim 9 for use in stimulating insulin release in a human or non-human animal subject in need thereof.

14. A compound according to any of claims 1 to 8 or a pharmaceutically acceptable salt thereof, or a composition according to claim 9 for use in enhancing insulin sensitivity in a human or non-human animal subject in need thereof.

15. A compound according to any of claims 1 to 8 or a pharmaceutically acceptable salt thereof, or a composition according to claim 9 for use in lowering blood glucose levels in a human or non-human animal subject in need thereof.


SEQUENCE LISTING

<110>  IPSEN PHARMA S.A.S.

<120>  Analogues of Exendin-4 and Exendin-3

<130>  P130035EP1

<140>  TBA
<141>  2008.09.05

<150>  US 60/967,855
<151>  2007.09.07

<160>  101

<170>  PatentIn version 3.5

<210>  1
<211>  39
<212>  PRT
<213>  Artificial Sequence

<220>
<223>  Exendin-3

<220>
<221>  MOD_RES
<222>  (39)..(39)
<223>  AMIDATION

<400>  1
His Ser Asp Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  2
<211>  39
<212>  PRT
<213>  Artificial Sequence

<220>
<223>  Exendin-4

<220>
<221>  MOD_RES
<222>  (39)..(39)
<223>  AMIDATION

<400>  2
His Gly Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  3
<211>  31
<212>  PRT

<213>  Artificial Sequence

<220>
<223>  GLP-1

<400>  3
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly Arg Gly
            20                  25                  30

<210>  4
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MOD_RES
<222>  (39)..(39)
<223>  AMIDATION

<400>  4
His Xaa Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  5
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutryic acid (Aib)

<220>
<221>  MISC_FEATURE
<222>  (14)..(14)
<223>  Xaa = norleucine (Nle), Leu, or 1-amino-1-cyclohexanecarboxylic
       acid (A6c)

<220>
<221>  MOD_RES
<222>  (39)..(39)
<223>  AMIDATION

<400>  5
His Xaa Glu Gly Thr Phe Thr Ser Asp Leu Ser Lys Gln Xaa Glu Glu

1                   5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  6
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (39)..(39)
<223>  AMIDATION

<400>  6
His Xaa Glu Gly Thr Phe Thr Ser Asp Leu Ser Arg Gln Xaa Glu Glu
1                   5                   10                  15
Glu Ala Val Arg Leu Phe Ile Glu Trp Leu Arg Asn Gly Gly Pro Ser
            20                  25                  30
Ser Gly Ala Pro Pro Pro Ser
        35

<210>  7
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE
<222>  (28)..(28)
<223>  Xaa = alpha-aminoisobutyric acid (Aib)

<220>
<221>  MOD_RES
<222>  (39)..(39)

<223>  AMIDATION

<400>  7
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Xaa Gly Gly Pro Ser
            20                  25                  30
Ser Gly Ala Pro Pro Pro Ser
        35

<210>  8
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = alpha-aminoisobutyric acid (Aib)

<220>
<221>  MOD_RES
<222>  (39)..(39)
<223>  AMIDATION

<400>  8
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly Pro Ser
            20                  25                  30
Ser Gly Ala Pro Pro Pro Ser
        35

<210>  9
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = beta-alanine (beta-Ala)

<220>
<221>  MOD_RES
<222>  (39)..(39)
<223>  AMIDATION

<400>  9
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly Pro Ser
            20                  25                  30
Ser Gly Ala Pro Pro Pro Ser
        35

<210>  10
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, Met, or
       1-amino-1-cyclohexanecarboxylic acid (A6c)

<220>
<221>  MISC_FEATURE
<222>  (26)..(26)
<223>  Xaa = 1-amino-1-cyclohexanecarboxylic acid (A6c)

<220>
<221>  MOD_RES
<222>  (39)..(39)
<223>  AMIDATION

<400>  10
His Xaa Glu Gly Thr Phe Thr Ser Asp Leu Ser Lys Gln Xaa Glu Glu
1               5                   10                  15
Glu Ala Val Arg Leu Phe Ile Glu Trp Xaa Lys Asn Gly Gly Pro Ser
            20                  25                  30
Ser Gly Ala Pro Pro Pro Ser
        35

<210>  11
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (10)..(10)
<223>  Xaa = 1-amino-1-cyclohexanecarboxylic acid (A6c)

<220>
<221>  MISC_FEATURE
<222>  (14)..(14)
<223>  Xaa = norleucine (Nle), Leu, or 1-amino-1-cyclohexanecarboxylic
      acid (A6c)

<220>
<221>  MOD_RES
<222>  (39)..(39)
<223>  AMIDATION

<400>  11
His Xaa Glu Gly Thr Phe Thr Ser Asp Xaa Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  12
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MISC_FEATURE
<222>  (18)..(18)
<223>  Xaa = alpha-aminoisobutyric acid (Aib)

<220>
<221>  MOD_RES
<222>  (39)..(39)
<223>  AMIDATION

<400>  12
His Xaa Glu Gly Thr Phe Thr Ser Asp Leu Ser Lys Gln Xaa Glu Glu
1               5                   10                  15
Glu Xaa Val Arg Leu Phe Ile Glu Trp Leu Lys Asn Gly Gly Pro Ser
            20                  25                  30

Ser Gly Ala Pro Pro Pro Ser
        35

<210>  13
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (11)..(11)
<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MOD_RES
<222>  (39)..(39)
<223>  AMIDATION

<400>  13
His Xaa Glu Gly Thr Phe Thr Ser Asp Leu Xaa Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  14
<211>  39
<212>  PRT
<213>  Artificial Sequence

<220>
<223>  Analogue of exendin-4

<220>
<221>  MISC_FEATURE
<222>  (2)..(2)
<223>  Xaa = D-alanine (D-Ala)

<220>
<221>  MISC_FEATURE
<222>  (14)..(14)
<223>  Xaa = norleucine (Nle), Leu, or Met

<220>
<221>  MOD_RES
<222>  (39)..(39)
<223>  AMIDATION

<400>  14

His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  15
<211>  39
<212>  PRT
<213>  Artificial Sequence

<220>
<223>  Analogue of exendin-4

<220>
<221>  MISC_FEATURE
<222>  (1)..(1)
<223>  Xaa = 3-(p-hydroxyphenyl)propionic acid (Hppa)

<220>
<221>  MISC_FEATURE
<222>  (14)..(14)
<223>  Xaa = norleucine (Nle), Leu, or Met

<220>
<221>  MOD_RES
<222>  (39)..(39)
<223>  AMIDATION

<400>  15
Xaa Gly Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  16
<211>  39
<212>  PRT
<213>  Artificial Sequence

<220>
<223>  Analogue of exendin-4

<220>
<221>  MISC_FEATURE
<222>  (1)..(1)
<223>  Xaa = urocanic acid (Ura), trans-3-(3-pyridyl) acrylic acid
       (Paa), or (4-pyridylthio) acetic acid (Pta)

<220>
<221>  MISC_FEATURE
<222>  (14)..(14)
<223>  Xaa = norleucine (Nle)

<220>
<221>  MOD_RES
<222>  (39)..(39)
<223>  AMIDATION

<400>  16

Xaa Gly Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  17
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (32)..(32)
<223>  modified with O-decanoyl

<220>
<221>  MOD_RES
<222>  (39)..(39)
<223>  AMIDATION

<400>  17
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  18
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES

<222>  (33)..(33)
<223>  modified with O-decanoyl

<220>
<221>  MOD_RES
<222>  (39)..(39)
<223>  AMIDATION

<400>  18
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  19
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (39)..(39)
<223>  AMIDATION

<220>
<221>  MOD_RES
<222>  (39)..(39)
<223>  modified with O-decanoyl

<400>  19
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  20
<211>  39
<212>  PRT
<213>  Artificial Sequence

<220>
<223>  Analogue of exendin-4

<220>
<221>  MISC_FEATURE
<222>  (2)..(2)

<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (27)..(27)
<223>  modified with N-epsilon-octanoyl

<220>
<221>  MOD_RES
<222>  (39)..(39)
<223>  AMIDATION

<400>  20
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  21
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (27)..(27)
<223>  modified with N-epsilon-octanoyl

<220>
<221>  MOD_RES
<222>  (39)..(39)
<223>  AMIDATION

<400>  21
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  22
<211>  39

<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MOD_RES
<222>  (12)..(12)
<223>  modified with N-epsilon-octanoyl

<220>
<221>  MISC_FEATURE
<222>  (14)..(14)
<223>  Xaa = norleucine (Nle) or Leu

<220>
<221>  MOD_RES
<222>  (39)..(39)
<223>  AMIDATION

<400>  22
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Arg Asn Gly Gly Pro Ser
            20                  25                  30
Ser Gly Ala Pro Pro Pro Ser
        35

<210>  23
<211>  42
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (40)..(40)
<223>  Xaa = D-Asp or Asp

<220>
<221>  MISC_FEATURE
<222>  (41)..(41)
<223>  Xaa = 5-aminovaleric acid (Ava)

<220>

<221>  MOD_RES
<222>  (42)..(42)
<223>  AMIDATION

<220>
<221>  MISC_FEATURE
<222>  (42)..(42)
<223>  Xaa = 11-aminoundecanoic acid (Aun)

<400>  23
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Arg Asn Gly Gly Pro Ser
            20                  25                  30
Ser Gly Ala Pro Pro Pro Ser Xaa Xaa Xaa
        35                  40

<210>  24
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MOD_RES
<222>  (39)..(39)
<223>  AMIDATION

<400>  24
His Xaa Asp Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  25
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, or 1-amino-1-cyclohexanecarboxylic
       acid (A6c)

<220>

<221>  MOD_RES
<222>  (39)..(39)
<223>  AMIDATION

<400>  25
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  26
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (39)..(39)
<223>  AMIDATION

<400>  26
His Xaa Asp Gly Thr Phe Thr Ser Asp Leu Ser Arg Gln Xaa Glu Glu
1               5                   10                  15
Glu Ala Val Arg Leu Phe Ile Glu Trp Leu Arg Asn Gly Gly Pro Ser
            20                  25                  30
Ser Gly Ala Pro Pro Pro Ser
        35

<210>  27
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE

<222>  (28)..(28)
<223>  Xaa = alpha-aminoisobutyric acid (Aib)

<220>
<221>  MOD_RES
<222>  (39)..(39)
<223>  AMIDATION

<400>  27
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Xaa Gly Gly Pro Ser
            20                  25                  30
Ser Gly Ala Pro Pro Pro Ser
        35

<210>  28
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = alpha-aminoisobutyric acid (Aib)

<220>
<221>  MOD_RES
<222>  (39)..(39)
<223>  AMIDATION

<400>  28
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly Pro Ser
            20                  25                  30
Ser Gly Ala Pro Pro Pro Ser
        35

<210>  29
<211>  39
<212>  PRT
<213>  Artificial Sequence

<220>
<223>  Analogue of exendin-3

<220>
<221>  MISC_FEATURE
<222>  (2)..(2)

<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = beta Ala

<220>
<221>  MOD_RES
<222>  (39)..(39)
<223>  AMIDATION

<400>  29
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly Pro Ser
            20                  25                  30
Ser Gly Ala Pro Pro Pro Ser
        35

<210>  30
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, Met, or
       1-amino-1-cyclohexanecarboxylic acid (A6c)

<220>
<221>  MISC_FEATURE
<222>  (26)..(26)
<223>  Xaa = 1-amino-1-cyclohexanecarboxylic acid (A6c)

<220>
<221>  MOD_RES
<222>  (39)..(39)
<223>  AMIDATION

<400>  30
His Xaa Asp Gly Thr Phe Thr Ser Asp Leu Ser Lys Gln Xaa Glu Glu
1               5                   10                  15
Glu Ala Val Arg Leu Phe Ile Glu Trp Xaa Lys Asn Gly Gly Pro Ser
            20                  25                  30
Ser Gly Ala Pro Pro Pro Ser
        35

<210>  31

<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (10)..(10)
<223>  Xaa = 1-amino-1-cyclohexanecarboxylic acid (A6c)

<220>
<221>  MISC_FEATURE
<222>  (14)..(14)
<223>  Xaa = norleucine (Nle), Leu, or 1-amino-1-cyclohexanecarboxylic
       acid (A6c)

<220>
<221>  MOD_RES
<222>  (39)..(39)
<223>  AMIDATION

<400>  31
His Xaa Asp Gly Thr Phe Thr Ser Asp Xaa Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  32
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MISC_FEATURE
<222>  (18)..(18)
<223>  Xaa = alpha-aminoisobutyric acid (Aib)

<220>
<221>  MOD_RES
<222>  (39)..(39)
<223>  AMIDATION

<400>  32
His Xaa Asp Gly Thr Phe Thr Ser Asp Leu Ser Lys Gln Xaa Glu Glu
1               5                   10                  15
Glu Xaa Val Arg Leu Phe Ile Glu Trp Leu Lys Asn Gly Gly Pro Ser
            20                  25                  30
Ser Gly Ala Pro Pro Pro Ser
        35

<210>  33
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (11)..(11)
<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MOD_RES
<222>  (39)..(39)
<223>  AMIDATION

<400>  33
His Xaa Asp Gly Thr Phe Thr Ser Asp Leu Xaa Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  34
<211>  39
<212>  PRT
<213>  Artificial Sequence

<220>
<223>  Analogue of exendin-3

<220>
<221>  MISC_FEATURE
<222>  (2)..(2)
<223>  Xaa = D-Ala

<220>
<221>  MISC_FEATURE
<222>  (14)..(14)
<223>  Xaa = norleucine (Nle), Leu, or Met

<220>
<221>  MOD_RES
<222>  (39)..(39)
<223>  AMIDATION

<400>  34
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  35
<211>  39
<212>  PRT
<213>  Artificial Sequence

<220>
<223>  Analogue of exendin-3

<220>
<221>  MISC_FEATURE
<222>  (1)..(1)
<223>  Xaa = 3-(p-hydroxyphenyl)propionic acid (Hppa)

<220>
<221>  MISC_FEATURE
<222>  (14)..(14)
<223>  Xaa = norleucine (Nle), Leu, or Met

<220>
<221>  MOD_RES
<222>  (39)..(39)
<223>  AMIDATION

<400>  35
Xaa Ser Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  36
<211>  39
<212>  PRT
<213>  Artificial Sequence

<220>
<223>  Analogue of exendin-3

<220>
<221>  MISC_FEATURE
<222>  (1)..(1)
<223>  Xaa = urocanic acid (Ura), trans-3-(3-pyridyl) acrylic acid
       (Paa), or (4-pyridylthio) acetic acid (Pta)

<220>
<221>  MISC_FEATURE
<222>  (14)..(14)
<223>  Xaa = norleucine (Nle)

<220>
<221>  MOD_RES
<222>  (39)..(39)
<223>  AMIDATION

<400>  36
Xaa Ser Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  37
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (32)..(32)
<223>  modified with O-decanoyl

<220>
<221>  MOD_RES
<222>  (39)..(39)
<223>  AMIDATION

<400>  37
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  38
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>

<221>  MISC_FEATURE
<222>  (14)..(14)
<223>  Xaa = norleucine (Nle) or Leu

<220>
<221>  MOD_RES
<222>  (33)..(33)
<223>  modified with O-decanoyl

<220>
<221>  MOD_RES
<222>  (39)..(39)
<223>  AMIDATION

<400>  38
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  39
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (39)..(39)
<223>  AMIDATION

<220>
<221>  MOD_RES
<222>  (39)..(39)
<223>  modified with O-decanoyl

<400>  39
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  40
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (17)..(17)
<223>  modified with O-decanoyl

<220>
<221>  MOD_RES
<222>  (39)..(39)
<223>  AMIDATION

<400>  40
His Xaa Asp Gly Thr Phe Thr Ser Asp Leu Ser Lys Gln Xaa Glu Glu
1               5                   10                  15
Ser Ala Val Arg Leu Phe Ile Glu Trp Leu Lys Asn Gly Gly Pro Ser
            20                  25                  30
Ser Gly Ala Pro Pro Pro Ser
        35

<210>  41
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (27)..(27)
<223>  modified with N-epsilon-octanoyl

<220>
<221>  MOD_RES
<222>  (39)..(39)
<223>  AMIDATION

<400>  41
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Pro Ser

20                  25                  30
Ser Gly Ala Pro Pro Pro Ser
        35

<210>  42
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (27)..(27)
<223>  modified with N-epsilon-octanoyl

<220>
<221>  MOD_RES
<222>  (39)..(39)
<223>  AMIDATION

<400>  42
His Xaa Asp Gly Thr Phe Thr Ser Asp Leu Ser Arg Gln Xaa Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  43
<211>  39
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MOD_RES
<222>  (12)..(12)
<223>  modified with N-epsilon-octanoyl

<220>
<221>  MISC_FEATURE
<222>  (14)..(14)
<223>  Xaa = norleucine (Nle) or Leu

<220>
<221>  MOD_RES
<222>  (39)..(39)
<223>  AMIDATION

<400>  43
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Arg Asn Gly Gly Pro Ser
            20                  25                  30
Ser Gly Ala Pro Pro Pro Ser
        35

<210>  44
<211>  42
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (40)..(40)
<223>  Xaa = Asp or D-Asp

<220>
<221>  MISC_FEATURE
<222>  (41)..(41)
<223>  Xaa = 5-aminovaleric acid (Ava)

<220>
<221>  MISC_FEATURE
<222>  (42)..(42)
<223>  Xaa = 11-aminoundecanoic acid (Aun)

<220>
<221>  MOD_RES
<222>  (42)..(42)
<223>  AMIDATION

<400>  44
His Xaa Asp Gly Thr Phe Thr Ser Asp Leu Ser Arg Gln Xaa Glu Glu
1               5                   10                  15
Glu Ala Val Arg Leu Phe Ile Glu Trp Leu Arg Asn Gly Gly Pro Ser
            20                  25                  30
Ser Gly Ala Pro Pro Pro Ser Xaa Xaa Xaa
        35                  40

<210>  45
<211>  30
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, Met or
       1-amino-1-cyclohexanecarboxylic acid (A6c)

<220>
<221>  MISC_FEATURE
<222>  (29)..(29)
<223>  Xaa = beta-Ala

<220>
<221>  MOD_RES
<222>  (30)..(30)
<223>  AMIDATION

<400>  45
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Arg
            20                  25                  30

<210>  46
<211>  30
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, Met or
       1-amino-1-cyclohexanecarboxylic acid (A6c)

<220>
<221>  MISC_FEATURE
<222>  (29)..(29)
<223>  Xaa = alpha-aminoisobutyric acid (Aib)

<220>
<221>  MOD_RES
<222>  (30)..(30)
<223>  AMIDATION

<400>  46
His Xaa Glu Gly Thr Phe Thr Ser Asp Leu Ser Lys Gln Xaa Glu Glu
1               5                   10                  15

Glu Ala Val Arg Leu Phe Ile Glu Trp Leu Lys Asn Xaa Arg
            20                  25                  30

<210>  47
<211>  30
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE
<222>  (29)..(29)
<223>  Xaa = beta-Ala

<220>
<221>  MOD_RES
<222>  (30)..(30)
<223>  AMIDATION

<220>
<221>  MISC_FEATURE
<222>  (30)..(30)
<223>  Xaa = D-Arg

<400>  47
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Xaa
            20                  25                  30

<210>  48
<211>  30
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE
<222>  (29)..(29)

<223>  Xaa = alpha-aminoisobutyric acid (Aib)

<220>
<221>  MOD_RES
<222>  (30)..(30)
<223>  AMIDATION

<220>
<221>  MISC_FEATURE
<222>  (30)..(30)
<223>  Xaa = D-Arg

<400>  48
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Xaa
            20                  25                  30

<210>  49
<211>  30
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE
<222>  (29)..(29)
<223>  Xaa = beta-Ala

<220>
<221>  MOD_RES
<222>  (30)..(30)
<223>  AMIDATION

<400>  49
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Arg Asn Xaa Arg
            20                  25                  30

<210>  50
<211>  30
<212>  PRT
<213>  Artificial Sequence

<220>
<223>  Analogue of exendin-4

<220>
<221>  MISC_FEATURE
<222>  (2)..(2)

<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE
<222>  (29)..(29)
<223>  Xaa = alpha-aminoisobutyric acid (Aib)

<220>
<221>  MOD_RES
<222>  (30)..(30)
<223>  AMIDATION

<400>  50
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Arg Asn Xaa Arg
            20                  25                  30

<210>  51
<211>  30
<212>  PRT
<213>  Artificial Sequence

<220>
<223>  Analogue of exendin-4

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = beta-Ala

<220>
<221>  MOD_RES
<222>  (30)..(30)
<223>  AMIDATION

<400>  51
His Gly Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Arg
            20                  25                  30

<210>  52
<211>  30
<212>  PRT
<213>  Artificial Sequence

<220>
<223>  Analogue of exendin-4

<220>
<221>  MISC_FEATURE
<222>  (14)..(14)

<223>  Xaa = norleucine (Nle), Leu, or Met

<220>
<221>  MISC_FEATURE
<222>  (29)..(29)
<223>  Xaa = alpha-aminoisobutyric acid (Aib)

<220>
<221>  MOD_RES
<222>  (30)..(30)
<223>  AMIDATION

<400>  52
His Gly Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Arg
            20                  25                  30

<210>  53
<211>  30
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE
<222>  (29)..(29)
<223>  Xaa = beta-alanine (beta-Ala)

<220>
<221>  MOD_RES
<222>  (30)..(30)
<223>  AMIDATION

<400>  53
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly
            20                  25                  30

<210>  54
<211>  30
<212>  PRT
<213>  Artificial Sequence

<220>
<223>  Analogue of exendin-4

<220>
<221>  MISC_FEATURE
<222>  (2)..(2)

<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = alpha-aminoisobutyric acid (Aib)

<220>
<221>  MOD_RES
<222>  (30)..(30)
<223>  AMIDATION

<400>  54
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly
            20                  25                  30

<210>  55
<211>  30
<212>  PRT
<213>  Artificial Sequence

<220>
<223>  Analogue of exendin-4

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = beta-Ala

<220>
<221>  MOD_RES
<222>  (30)..(30)
<223>  AMIDATION

<400>  55
His Gly Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly
            20                  25                  30

<210>  56
<211>  30
<212>  PRT
<213>  Artificial Sequence

<220>
<223>  Analogue of exendin-4

<220>
<221>  MISC_FEATURE
<222>  (14)..(14)

<223>  Xaa = norleucine (Nle), Leu, or Met

<220>
<221>  MISC_FEATURE
<222>  (29)..(29)
<223>  Xaa = alpha-aminoisobutyric acid (Aib)

<220>
<221>  MOD_RES
<222>  (30)..(30)
<223>  AMIDATION

<400>  56
His Gly Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly
            20                  25                  30

<210>  57
<211>  31
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (31)..(31)
<223>  AMIDATION

<220>
<221>  MOD_RES
<222>  (31)..(31)
<223>  modified with O-decanoyl

<400>  57
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Ser
            20                  25                  30

<210>  58
<211>  30
<212>  PRT
<213>  Artificial Sequence

<220>
<223>  Analogue of exendin-4

<220>
<221>  MISC_FEATURE
<222>  (2)..(2)

<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (17)..(17)
<223>  modified with O-decanoyl

<220>
<221>  MOD_RES
<222>  (30)..(30)
<223>  AMIDATION

<400>  58
His Xaa Glu Gly Thr Phe Thr Ser Asp Leu Ser Lys Gln Xaa Glu Glu
1               5                   10                  15
Ser Ala Val Arg Leu Phe Ile Glu Trp Leu Lys Asn Gly Gly
            20                  25                  30

<210>  59
<211>  30
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (27)..(27)
<223>  modified with N-epsilon-octanoyl

<220>
<221>  MOD_RES
<222>  (30)..(30)
<223>  AMIDATION

<400>  59
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly
            20                  25                  30

<210>  60
<211>  30
<212>  PRT
<213>  Artificial Sequence

<220>

<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (27)..(27)
<223>  modified with N-epsilon-octanoyl

<220>
<221>  MOD_RES
<222>  (30)..(30)
<223>  AMIDATION

<400>  60
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Lys Asn Gly Gly
            20                  25                  30

<210>  61
<211>  30
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MOD_RES
<222>  (12)..(12)
<223>  modified with N-epsilon-octanoyl

<220>
<221>  MISC_FEATURE
<222>  (14)..(14)
<223>  Xaa = norleucine (Nle) or Leu

<220>
<221>  MOD_RES
<222>  (30)..(30)
<223>  AMIDATION

<400>  61
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Arg Asn Gly Gly
            20                  25                  30

<210>  62

<211>  33
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (31)..(31)
<223>  Xaa = Asp or D-Asp

<220>
<221>  MISC_FEATURE
<222>  (32)..(32)
<223>  Xaa = 5-aminovaleric acid (Ava)

<220>
<221>  MISC_FEATURE
<222>  (33)..(33)
<223>  Xaa = 11-aminoundecanoic acid (Aun)

<220>
<221>  MOD_RES
<222>  (33)..(33)
<223>  AMIDATION

<400>  62
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Arg Asn Gly Arg Xaa Xaa
            20                  25                  30
Xaa

<210>  63
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, 1-amino-1-cyclohexanecarboxylic acid
       (A6c), or Met

<220>
<221>  MISC_FEATURE
<222>  (29)..(29)
<223>  Xaa = beta-Ala

<220>
<221>  MOD_RES
<222>  (30)..(30)
<223>  AMIDATION

<400>  63
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Arg
            20                  25                  30

<210>  64
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, 1-amino-1-cyclohexanecarboxylic acid
       (A6c), or Met

<220>
<221>  MISC_FEATURE
<222>  (29)..(29)
<223>  Xaa = alpha-aminoisobutyric acid (Aib)

<220>
<221>  MOD_RES
<222>  (30)..(30)
<223>  AMIDATION

<400>  64
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Arg
            20                  25                  30

<210>  65
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221> MISC_FEATURE
<222> (14)..(14)
<223> Xaa = norleucine (Nle) or Leu

<220>
<221> MISC_FEATURE
<222> (29)..(29)
<223> Xaa =beta-Ala

<220>
<221> MOD_RES
<222> (30)..(30)
<223> AMIDATION

<220>
<221> MISC_FEATURE
<222> (30)..(30)
<223> Xaa = D-Arg

<400> 65
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Xaa
            20                  25                  30

<210> 66
<211> 30
<212> PRT
<213> Artificial Sequence

<220>
<223> Analogue of exendin-3

<220>
<221> MISC_FEATURE
<222> (2)..(2)
<223> Xaa = alpha-aminoisobutyric acid (Aib)

<220>
<221> MISC_FEATURE
<222> (14)..(14)
<223> Xaa = norleucine (Nle) or Leu

<220>
<221> MISC_FEATURE
<222> (29)..(29)
<223> Xaa = alpha-aminoisobutyric acid (Aib)

<220>
<221> MOD_RES
<222> (30)..(30)
<223> AMIDATION

<220>
<221> MISC_FEATURE
<222> (30)..(30)
<223> Xaa = D-Arg

<400> 66
His Xaa Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Xaa Xaa
            20                  25                  30

<210>  67
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE
<222>  (29)..(29)
<223>  Xaa = beta-Ala

<220>
<221>  MOD_RES
<222>  (30)..(30)
<223>  AMIDATION

<400>  67
His Xaa Asp Gly Thr Phe Thr Ser Asp Leu Ser Arg Gln Xaa Glu Glu
1               5                   10                  15
Glu Ala Val Arg Leu Phe Ile Glu Trp Leu Arg Asn Xaa Arg
            20                  25                  30

<210>  68
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE
<222>  (29)..(29)
<223>  Xaa = alpha-aminoisobutyric acid (Aib)

<220>
<221>  MOD_RES
<222>  (30)..(30)

<223>  AMIDATION

<400>  68
His Xaa Asp Gly Thr Phe Thr Ser Asp Leu Ser Arg Gln Xaa Glu Glu
1               5                   10                  15
Glu Ala Val Arg Leu Phe Ile Glu Trp Leu Arg Asn Xaa Arg
            20                  25                  30

<210>  69
<211>  30
<212>  PRT
<213>  Artificial Sequence

<220>
<223>  Analogue of exendin-3

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = beta-Ala

<220>
<221>  MOD_RES
<222>  (30)..(30)
<223>  AMIDATION

<400>  69
His Ser Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Arg
            20                  25                  30

<210>  70
<211>  30
<212>  PRT
<213>  Artificial Sequence

<220>
<223>  Analogue of exendin-3

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = alpha-aminoisobutyric acid (Aib)

<220>
<221>  MOD_RES
<222>  (30)..(30)
<223>  AMIDATION

<400>  70
His Ser Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Xaa Arg
            20                  25                  30

<210>  71
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MISC_FEATURE
<222>  (29)..(29)
<223>  Xaa = beta-Ala

<220>
<221>  MOD_RES
<222>  (30)..(30)
<223>  AMIDATION

<400>  71
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly
            20                  25                  30

<210>  72
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = alpha-aminoisobutyric acid (Aib)

<220>
<221>  MOD_RES
<222>  (30)..(30)

<223>  AMIDATION

<400>  72
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly
            20                  25                  30

<210>  73
<211>  30
<212>  PRT
<213>  Artificial Sequence

<220>
<223>  Analogue of exendin-3

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = beta-Ala

<220>
<221>  MOD_RES
<222>  (30)..(30)
<223>  AMIDATION

<400>  73
His Ser Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly
            20                  25                  30

<210>  74
<211>  30
<212>  PRT
<213>  Artificial Sequence

<220>
<223>  Analogue of exendin-3

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = alpha-aminoisobutyric acid (Aib)

<220>
<221>  MOD_RES
<222>  (30)..(30)
<223>  AMIDATION

<400>  74
His Ser Asp Gly Thr Phe Thr Ser Asp Leu Ser Lys Gln Xaa Glu Glu
1               5                   10                  15

Glu Ala Val Arg Leu Phe Ile Glu Trp Leu Lys Asn Xaa Gly
            20                  25                  30

<210>  75
<211>  31
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (31)..(31)
<223>  AMIDATION

<220>
<221>  MOD_RES
<222>  (31)..(31)
<223>  modified with O-decanoyl

<400>  75
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly Ser
            20                  25                  30

<210>  76
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (17)..(17)
<223>  modified with O-decanoyl

<220>
<221>  MOD_RES
<222>  (30)..(30)

<223>  AMIDATION

<400>  76
His Xaa Asp Gly Thr Phe Thr Ser Asp Leu Ser Lys Gln Xaa Glu Glu
1               5                   10                  15
Ser Ala Val Arg Leu Phe Ile Glu Trp Leu Lys Asn Gly Gly
            20                  25                  30

<210>  77
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (27)..(27)
<223>  modified with N-epsilon-octanoyl

<220>
<221>  MOD_RES
<222>  (30)..(30)
<223>  AMIDATION

<400>  77
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Lys Asn Gly Gly
            20                  25                  30

<210>  78
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle) or Leu

<220>
<221>  MOD_RES
<222>  (27)..(27)

<223>  modified with N-epsilon-octanoyl

<220>
<221>  MOD_RES
<222>  (30)..(30)
<223>  AMIDATION

<400>  78
His Xaa Asp Gly Thr Phe Thr Ser Asp Leu Ser Arg Gln Xaa Glu Glu
1               5                   10                  15
Glu Ala Val Arg Leu Phe Ile Glu Trp Leu Lys Asn Gly Gly
            20                  25                  30

<210>  79
<211>  30
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MOD_RES
<222>  (12)..(12)
<223>  modified with N-epsilon-octanoyl

<220>
<221>  MISC_FEATURE
<222>  (14)..(14)
<223>  Xaa = norleucine (Nle) or Leu

<220>
<221>  MOD_RES
<222>  (30)..(30)
<223>  AMIDATION

<400>  79
His Xaa Asp Gly Thr Phe Thr Ser Asp Leu Ser Lys Gln Xaa Glu Glu
1               5                   10                  15
Glu Ala Val Arg Leu Phe Ile Glu Trp Leu Arg Asn Gly Gly
            20                  25                  30

<210>  80
<211>  33
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)

<223>  Xaa = norleucine (Nle)

<220>
<221>  MISC_FEATURE
<222>  (31)..(31)
<223>  Xaa = Asp or D-Asp

<220>
<221>  MISC_FEATURE
<222>  (32)..(32)
<223>  Xaa = 5-aminovaleric acid (Ava)

<220>
<221>  MOD_RES
<222>  (33)..(33)
<223>  AMIDATION

<220>
<221>  MISC_FEATURE
<222>  (33)..(33)
<223>  Xaa = 11-aminoundecanoic acid (Aun)

<400>  80
His Xaa Asp Gly Thr Phe Thr Ser Asp Leu Ser Arg Gln Xaa Glu Glu
1               5                   10                  15
Glu Ala Val Arg Leu Phe Ile Glu Trp Leu Arg Asn Gly Arg Xaa Xaa
            20                  25                  30
Xaa

<210>  81
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, Met, or
       1-amino-1-cyclohexanecarboxylic acid (A6c)

<220>
<221>  MISC_FEATURE
<222>  (29)..(29)
<223>  Xaa = beta-Ala

<220>
<221>  MOD_RES
<222>  (39)..(39)
<223>  AMIDATION

<400>  81
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Arg Asn Xaa Gly Pro Ser
            20                  25                  30

Ser Gly Ala Pro Pro Pro Ser
        35

<210>  82
<211>  39
<212>  PRT
<213>  Artificial Sequence

<220>
<223>  Analogue of exendin-4

<220>
<221>  MISC_FEATURE
<222>  (14)..(14)
<223>  Xaa = norleucine (Nle) or Met

<220>
<221>  MISC_FEATURE
<222>  (29)..(29)
<223>  Xaa = beta-Ala

<220>
<221>  MOD_RES
<222>  (39)..(39)
<223>  AMIDATION

<400>  82
His Gly Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly Pro Ser
            20                  25                  30
Ser Gly Ala Pro Pro Pro Ser
        35

<210>  83
<211>  39
<212>  PRT
<213>  Artificial Sequence

<220>
<223>  Analogue of exendin-4

<220>
<221>  MISC_FEATURE
<222>  (14)..(14)
<223>  Xaa = norleucine (Nle), Leu, or Met

<220>
<221>  MISC_FEATURE
<222>  (29)..(29)
<223>  Xaa = alpha-aminoisobutyric acid (Aib)

<220>
<221>  MOD_RES
<222>  (39)..(39)
<223>  AMIDATION

<400>  83
His Gly Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly Pro Ser
            20                  25                  30
Ser Gly Ala Pro Pro Pro Ser

        35

<210>  84
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, Met, or
       1-amino-1-cyclohexanecarboxylic acid (A6c)

<220>
<221>  MISC_FEATURE
<222>  (29)..(29)
<223>  Xaa = beta-Ala

<220>
<221>  MOD_RES
<222>  (39)..(39)
<223>  AMIDATION

<400>  84
His Xaa Glu Gly Thr Phe Thr Ser Asp Leu Ser Arg Gln Xaa Glu Glu
1               5                   10                  15
Glu Ala Val Arg Leu Phe Ile Glu Phe Leu Arg Asn Xaa Gly Pro Ser
            20                  25                  30
Ser Gly Ala Pro Pro Pro Ser
        35

<210>  85
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle), Leu, Met, or
       1-amino-1-cyclohexanecarboxylic acid (A6c)

<220>
<221>  MISC_FEATURE
<222>  (29)..(29)
<223>  Xaa = beta-Ala

<220>
<221>  MOD_RES
<222>  (39)..(39)
<223>  AMIDATION

<400>  85
His Xaa Glu Gly Thr Phe Thr Ser Asp Leu Ser Lys Gln Xaa Glu Glu
1               5                   10                  15
Glu Ala Val Arg Leu Phe Ile Glu Phe Leu Lys Asn Xaa Gly Pro Ser
            20                  25                  30
Ser Gly Ala Pro Pro Pro Ser
        35

<210>  86
<211>  31
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (29)..(29)
<223>  Xaa = beta-Ala

<220>
<221>  MISC_FEATURE
<222>  (31)..(31)
<223>  Xaa = 4-(2-aminoethyl)-1-carboxymethyl-piperazine (Acp)

<220>
<221>  MOD_RES
<222>  (31)..(31)
<223>  AMIDATION

<400>  86
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Arg Asn Xaa Arg Xaa
            20                  25                  30

<210>  87
<211>  31
<212>  PRT
<213>  Artificial Sequence

<220>
<223>  Analogue of exendin-4

<220>
<221>  MISC_FEATURE
<222>  (2)..(2)

<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (29)..(29)
<223>  Xaa = beta-Ala

<220>
<221>  MISC_FEATURE
<222>  (31)..(31)
<223>  Xaa = 4-(2-aminoethyl)-1-carboxymethyl-piperazine (Acp)

<220>
<221>  MOD_RES
<222>  (31)..(31)
<223>  AMIDATION

<400>  87
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Arg Xaa
            20                  25                  30

<210>  88
<211>  40
<212>  PRT
<213>  Artificial Sequence

<220>
<223>  Analogue of exendin

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (29)..(29)
<223>  Xaa = beta-Ala

<220>
<221>  MISC_FEATURE
<222>  (40)..(40)
<223>  Xaa = 4-(2-aminoethyl)-1-carboxymethyl-piperazine (Acp)

<220>
<221>  MOD_RES
<222>  (40)..(40)
<223>  AMIDATION

<400>  88
His Xaa Glu Gly Thr Phe Thr Ser Asp Leu Ser Arg Gln Xaa Glu Glu

1                   5                   10                  15
Glu Ala Val Arg Leu Phe Ile Glu Trp Leu Arg Asn Xaa Gly Pro Ser
            20                  25                  30
Ser Gly Ala Pro Pro Pro Ser Xaa
        35                  40

<210>  89
<211>  40
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (29)..(29)
<223>  Xaa = beta-Ala

<220>
<221>  MISC_FEATURE
<222>  (40)..(40)
<223>  Xaa = 4-(2-aminoethyl)-1-carboxymethyl-piperazine (Acp)

<220>
<221>  MOD_RES
<222>  (40)..(40)
<223>  AMIDATION

<400>  89
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly Pro Ser
            20                  25                  30
Ser Gly Ala Pro Pro Pro Ser Xaa
        35                  40

<210>  90
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MOD_RES
<222>  (12)..(12)

<223>  modified with
N-epsilon-4-(2-aminoethyl)-1-carboxymethyl-piperazine(Acp)-octanoyl
(N-epsilon-Acp-octanoyl)

<220>
<221>  MISC_FEATURE
<222>  (14)..(14)
<223>  Xaa = norleucine (Nle)

<220>
<221>  MISC_FEATURE
<222>  (27)..(27)
<223>  Xaa = Arg or Lys

<220>
<221>  MISC_FEATURE
<222>  (29)..(29)
<223>  Xaa = beta-Ala

<220>
<221>  MOD_RES
<222>  (39)..(39)
<223>  AMIDATION

<400>  90
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Xaa Asn Xaa Gly Pro Ser
            20                  25                  30
Ser Gly Ala Pro Pro Pro Ser
        35

<210>  91
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (12)..(12)
<223>  Xaa = Arg or Lys

<220>
<221>  MISC_FEATURE
<222>  (14)..(14)
<223>  Xaa = norleucine (Nle)

<220>
<221>  MOD_RES
<222>  (27)..(27)
<223>  modified with
N-epsilon-4-(2-aminoethyl)-1-carboxymethyl-piperazine(Acp)-octanoyl
(N-epsilon-Acp-octanoyl)

<220>

<221>  MISC_FEATURE
<222>  (29)..(29)
<223>  Xaa = beta-Ala

<220>
<221>  MOD_RES
<222>  (39)..(39)
<223>  AMIDATION

<400>  91
His Xaa Glu Gly Thr Phe Thr Ser Asp Leu Ser Xaa Gln Xaa Glu Glu
1               5                   10                  15
Glu Ala Val Arg Leu Phe Ile Glu Trp Leu Lys Asn Xaa Gly Pro Ser
            20                  25                  30
Ser Gly Ala Pro Pro Pro Ser
        35

<210>  92
<211>  39
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MOD_RES
<222>  (27)..(27)
<223>  modified with
N-epsilon-4-(2-aminoethyl)-1-carboxymethyl-piperazine(Acp)-decanoyl
(N-epsilon-Acp-decanoyl)

<220>
<221>  MISC_FEATURE
<222>  (29)..(29)
<223>  Xaa = beta-Ala

<220>
<221>  MOD_RES
<222>  (39)..(39)
<223>  AMIDATION

<400>  92
His Xaa Glu Gly Thr Phe Thr Ser Asp Leu Ser Lys Gln Xaa Glu Glu
1               5                   10                  15
Glu Ala Val Arg Leu Phe Ile Glu Trp Leu Lys Asn Xaa Gly Pro Ser
            20                  25                  30
Ser Gly Ala Pro Pro Pro Ser
        35

<210>  93
<211>  39
<212>  PRT

<213>  Artificial Sequence

<220>
<223>  Analogue of exendin-4

<220>
<221>  MOD_RES
<222>  (39)..(39)
<223>  C-terminus is the free acid

<400>  93
His Gly Glu Gly Thr Phe Thr Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35

<210>  94
<211>  41
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (40)..(40)
<223>  Xaa = 5-aminovaleric acid (Ava)

<220>
<221>  MOD_RES
<222>  (41)..(41)
<223>  AMIDATION

<220>
<221>  MISC_FEATURE
<222>  (41)..(41)
<223>  Xaa = 11-aminoundecanoic acid (Aun)

<220>
<221>  MOD_RES
<222>  (41)..(41)
<223>  AMIDATION

<400>  94
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Arg Asn Gly Gly Pro Ser
            20                  25                  30
Ser Gly Ala Pro Pro Pro Ser Xaa Xaa
        35                  40

<210>  95
<211>  41
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (40)..(40)
<223>  Xaa = 5-aminovaleric acid (Ava)

<220>
<221>  MOD_RES
<222>  (41)..(41)
<223>  AMIDATION

<220>
<221>  MISC_FEATURE
<222>  (41)..(41)
<223>  Xaa = 11-aminoundecanoic acid (Aun)

<400>  95
His Xaa Asp Gly Thr Phe Thr Ser Asp Leu Ser Arg Gln Xaa Glu Glu
1               5                   10                  15
Glu Ala Val Arg Leu Phe Ile Glu Trp Leu Arg Asn Gly Gly Pro Ser
            20                  25                  30
Ser Gly Ala Pro Pro Pro Ser Xaa Xaa
        35                  40

<210>  96
<211>  32
<212>  PRT
<213>  Artificial Sequence

<220>
<223>  Analogue of exendin-4

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (31)..(31)

<223>  Xaa = 5-aminovaleric acid (Ava)

<220>
<221>  MOD_RES
<222>  (32)..(32)
<223>  AMIDATION

<220>
<221>  MISC_FEATURE
<222>  (32)..(32)
<223>  Xaa = 11-aminoundecanoic acid (Aun)

<400>  96
His Xaa Glu Gly Thr Phe Thr Ser Asp Leu Ser Arg Gln Xaa Glu Glu
1               5                   10                  15
Glu Ala Val Arg Leu Phe Ile Glu Trp Leu Arg Asn Gly Arg Xaa Xaa
            20                  25                  30

<210>  97
<211>  32
<212>  PRT
<213>  Artificial Sequence

<220>
<223>  Analogue of exendin-3

<220>
<221>  MISC_FEATURE
<222>  (2)..(2)
<223>  Xaa = alpha-aminoisobutyric acid (Aib)

<220>
<221>  MISC_FEATURE
<222>  (14)..(14)
<223>  Xaa = norleucine (Nle)

<220>
<221>  MISC_FEATURE
<222>  (31)..(31)
<223>  Xaa = 5-aminovaleric acid (Ava)

<220>
<221>  MOD_RES
<222>  (32)..(32)
<223>  AMIDATION

<220>
<221>  MISC_FEATURE
<222>  (32)..(32)
<223>  Xaa = 11-aminoundecanoic acid (Aun)

<400>  97
His Xaa Asp Gly Thr Phe Thr Ser Asp Leu Ser Arg Gln Xaa Glu Glu
1               5                   10                  15
Glu Ala Val Arg Leu Phe Ile Glu Trp Leu Arg Asn Gly Arg Xaa Xaa
            20                  25                  30

<210>  98
<211>  13
<212>  PRT
<213>  Artificial Sequence

<220>

<223>  Artificial peptide

<220>
<221>  MISC_FEATURE
<222>  (1)..(1)
<223>  Xaa = t-butyloxycarbonyl (Boc)

<220>
<221>  MOD_RES
<222>  (2)..(2)
<223>  modified with xanthyl (Xan)

<220>
<221>  MISC_FEATURE
<222>  (3)..(3)
<223>  Xaa = beta-Ala

<220>
<221>  MOD_RES
<222>  (6)..(7)
<223>  modified with benzyl (Bzl)

<220>
<221>  MOD_RES
<222>  (13)..(13)
<223>  modified with benzyl (Bzl) and with 4-methylbenzhydrylamine (MBHA)

<400>  98
Xaa Asn Xaa Gly Pro Ser Ser Gly Ala Pro Pro Pro Ser
1               5                   10

<210>  99
<211>  8
<212>  PRT
<213>  Artificial Sequence

<220>
<223>  Artificial peptide

<220>
<221>  MISC_FEATURE
<222>  (1)..(1)
<223>  Xaa = t-butyloxycarbonyl (Boc)

<220>
<221>  MOD_RES
<222>  (2)..(2)
<223>  modified with benzyl (Bzl)

<220>
<221>  MOD_RES
<222>  (8)..(8)
<223>  modified with benzyl (Bzl) and with 4-methylbenzhydrylamine (MBHA)

<400>  99
Xaa Ser Gly Ala Pro Pro Pro Ser
1               5

<210>  100
<211>  13
<212>  PRT
<213>  Artificial Sequence

<220>
<223>  Artificial peptide

<220>
<221>  MISC_FEATURE
<222>  (1)..(1)
<223>  Xaa = t-butyloxycarbonyl (Boc)

<220>
<221>  MOD_RES
<222>  (2)..(2)
<223>  modified with xanthyl (Xan)

<220>
<221>  MOD_RES
<222>  (6)..(7)
<223>  modified with benzyl (Bzl)

<220>
<221>  MOD_RES
<222>  (13)..(13)
<223>  modified with benzyl (Bzl) and with 4-methylbenzhydrylamine (MBHA)

<400>  100
Xaa Asn Gly Gly Pro Ser Ser Gly Ala Pro Pro Pro Ser
1               5                   10

<210>  101
<211>  39
<212>  PRT
<213>  Artificial Sequence

<220>
<223>  Analogue of exendin-4

<220>
<221>  MOD_RES
<222>  (7)..(7)
<223>  modified with N,N-tetramethylamidino (Tma)

<220>
<221>  MOD_RES
<222>  (39)..(39)
<223>  AMIDATION

<400>  101
His Gly Glu Gly Thr Phe His Ser Asp Leu Ser Lys Gln Met Glu Glu
1               5                   10                  15
Glu Ala Val Arg Leu Phe Ile Glu Trp Leu Lys Asn Gly Gly Pro Ser
            20                  25                  30
Ser Gly Ala Pro Pro Pro Ser
        35